(19) **Europäisches Patentamt** / **European Patent Office** / **Office européen des brevets**

(11) **EP 3 239 875 B1**

(12) # EUROPEAN PATENT SPECIFICATION

(45) Date of publication and mention
of the grant of the patent:
**02.10.2019 Bulletin 2019/40**

(21) Application number: **15873256.0**

(22) Date of filing: **25.12.2015**

(51) Int Cl.:
***C12Q 1/6881*** (2018.01)    ***C12Q 1/6869*** (2018.01)

(86) International application number:
**PCT/JP2015/086194**

(87) International publication number:
**WO 2016/104688 (30.06.2016 Gazette 2016/26)**

(54) **METHOD FOR DETERMINING GENOTYPE OF PARTICULAR GENE LOCUS GROUP OR INDIVIDUAL GENE LOCUS, DETERMINATION COMPUTER SYSTEM AND DETERMINATION PROGRAM**

VERFAHREN ZUR BESTIMMUNG DES GENOTYPS EINER BESTIMMTEN GENORTGRUPPE ODER EINES EINZELNEN GENORTS, BESTIMMUNGSCOMPUTERSYSTEM UND BESTIMMUNGSPROGRAMM

PROCÉDÉ PERMETTANT DE DÉTERMINER LE GÉNOTYPE D'UN GROUPE PARTICULIER DE LOCUS DE GÈNES OU D'UN LOCUS DE GÈNE INDIVIDUEL, SYSTÈME INFORMATIQUE DE DÉTERMINATION ET PROGRAMME DE DÉTERMINATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.12.2014 JP 2014265704**

(43) Date of publication of application:
**01.11.2017 Bulletin 2017/44**

(73) Proprietor: **National University Corporation, Tohoku University**
**Sendai-shi, Miyagi 980-8577 (JP)**

(72) Inventors:
• **NAGASAKI Masao**
  **Sendai-shi**
  **Miyagi 980-8577 (JP)**
• **NARIAI Naoki**
  **Sendai-shi**
  **Miyagi 980-8577 (JP)**
• **KOJIMA Kaname**
  **Sendai-shi**
  **Miyagi 980-8577 (JP)**

(74) Representative: **Henkel, Breuer & Partner Patentanwälte**
**Maximiliansplatz 21**
**80333 München (DE)**

(56) References cited:
JP-A- 2014 502 845      JP-A- 2014 507 133
JP-A- 2014 533 858      US-A1- 2005 049 795

• **BRESLER GUY ET AL: "Optimal assembly for high throughput shotgun sequencing.", BMC BIOINFORMATICS, vol. 14, no. Suppl 5, S18, 2013, XP002780071, ISSN: 1471-2105, DOI: 10.1186/1471-2105-14-S5-S18**
• **LIU TSUNGLIN ET AL: "Optimizing Information in Next-Generation-Sequencing (NGS) Reads for Improving De Novo Genome Assembly", PLOS ONE, vol. 8, no. 7, July 2013 (2013-07), XP002780072,**
• **MARTIN JOHN ET AL: "Optimizing Read Mapping to Reference Genomes to Determine Composition and Species Prevalence in Microbial Communities", PLOS ONE, vol. 7, no. 6, June 2012 (2012-06), XP002780073,**
• **KANAME KOJIMA ET AL: "Short tandem repeat number estimation from paired-end reads for multiple individuals by considering coalescent tree", BMC GENOMICS, vol. 17, no. S5, 1 August 2016 (2016-08-01), XP055467093, DOI: 10.1186/s12864-016-2821-0**

**(Cont. next page)**

- **NARIAI, NAOKI ET AL.: 'TIGAR: transcript isoform abundance estimation method with gapped alignment of RNA-Seq data by variational Bayesian inference' BIOINFORMATICS vol. 29, no. 18, 15 September 2013, pages 2292 - 2299, XP055396200 DOI: 10.1093/BIOINFORMATICS/BTT381**

**Description**

Technical Field

The present invention relates to the field of nucleic acid-based genetic analysis, and more particularly provides a method of genotyping selected gene loci or an individual gene locus easily and highly sensitively based on read information of human DNA generated from high-throughput sequencers. Preferred examples of the gene loci or individual gene locus include gene loci or an individual gene locus of the major histocompatibility complex (MHC). Human MHC is a human leucocyte antigen (HLA).

Background Art

The genotype (allele) of particular gene loci is associated with various diseases and phenotypes of human and other organisms.

For example, alleles of HLA which is s human MHC are important not only for judgment of compatibility in organ transplantation but also for associations with drug susceptibility, and numerous diseases, such as diabetes, autoimmune diseases, and narcolepsy, which have been recently reported. Therefore, it is extremely important to properly determine the genotype of each HLA locus in HLA loci in clinical applications.

Currently, a most popularly used HLA typing method employs PCR amplification of a target region by designing primers flanking an HLA allele, followed by DNA sequencing (for example, Patent Document 1). However, with this method, it is difficult to accurately estimate HLA alleles, because of problems such as amplification of pseudogene region due to an effect from off-target amplification when using the amplification primers. Although Patent Document 1 aims to solve this problem by using a specific primer set, it is likely that there will be a certain amount of inaccuracy in HLA typing as it uses PCR amplification, because the target region of the HLA locus is practically limited, and there exists wide variety of polymorphisms between individuals in HLA genes.

Furthermore, there is a method to determine HLA types by SNP genotyping at an HLA locus by designing cDNA probes based on known HLA allele sequences (Itoh Y et al., Immunogenetics 2005). However, with this method, it is difficult to determine new alleles, and there is a problem that individual genomic variation in alleles affects SNP typing accuracy.

Prior Art Documents

Patent Documents

Patent Document 1: WO 2014/065410 A1

Disclosure of the invention

Problems to be Solved by the Invention

As described above, when determining genotypes in a particular locus of MHC by using high performance sequencers in any form, inaccuracy of amplification such as amplification of an off-target region, at the stage of performing a gene amplification method such as a PCR, and others, is always a problem regardless of how an appropriate amplification primer is used. There exists a similar problem, for any gene loci, where there are other loci with similar nucleotide sequences, or genetic polymorphism exists, during the process of determining genotypes of each locus of the gene loci.

Therefore, means for optimizing read information obtained by a high-performance sequencer that is aligned to a particularly complicated gene loci, such as MHC loci, is required for genotyping each locus.

The present invention aims to provide the optimizing scheme from the viewpoint of probabilistic statistical processing.

Means for Solving the Problems

The inventors of the present invention selected HLA loci, which is human MHC loci, as a loci in the process of investigating toward solving this problem, and from the subject's sample DNA, whole-genome sequencing read data was obtained by a high-throughput sequencer, and then this read data was aligned to the HLA allele reference sequences of these loci to obtain mapping correspondence information between read data and HLA allele reference sequences. By performing maximum likelihood estimation procedure, or more preferably Bayesian inference procedure, the expected number of mappings of each read to each HLA allele, and the allele frequency of each HLA allele with respect to the read data were obtained, and the inventors have found that optimization of the expected number of mappings for each HLA allele that can be used for extremely accurate HLA typing is done, and the inventors completed the present invention which can accurately determine the allele at each locus where there are multiple loci having similar nucleotide base sequences in the genome or a large number of genetic polymorphisms exits in these loci. In the present invention, the "high-throughput sequencer" is a sequencer that can provide a large amount of read information in a relatively short period of time, which can be used in the practice of the present invention, and includes a so-called "next generation sequencer". Examples of the next generation sequencer at the time of carrying out the present invention include Genome Sequencer FLX (Roche (454)), Genome Analyzer IIx, HiSeq 2000, HiSeq 2500, MiSeq (both Illumina), SOLiD (Applied Biosystems), PacBio RS II (Pacific Biosciences) and the like. However, the high-throughput sequencer in the present invention is not limited to these sequencers, and includes all the high-throughput sequencers currently provided, and will be provided in the future.

At the time of completion of the present invention, two types of read information are generally recognized: single-end read information and paired-end read information. The single-end read information is read information on a fixed length or variable length (about 50 to 300 bp in general) at one end of the base sequence of the DNA fragment corresponding to the read, and the paired-end read information is read information on a fixed length or variable length (about 50 to 300 bp in general) at both ends of the DNA fragment. Although the content of the read information is also progressing rapidly in accordance with the advancement of the technology, it is possible to apply the present invention to the present or future read information.

As described above, the selected gene loci that include a gene locus where there are multiple genes or pseudogenes having a similar nucleotide sequence, and gene loci where there are many known genetic polymorphisms (alleles) exist at each locus, are the preferred loci. For such gene loci, as a common general knowledge of those skilled in this field, for example, cytochrome P450 (Cytochrome P450: CYP) loci, gene loci encoding immunoglobulin, gene loci encoding T cell receptors, gene loci encoding olfactory receptors, and the like are known, in addition to the MHC loci such as HLA loci. Cytochrome P 450 is a generic term for enzymes belonging to the oxidoreductase family involved in drug metabolism and detoxification, and 57 functional genes and 58 pseudogenes are known in human. Furthermore, at least 2,000 genetic polymorphisms (alleles) of the loci have been known so far, and it is known that metabolic rate of various drugs for each individual is different according to these genetic polymorphisms (alleles). Even if the HLA loci, which is one of the MHC loci of the examples disclosed in the present specification, is replaced by another complex loci, for example, the cytochrome P450 loci, it was obvious at the time of completion of the present invention that similar convenient utilities are obtained.

In addition, an individual gene locus is a gene locus constituting the gene loci, and for example, each of HLA-A, HLA-B, HLA-C is an individual gene locus for HLA gene loci.

[A] The optimization method of the present invention

The present invention provides a method for optimizing read information of DNA (hereinafter also referred to as the optimization method of the present invention), which is characterized by executing all or a part of the following steps (1) to (6) using read information in which mapping correspondence of each read to alleles of particular gene loci or an individual gene locus (hereinafter also referred to as read mapping information of particular gene loci or an individual gene locus) is obtained by mapping a nucleotide sequence of a DNA read of data where read information of DNA derived from alleles of selected gene loci or an individual gene locus (hereinafter also referred to as particular gene loci or an individual gene locus) is mixed, where there are multiple loci having similar nucleotide base sequences in a genome or a large number of genetic polymorphisms exits in the loci. The optimization method of the present invention is a method executed in a computer.

(1) A step in which, for each read, quantification of the expected number of mappings to each allele of particular gene loci or an individual gene locus, is performed.
"Expected number of mappings" is defined for each read against each allele, "total number of expected mappings" described later is defined for each allele, and "sum of total number of expected mappings" is the number of mappings defined for the gene loci or individual gene locus. In the present invention, "mapping" and "alignment" are synonymous.
(2) A step in which the expected number of mappings quantified in step (1) is summed for each allele of the gene loci or individual gene locus to calculate the total number of expected mappings.
The step (2) can be represented by a mathematical expression, for example, by the following formula (I), showing derivation of the total number of expected mapping $r_t$ generated from the allele t of particular gene loci or an individual gene locus by summing the currently estimated values of the abundance parameter.
[Mathematical 1]

$$r_t = \sum_n E_z [Z_{nt}] \qquad (I)$$

[In the expression, $Z_{nt}$ is an indicator variable, where it equals to 1, if read n is generated from the allele t, and 0 otherwise, and $E_z[Z_{nt}]$ is an expected value of $Z_{nt}$. Here, an expected value of $Z_{nt}$ is equivalent to the posterior probability of $Z_{nt}=1$.]

(3) A step that calculates a fraction of reads allocated to each allele of the gene loci or individual gene locus relative to the total read amount mapped to alleles of the gene loci or individual gene locus, where the total number of expected mappings calculated in step (2) is divided by the sum of the total number of expected mappings of all the alleles of the gene loci or individual gene locus.

The step (3) can be represented by a mathematical expression, for example, by the following expression (II), showing $E_q[\theta_t]$, a fraction of reads assigned to an allele of particular gene loci or an individual locus, relative to the total read amount.

[Mathematical 2]

$$E_q [\theta_t] = r_t \diagup (\sum_{t'} r_{t'}) \qquad (II)$$

[In the expression, $r_t$ is the total number of expected mapping generated from the allele t of particular gene locus or an individual gene locus and $\theta$ is the parameter indicating the frequency of the abundance of reads on the allele of particular gene loci or an individual gene locus.]

(4) A step in which the fraction of reads obtained in step (3) is assigned to each allele of the gene loci or individual gene locus as frequency, and based on the assigned frequencies, for each read, the expected number of mappings to each allele of the gene loci or individual gene locus is calculated again.

(5) A step in which step (2) or (3) is executed again for calculating a fraction of reads allocated to an allele of the gene loci or individual gene locus relative to the total read amount mapped to alleles of the gene loci or individual gene locus, based on the updated expected number of mappings obtained in step (4).

(6) A step in which step (4) and (5) are repeatedly executed, until for all reads difference between the expected number of mappings to each allele of the gene loci or individual gene locus calculated in step (4) and that calculated in the previous step (4) is not recognized, or for all alleles in the gene loci or individual gene locus difference between the fraction of reads calculated in step (5) and that calculated in the previous step (5) is not recognized, and the converged expected number of mappings for each read for each allele of the gene loci or individual gene locus, or, the converged fraction of reads for alleles of the gene loci or individual gene locus, is recognized as optimized data.

In the optimization method of the present invention as described above, when for each read, the expected number of mappings for each allele of the particular gene loci or individual gene locus is estimated in steps (1) and (4), and the allele frequencies for each allele of the particular gene loci or individual gene locus are calculated in steps (3) and (5), an Expectation Maximization algorithm (EM algorithm) is used to perform maximum likelihood estimation, and more preferably, a variational Bayesian method is used to perform Bayesian estimation.

Here, assumptions for performing optimization means such as the EM algorithm and the variational Bayesian method will be described. This is a more detailed explanation of the disclosure in the earlier application (Japanese Patent Application No. 2014-265704: hereinafter also referred to as the earlier application) which is the basis of priority claim of the present application. In the present invention, besides the EM algorithm and the variational Bayes method, a Bayesian method, a Gibbs sampling method, a MCMC method, an EP method, a PowerEP method, and others can be used.

The symbols indicating parameters, numbers, and other variables described in the specification, claims, and drawings are symbols for convenience of disclosure of the invention, and the present invention is not limited by these types of symbols at all.

As variables and parameters to be estimated by an inference method of the present invention, observed data, objective parameters, and latent variables connecting observed data and objective parameters can be enumerated, and, for example, can be set as follows.

[Observed data]

Observed data (hereinafter also referred to as $R_n$) is "nucleotide sequence of a DNA read (hereinafter also referred to as read n) of data where read information of DNA derived from alleles of particular gene loci or an individual gene locus is mixed", as described above. DNA mixed data is data as a whole, obtained by mapping reads to allele reference sequences of particular gene loci or an individual gene locus such as HLA loci or locus, HLA being a human MHC, as well as DNA reads themselves generated from DNA sequencing of a specimen. For example, when the particular gene loci is HLA loci, the reference sequence can be obtained from the IMGT/HLA database or other similar databases, but for these gene loci, genome sequences determined in the past studies using another specimen by sequencing or other methods is also possible to use as reference sequences. When a new genotype of the particular gene loci, for example, a new HLA type is found, it is preferable that the new genotype is successively registered into a database or other resources. It is also preferable that a novel genotype revealed by the present invention is registered. Determination of a new genotype with the present invention will be described later.

The observed data $R_n$ is the nucleotide sequence of the n-th read data of the DNA mixed data of N (N is a natural number: the number of reads) as described above. It is assumed that read data is observed as N independent and uniformly distributed read data. In the case of single-end read, one observed data $R_n$ corresponds to one read, but in the case of paired-end read, two observed data corresponds to a pair of nucleotide sequences at both ends of one fragment as a group. That is, in the case of a paired-end read, for example, a pair of $R_{na}$ and $R_{nb}$ is constructed, but since these are nucleotide information derived from the same fragment, by treating this set of reads as one unit, it is possible to handle it under the same statistical model similarly for single-end read (Nariai et al., Bioinformatics: 15; 29 (18) 2013). Specifically, it is possible to deal with the calculation as described in [Complete likelihood of paired-end model] described below based on the previous literature.

[Objective parameter]

The objective parameter is a parameter that is estimated on the basis of the observed data $R_n$. The present invention estimates one objective parameter (hereinafter also referred to as $\theta$).

The objective parameter $\theta$ is a vector representing the allele frequency of particular gene loci or an individual gene locus such as HLA loci or locus, HLA being a human MHC loci. For example, the parameter vector $\theta=(\theta_1,...,\theta_T)'$ (hereinafter, "′" in a vector or matrix indicates a transpose unless otherwise specified), and the fraction of the abundance for each allele of particular gene loci or an individual gene locus can be expressed under the condition below.

[Mathematical 3]

$$\sum_{t=1}^{t=T} \theta_t = 1$$

In this case, it is assumed that there are T (T is a natural number) alleles of a particular gene loci or an individual locus exist, and an allele of particular gene loci or an individual locus is counted as t (t is an integer of 1 or more).

By estimating the objective parameter $\theta$, it is possible to estimate the allele frequency or genotype of particular gene loci or an individual gene locus, which is the purpose of the present invention.

[Latent variables]

Latent variables, or hidden variables, are used to describe from which allele of particular gene loci or an individual locus the observed data $R_n$ is generated, and from which position in an allele of particular gene loci or an individual gene locus it is generated. In the present invention, by estimating parameter $\theta$ with incorporating the two kinds of latent variables $(T_n, S_n)$ or $T_n$ alone, these objective variables can be accurately estimated, and further, it is possible to estimate the genotype of each locus of the particular gene loci or the individual gene locus such as HLA loci or locus, HLA being a human MHC. It is possible to precisely estimate the genotype of each locus by estimating the parameter $\theta$ from the observed data $R_n$, where these latent variables are incorporated as dependent on the observed data $R_n$.

The above latent variable $T_n$ is a variable depending on the above $\theta$, representing allele selection of particular gene loci or an individual gene locus such as HLA loci or locus, HLA being a human MHC, of read n. $T_n = t$ means that read n is generated from allele t of particular gene loci or an individual gene locus.

The above latent variable $S_n$ is a variable depending on $T_n$ representing the start position of the read n. $S_n = s$ indicates that read n is generated from position s ($1 \leq s \leq l_t - L + 1$) (where $l_t$ is the length of allele t of particular gene loci or an individual gene locus and L is the read length). Here, the length $l_t$ of the allele of particular gene loci or an individual gene locus such as HLA loci or locus, HLA being a human MHC, which is a target of the present invention, is several hundreds of bases to several tens of thousands of bases in length, and generally, the length is longer than the base length of the read. Also, if the starting position s is 1, it means that the read has been generated from the first base of the allele of the particular gene loci or individual locus. In other words, $S_n$ means the starting position in the reference sequence of the allele of the particular gene loci or individual locus such as HLA loci or locus, HLA being human MHC, when the read is mapped to the reference sequence.

As described later, in particular in the case of a paired-end read model, for example, a latent variable $F_n$ and others representing the length of DNA fragment between the paired-end reads can be incorporated into genotype estimation, together with $T_n$ and $S_n$.

[Representation of the estimation method of the present invention]

For example, the estimation method of the present invention using the above variables can be represented as "An optimization method in which mapping of the read information of the DNA from the subject to alleles of particular gene loci or an individual gene locus is optimized by a computer, including a step to determine for each read the expected number of mappings to alleles of the gene loci or individual gene locus, and a step to estimate allele frequencies $\theta$ of the gene loci or individual gene locus as an object parameter (where $\theta$ is the T dimensional vector, T is the number of alleles of the gene loci or individual gene locus), given nucleotide sequences of all reads in data in which read information of DNA derived from alleles of selected gene loci or an individual gene locus is mixed, as observed data R, that is characterized in that with respect to (a) a latent variable $T_n$ dependent on $\theta$ related to the allele selection of the gene loci or individual gene locus of read n, and (b) a latent variable $S_n$ dependent on $T_n$ related to the starting position of n, and given the nucleotide sequence of read n as observed data $R_n$, at least (i) variables $T_n$ and $S_n$ or (ii) variable $T_n$ is incorporated to calculate an estimate of the parameter in the estimation process of the objective parameter $\theta$, so that observed data $R_n$ depends on the latent variables during the estimation process of the objective parameter $\theta$ from observed data $R_n$".

[Complete likelihood of single-end model]

The complete likelihood (posterior joint distribution) of the parameters of the optimization method of the present invention including dependency between variables using the above variables is decomposed as the product of the conditional probabilities. Specifically, it is represented by the following formula (1). Each symbol is as described above unless otherwise specified.
[Mathematical 4]

$$p(T_n, S_n, R_n \mid \boldsymbol{\theta}) = p(T_n \mid \boldsymbol{\theta})\, p(S_n \mid T_n)\, p(R_n \mid T_n, S_n) \qquad (1)$$

In the formula (1), $p(T_n = t \mid \theta)$ is the probability that read n is generated from allele t of particular gene loci or an individual locus given $\theta$. This probability can be calculated as $p(T_n = t \mid \theta) = \theta_t$ ((1) (a)).

$p(S_n = s \mid T_n = t)$ is the probability that read n is generated from position s given the allele t of the particular gene loci or individual locus. This probability can be calculated as $p(S_n = s \mid T_n = t) = 1 / (l_t - L + 1)$ ((1) (b)). $l_t$ is the length of the reference sequence of allele t, and L is the read length.

$p(R_n \mid T_n = t, S_n = s)$ is the probability of observing the nucleotide sequence of read n, given allele selection of the particular gene loci or individual locus and, the starting position of read n. Here, it is preferable to introduce the indicator variable $Z_{nts}$ for summarizing $T_n$ and $S_n$, or the indicator variable $Z_{nt}$ for summarizing $T_n$ ((1) (c)).

$Z_{nts}$ is equal to 1 when $(T_n, S_n) = (t, s)$, and is zero otherwise. Suppose $\pi_n$ is a set of all (t, s) pairs for possible mappings of read n, then for each $(t, s) \in \pi_n$ the following equation (2) can be used to calculate the probability of observing read sequence:
[Mathematical 5]

$$p(R_n \mid Z_{nts} = 1) = \prod_{x=1}^{L} subst(r_n[x], q_n[x], c_t[x]) \qquad (2)$$

(where subst(,,) is a base quality score dependent substitution probability function taking a numerical value obtained by subtracting a sequence substitution error from 1, $r_n[x]$ is the nucleotide character at position x of read n, $q_n[x]$ is the base quality score of position x of read n, and $c_t[x]$ is the base character of position x of the corresponding DNA sequence of allele t of the particular gene loci or individual locus.)
The base quality score substitution probability function, "subst(,,)" can be determined according to the Phred base quality score and can also be estimated from the best alignment of the read to the reference DNA sequence from the DNA-Seq data. The Phred base quality score is a score indicating a base reading accuracy provided together with nucleotide sequence information outputted in FASTQ format from a high-throughput sequencer, that is, a score indicating an error rate outputted by the sequencer (Phred quality Score). Specifically, the score Q is calculated as follows:

$$Q = -10 \log_{10} Y$$

(Y is the error rate).

On the other hand, $Z_{nt}$ is equal to 1 if $T_n = t$, and is zero otherwise. Since $Z_{nt}$ takes all the $Z_{nts}$ mentioned above into consideration with respect to possible s for each t, from (2), the probability of observing read sequence can be calculated as below.

[Mathematical 6]

$$p(R_n \mid Z_{nt} = 1) = \sum_{s=1}^{l_t - L + 1} p(R_n \mid Z_{nts} = 1) \qquad (2)'$$

Although the above is a calculation formula that takes into consideration the sequence substitution errors, equations that take into consideration sequence insertion / deletion errors can also be easily derived (Nariai et al., Bioinformatics: 15; 29 (18)).

The complete likelihood formula of the estimation method of the present invention shown in the above formula (1) is interpreted as described above.

This equation (1) serves as the basis for finding the posterior probability and posterior distribution of the latent variable according to the estimation method of the present invention using single-end model.

As for the latent variable $Z_{nt}$, it is possible to define it as independent of $Z_{nts}$, that is, not as a latent variable based on $Z_{nts}$ as described above, in which the mapped position "s" described above is not considered at all. For example, if the read n is mapped somewhere in the allele t, it is also possible to calculate the expected number of mappings using only the mapping score given by the mapping tool without considering the position "s". In principle, latent variable $Z_{nt}$ in the following disclosure is used as a variable based on $Z_{nts}$, but it is also possible to use $Z_{nt}$ as a variable independent of $Z_{nts}$ shown here.

[Complete likelihood of paired-end model]

In the case of paired-end data, complete likelihood (posterior joint distribution) of the parameters of the optimization method of the present invention including dependency between variables using the above variables is decomposed as the product of the conditional probabilities. Specifically, it is represented by the following expression (3). Each symbol is as described above unless otherwise specified.

[Mathematical 7]

$$p(T_n, S_n, F_n, R_{na}, R_{nb} \mid \theta) = p(T_n \mid \theta) p(F_n \mid T_n) p(S_n \mid T_n, F_n) p(R_{na} \mid T_n, S_n) p(R_{nb} \mid T_n, S_n, F_n) \qquad (3)$$

Here, $F_n$ is the length of a nucleotide fragment (fragment) estimated from the mapping of the pair of paired-end reads "$R_{na}$ and $R_{nb}$" to the reference sequence.

On the right-hand side of expression (3), $p(T_n = t \mid \theta)$ is the probability that read n is generated from allele t of particular gene loci or an individual gene locus given $\theta$. This probability can be calculated as $p(T_n = t \mid \theta) = \theta_t$ ((3) (a)).

$p(F_n = f \mid T_n = t)$ is the probability that the length f of the nucleotide fragment will be generated given the allele t of the locus. Let $d_F(x)$ be the distribution of the length of the nucleotide fragment given in advance. The probability can be calculated as below.

[Mathematical 8]

$$p(F_n = f \mid T_n = t) = d_F(f) / \sum_{x=L}^{l_t} d_F(x) \qquad (3)(a')$$

Here $l_t$ is the length of the reference sequence of allele t, and L is the read length. Distribution $d_F(x)$ of the length of the nucleotide fragment is given as a normal distribution of, for example, mean $\mu_F$ and standard deviation $\sigma_F$. The mean $\mu_F$ and the standard deviation $\sigma_F$ may be specified as long as the distribution of the nucleotide fragment length is experimentally known at the time of preparing the nucleotide fragment, but can be estimated and specified from the result of alignment of many paired-end reads beforehand.

$p(S_n = s \mid T_n = t, F_n = f)$ is the probability that the pair of read n of fragment length f is generated from position s, given the allele t of the particular gene loci or individual gene locus. This probability can be calculated as $p(S_n = s \mid T_n = t, F_n = f) = 1 / (l_t - f + 1)$ ((3) (b)). $l_t$ is the length of the reference sequence of allele t, L is the lead length, and f is the length of the base fragment.

$p(R_{na} \mid t_n = t, S_n = s)$ and $p(R_{nb} \mid T_n = t, S_n = s, F_n = f)$ can be calculated given allele selection of the particular gene loci or individual gene locus, start position of the pair of read n, and fragment length by the following expressions (4-1, 4-2):

[Mathematical 9]

$$p(R_{na}|T_n, S_n) = \prod_{x=1}^{L} subst(r_{na}[x], q_{na}[x], c_{ta}[x]) \qquad (4-1)$$

$$p(R_{nb}|T_n, S_n, F_n) = \prod_{x=1}^{L} subst(r_{nb}[x], q_{nb}[x], c_{tb}[x]) \qquad (4-2)$$

Here, subst(,,) is a base quality score dependent substitution probability function taking a numerical value obtained by subtracting a sequence substitution error from 1, $r_{na}[x]$ is nucleotide base character of position x of the first pair of read n ($R_{na}$), $q_{na}[x]$ is the base quality score of position x of the first pair of read n, $c_{ta}[x]$ is the corresponding nucleotide base character of allele t of the particular gene loci or individual gene locus at which the first pair of read n is aligned at position x, $r_{nb}[x]$ is nucleotide base character of position x of the second pair of read n ($R_{nb}$), $q_{nb}[x]$ is the base quality score of position x of the second pair of read n, and $c_{tb}[x]$ is the corresponding nucleotide base character of allele t of the particular gene loci or individual gene locus at which the second pair of read n is aligned at position x.

Expression (3) above is a basis for calculating the posterior probability and posterior distribution of the latent variables according to the estimation method of the present invention using the paired-end model.

The disclosure of the latent variable $Z_{nt}$ in the above single-end model can also be applied to this paired-end model.

[Hyperparameter]

In particular, when Bayesian estimation method such as variational Bayesian method is performed as the estimation means, it is preferable that hyperparameter $\alpha_0$ $(0 < \alpha_0)$ is incorporated and calculated, especially $0 < \alpha_0 \leq 0.1$, or $\alpha_0$ that maximizes the lower bound of the log likelihood being preferred. It is possible to carry out Bayesian estimation that is robust against outliers by incorporating an appropriate hyperparameter $\alpha_0$ of an appropriate value.

This can be expressed, for example, by the following formula (II)' based on the above formula (II).

[Mathematical 10]

$$E_q[\theta_t] = \alpha_t / (\Sigma_{t'} \alpha_{t'}) \qquad (II)'$$

[where $\alpha_t = \alpha_0 + r_t$]

Hyper parameter $\alpha_0$ is a constant to be considered in the framework of Bayesian estimation. That is, the parameter θ indicating the abundance of reads on alleles of the particular gene loci or individual gene locus can be estimated as a posterior distribution in the framework of Bayesian estimation, and here, we assume the Dirichlet distribution (Formula (III)):

[Mathematical 11]

$$p(\boldsymbol{\theta}) = \frac{1}{C} \prod_{t=1}^{T} \theta_t^{\alpha_t - 1} \qquad (III)$$

[where C is a constant, $\Pi^T_{t=1}\theta_t=1$, T is the number of alleles at particular gene loci or an individual gene locus under consideration, and $\alpha_t$ is the hyperparameter.]

The hyperparameter $\alpha_0$ which controls the complexity of the parameter θ (the number of $\alpha_t > 0$) is selected such that it maximizes the log marginal likelihood of the observed data.

Estimating the posterior distribution of θ given observed data requires integral with respect to latent variables and is difficult to calculate in closed form. Therefore, by assuming the factorization of the latent variables and the parameter θ, the equation is derived by approximating integrals in the posteriori probability distribution, which is described in formula (II)'.

The hyperparameter will be described later.

The optimization method of the present invention described above can be used without any particular limitation as long as it is applied to the read mapping information of the particular gene loci or individual gene locus. For example, the read mapping information of the gene locus can be the one obtained by processing with a high-throughput sequencer on a gene amplification product prepared with a primer corresponding to a certain locus of the particular gene loci or individual gene locus, or alternatively, it can be read information obtained by mapping the read mapping information of the gene locus to alleles at the locus. However, the optimization method of the present invention can be applied to the read mapping information of particular gene loci or an individual gene locus obtained by mapping read information generated from whole-genome sequencing of the subject sample to alleles of the particular gene loci or individual gene locus, without performing the step of amplifying genetic elements of the subject at a gene locus of the particular gene loci or individual gene locus.

In the present invention, an optimization method of the present invention is provided, which is characterized by executing the following steps (a) and (b) in mapping reads for acquiring mapping read information of the particular gene loci or individual gene locus. Hereinafter, the process of performing these steps is also referred to as a "mapping process of the particular gene loci or individual gene locus of the present invention".

(a) A step in which reads are mapped to the human reference genome sequence and then those mapped to alleles of particular gene loci or an individual gene locus are extracted, where the reads contain nucleotide sequence information of a subject generated from a DNA sequencer.

It is preferred that this initial mapping target is a human whole genome sequence, and it is customary that the target is the genome sequence of a specific person (a person selected as an analysis target or a combination of specific persons). It is usually a genome sequence determined by the Genome Reference Consortium or other institutions. With this first mapping, reads unrelated to alleles of the particular gene loci or individual gene locus can be excluded from the downstream analysis. In addition to the human whole genome sequence described above, for example, targeted sequence data, Exome sequence data, RNA sequence data, and long-read sequence data such as PacBio RS II, Oxford Nanopore and others can also be used as the mapping target sequence.

(b) A step in which the read sequence information mapped to alleles of the particular gene loci or individual gene locus obtained in step (a) is mapped to reference nucleotide sequences of alleles of the particular gene loci or individual gene locus that are registered in a database, and reads mapped to each allele of the gene loci or individual gene locus are extracted, and read information is obtained, in which mapping of each read to each allele of the gene loci or individual gene locus is identified.

The target of this second mapping is the genomic sequence of all alleles of the particular gene loci or individual gene locus registered in a database. As a result, it is possible to obtain provisional read mapping information of the particular gene loci or individual gene locus.

The mapping process in step (b) above, which corresponds to the mapping process of the particular gene loci or individual gene locus of the present invention, is preferred to be executed with allowing multiple mapping to alleles of the particular gene loci or individual gene locus. When the mapping targets are intentionally narrowed down at this point, there is a strong possibility that inappropriate biases will be included with respect to the read mapping information of the particular gene loci and individual gene locus to be derived.

As described above, the sequence information of reads obtained by a DNA sequencer used in the above step (a) may be the sequence information of paired-end, in which reads from both ends of each DNA fragment (about 50 to 300 bp each) are generated. Although the sequence information of single-end, in which reads from one side of each DNA fragment (about 50 to 300 bp) may be used, but paired-end read data is desirable to achieve higher accurate mapping, and as a result, higher accurate estimation of alleles, because the sequence information corresponding to one DNA fragment (usually about 300 to 1000 bp) can be specified based on the range bounded by the reads of both ends.

Also, it is preferable that, in addition to the reads mapped to alleles of the particular gene loci or individual gene locus in step (a), reads not mapped to the human genome are also extracted and are used in step (b). This is because the target genome for the mapping in the above step (a) is a genome of a specific person or a combination of multiple persons, and it is generally assumed that genome sequences will not be the same compared with that of the sample. For example, when the particular gene loci is HLA loci, HLA being human MHC, if the target genome for the mapping is the genome of Westerners and the subject is Japanese, then there is a possibility that the sequence of HLA loci differs greatly from the target genome, and hence there is a possibility that reads derived from HLA loci of the subject is not mapped to the target genome for mapping. In order to avoid this problem, the above processing is performed.

[B] The determination method of the present invention

It is possible to use the read mapping information of the particular gene loci or individual gene locus obtained by the optimization method of the present invention for an indicator of the genotype of each locus of the particular gene loci or individual gene locus. Particularly, if reads are generated from alleles of the gene locus by gene amplification operation such as PCR method using a gene amplification primer corresponding to the particular gene loci or individual gene locus at the stage of using the high-throughput sequencer, this tendency is easily recognized. In that case, the individual depth of coverage of reads for each allele of the gene locus is calculated from the fraction of reads in the read mapping information of the gene locus, and one or two alleles with the largest individual depth of coverage are determined as the genotype of the gene locus (determination method of the present invention). However, even in such cases, it is preferable to reexamine the results and eliminate the possibility of false positives as much as possible.

In addition, when the processes that narrow down to the gene locus of the gene loci using the gene amplification method was not performed beforehand, including the case where the mapping process of the particular gene loci or the individual gene locus of the present invention is perfomed, it is more preferable to reexamine the results.

In the case of a method that does not narrow down using a gene amplification method beforehand (for example, when a whole-genome reference sequence is used as a reference sequence), it is more desirable to perform the following reexamination process.

A preferred embodiment of the determination method of the present invention is a method of determining the genotype of each locus of the particular gene loci or individual gene locus extremely accurately, by re-evaluating read mapping information of the particular gene loci or individual gene locus obtained by the optimization method of the present invention described above with calculation of the individual depth of coverage.

Thus, the present invention provides genotype determination method for each locus of the particular gene loci or individual gene locus, which is characterized by calculating the individual depth of coverage of each allele from the fraction of reads on the allele of the gene loci or individual gene locus obtained by the optimization method of the present invention, by selecting the top two or less than two alleles of a gene locus of the particular gene loci or individual gene locus from a list of alleles that are sorted based on the individual depth of coverage by descending order, by setting a threshold of depth of coverage of alleles at 5-50%, or more preferably, 10-30%, of the depth of coverage of total reads in the determination method of genotype of each locus of the particular gene loci and individual gene locus for determining alleles as the genotype of the gene locus, and by eliminating alleles of the particular gene loci or individual gene locus whose individual depth of coverage are less than the threshold from genotyping of the gene loci or individual gene locus.

The "depth of coverage of total reads" indicates the average number of duplicate reading performed on the genome of the subject with a high-throughput sequencer, calculated based on the number of DNA fragments at each nucleotide position. Specifically, it is the total number of nucleotide bases contained in all reads generated by the sequencer divided by the total number of nucleotide bases of the genome (total human genome is 3 billion nucleotide bases). For example, if there are 900 million reads of 100 bp single-end, the depth of coverage of total reads is "100 $\times$ 900 million / 3 billion = 30", which is described as "30$\times$". Here, "total number of nucleotide bases of the genome" becomes the total number of nucleotide bases at a specific HLA locus, when this is not a whole-genome sequence data, for example, when the target region is "a particular HLA locus".

The "individual depth of coverage" means, in the present invention, indicates on average how many reads are overlapped at each position of the allele in the particular gene loci or individual gene locus, which can be calculated from the fraction of reads on the allele of the particular gene loci or individual gene locus obtained by the optimization method of the present invention.

Specifically, "the number of reads allocated to the allele of the particular gene loci or individual gene locus" is defined as "the fraction of reads on the allele of the particular gene loci or individual gene locus $\times$ total number of reads mapped to alleles of the gene loci or individual gene locus".

As described above, "the fraction of reads on the allele of the particular gene loci or individual gene locus" is calculated according to the optimization method of the present invention. Also, "the total number of reads mapped to alleles of particular gene loci or individual gene locus" is derived in, for example, "mapping process of the particular gene loci or individual gene locus of the present invention". Thus, "the number of reads allocated to the allele on the particular gene loci or individual gene locus" is calculated by performing the optimization method of the present invention.

Here, "individual depth of coverage" is calculated by "total number of nucleotide bases of reads mapped to the allele of particular gene loci or individual gene locus / the number of nucleotide bases of the allele reference sequence of the gene loci or individual gene locus".

Since an average number of nucleotide bases per read is known, "the total number of nucleotide bases of reads mapped to the allele at the particular gene loci or individual gene locus" is calculated by multiplying "the number of reads allocated to the allele at the gene locus" by the average number of nucleotide bases per read. "The number of nucleotide bases of allele reference sequence of the particular gene loci or individual gene locus" is a known number, which is different for each allele of the particular gene loci or individual gene locus. Thus, the "individual depth of coverage" of the allele of the particular gene loci or individual gene locus is, as described above, calculated based on the fraction of reads on the allele of the particular gene loci or individual gene locus obtained by the optimization method of the present invention.

In summary, the individual depth of coverage "$d_t$" in the allele of the particular gene loci or individual gene locus is calculated by the following equation (IV).

[Mathematical 12]

$$d_t = \left( \sum_{n=1}^{N} E[Z_{nt}] c_n \right) / l_t \qquad \text{(IV)}$$

[where N is the total number of reads, $c_n$ is the number of nucleotide bases contained in read n, $E[Z_{nt}]$ is the expected number of mappings of read n to allele t at the locus, and $l_t$ is the length of reference sequence of allele at each locus (the number of nucleotide base). t can range from 1 to T (the total number of alleles at the locus), and n can range from 1 to N.]

Note that $E[Z_{nt}]$ shown here can also be calculated by adding $E[Z_{nts}]$ mentioned above with respect to all possible s for each allele t.

By setting the "rejection depth of coverage" based on the depth of coverage of total reads as described above, an allele of the particular gene loci or individual gene locus having a small individual depth of coverage is excluded from genotyping, which leads to increase in accuracy of the determination method of the present invention by excluding, so to speak, a false positive allele candidate of the particular gene loci or individual gene locus.

The rejection depth of coverage can be selected as the frequency number of 5 to 50%, preferably 10 to 30% of the depth of coverage of total reads as described above. If the rejection depth of coverage is small, alleles of the particular gene loci or individual gene locus have a greater chance of becoming candidates for genotyping of the particular gene loci or individual gene locus in the determination method of the present invention, but the risk of choosing false positives will also increase. Conversely, if the rejection depth of coverage is large, there is less possibility of choosing false positives, but there is a higher possibility that it rejects alleles that represent true genotype of the particular gene loci or individual gene locus of the subject.

The reason of selecting "the top two or less than two alleles of a gene locus of the particular gene loci or individual gene locus from a list of alleles that are sorted based on the individual depth of coverage by descending order", or the maximum value of the number of alleles at each locus of the particular gene loci or individual gene locus is set to "two", is that the two different alleles at the locus will be determined as heterozygotes of these, and alleles of more than this number are excluded as noise. If the number is "one", the gene locus will be determined as homozygous of the allele, or heterozygous of the allele and another that is unknown, and if the number is "zero", the alleles of the corresponding locus will be determined as unknown.

More specifically about a manner of the determination method of the present invention, after excluding alleles for genotyping the particular gene loci or individual locus as described above, it is preferable that the following (i) or (ii) is determined.

(i) For the case that there is one allele that was selected for genotyping the gene locus of the particular gene loci or individual gene locus, if the individual depth of coverage of the allele is twice or more of the rejection threshold value described before, it is determined that the genotype is homozygous of the allele, and if it is less than twice of the rejection threshold, it is determined that the genotype is heterozygous of the allele and another allele is not determined, and

(ii) For the case that there are two alleles that were selected for genotyping the gene locus of the particular gene loci or individual gene locus, if the individual depth of coverage of the one whose individual depth of coverage is larger is less than twice of the smaller one, it is determined that the genotype is heterozygous of the two alleles, or if the individual depth of coverage of the one whose individual depth of coverage is larger is twice or more of the smaller one, it is determined that the genotype is homozygous of the allele whose individual depth of coverage is larger.

Performing the determination method of the present invention in this manner makes the determination of alleles of each locus of the particular gene loci or individual gene locus of the subject more accurately.

When the allele to be determined is novel, a known allele that is closest to the novel allele is determined first, and by recognizing differences between the known allele and the novel allele due to substitutions, insertions, deletions, and other variations of the nucleotide sequence, the nucleotide sequence of the novel allele can be determined. It is preferable that the nucleotide sequence of the novel allele is successively registered as a new allele in a target database or other databases.

Hereinafter, the optimization method and determination method of the present invention may be collectively referred to as "the method of the present invention".

[C] The computer system of the present invention

The computer system of the present invention is a system which is a device for performing the method of the present invention described above, and the same terms overlap as a concept unless otherwise noted. "Algorithm" means a formalized form of a procedure for solving a problem, which is the same as a general concept in computer fields.

The computer system of the present invention can include hardware related to a normal computer system. That is, in addition to a "recording unit" corresponding to a normal hard disk drive and an "arithmetic processing unit" corresponding to a CPU, for example, a "temporary storage unit" corresponding to RAM, an "operation unit" corresponding to a keyboard, a mouse, a touch panel, and other interfaces, a "display unit" corresponding to a display, an "input/output interface (IF) unit" corresponding to a serial or parallel interface corresponding to an operation unit, and a "communication interface (IF) unit" equipped with a video memory and a D/A conversion unit for outputting analog signal corresponding to the video mode of the display unit. The communication IF unit can exchange data with external information, particularly human genome information such as human genome databases.

Unless stated otherwise, the processing performed by the "processing unit" of the computer system of the present invention will be described below. "Operation processing unit" acquires data of the human genome database, in particular via the "communication IF unit" by operating the "operation unit", records it in the "recording unit", stores data read from the "recording unit" to the "temporary storage unit" occasionally, performs predetermined processing, and records the result again in the "recording unit". The "processing unit" creates screen data for prompting the operation of the "operation unit" and screen data for displaying the processing result, and displays these images on the "display unit" via the video RAM of the input IF unit. The program of the present invention is recorded at the time of use or recorded in advance in the "recording unit" or recorded in external hardware resources, and in "arithmetic processing unit" as necessary, arithmetic processing according to the described algorithm is performed.

The computer system of the present invention is a computer system for optimizing the read mapping information of the particular gene loci or individual locus, comprising a recording unit and an arithmetic processing unit, which is characterized in that all or a part of the following processes (A) to (G), is executed:

(A) In the recording unit, read information of DNA derived from the subject is recorded as data of nucleotide sequence of read and alleles of the gene loci or individual gene locus to which the read is mapped to,

(B) In the arithmetic processing unit, based on the information of the recording section, for each read, the expected number of mappings for each allele of the gene loci or individual gene locus is calculated by executing numerical processing,

(C) For each allele, the expected number of mappings quantified in the process (B) is summed for each allele of the gene loci or individual gene locus to calculate the total number of expected mappings,

(D) For each allele, the total number of expected mappings calculated in the process (C) is divided by the sum of the total number of expected mappings for all alleles of the gene loci or individual gene locus, and the process of calculating the fraction of reads allocated to each allele of the gene loci or the individual gene locus with respect to the total amount of reads mapped to alleles of the gene loci or individual gene locus is executed,

(E) The fraction of reads calculated in the above process (C) is assigned as frequency to each allele of the gene loci or individual gene locus, and given the assignment frequency, the process (B) described above is executed again in which for each read, the expected number of mappings to each allele of the gene loci or individual gene locus is calculated,

(F) The process (C) or (D) is executed again for the newly obtained expected number of mappings calculated by the process (E) described above, and the process of calculating the fraction of reads allocated to each allele of the gene loci or individual gene locus with respect to the total amount of reads mapped to alleles of the gene loci or the individual gene locus is executed,

(G) the processes (E) and (F) described above are repeatedly executed, until for all reads difference between the number of expected mappings to each allele of the gene loci or individual gene locus calculated in the process (E) and that calculated in the previous process (E) is not recognized, or for all alleles of the gene loci or individual gene locus difference between the value of the fraction of reads calculated in the process (F) and that calculated in the previous process (F) is not recognized, and the converged number of expected mappings for each read to each allele of the gene loci or individual gene locus, or the converged value of the fraction of reads for each allele (allele frequency) of the gene loci or individual gene locus, is recognized as the optimized data.

It is preferable that the above processes (B) to (G) are performed comprehensively on all alleles of the particular gene loci or individual gene locus. This comprehensive execution means that the optimization algorithm of mapping all reads is executed for all alleles of the gene loci or individual gene locus at the same time.

Furthermore, in the computer system of the present invention, the mapping of the read information of the gene of the subject to alleles of the gene loci or individual gene locus that are registered in the database can be executed by the following processes (a) and (b). In the case where the particular gene loci are targeted, it is preferable that the following processes (a) and (b) are carried out simultaneously for all the loci.

> (a) The process in which after mapping sequence information of the subject's reads obtained by a DNA sequencer to the reference nucleotide sequence of whole human genome, reads mapped to each locus of the gene loci or individual gene locus are extracted.
> (b) The process in which after mapping the reads mapped to the gene loci or individual gene locus extracted from the process (a) to nucleotide sequences of alleles of the gene loci or individual gene locus registered in the database, mapping information and mapping states, that is, identified read information of mapping position, differences between the read sequence and the reference sequence, and a mapping score, for each read to each allele of the gene loci or individual gene locus, are obtained.

It is preferred that the mapping performed in the process (b) allows one read to be mapped to multiple alleles of the particular gene loci or individual gene locus.

It is preferred that in addition to the reads mapped to alleles of the particular gene loci or individual gene locus obtained in the process (a), reads that were not mapped to the whole human genome are extracted and processed together, so that it becomes the target of the re-mapping in the process (b).

Furthermore, the present invention provides a computer system for determining the genotype of each gene locus of the particular gene loci or individual gene locus of a subject, comprising a recording unit and an arithmetic processing unit, and is characterized in that all or a part of the following processes ($\alpha$) to ($\delta$) is executed;

> ($\alpha$) In the recording unit, at least allele frequencies and depth of coverage of total reads of the gene loci or individual gene locus of the subject that were obtained by the optimization method of the present invention are recorded,
> ($\beta$) In the arithmetic processing unit, based on the allele frequencies of the gene loci or the individual gene locus based on the recording unit, processing of calculating the individual depth of coverage of each allele of the gene loci or individual gene locus, and processing of allocating the calculated individual depth of coverage to the alleles of the gene loci or the individual gene locus, are executed, ($\gamma$) Given a rejection threshold value, which is set as a frequency number of 5 to 50%, preferably 10 to 30% of the average depth of coverage of all reads, the process in which alleles of the gene loci or individual gene locus whose individual depth of coverage is smaller than the threshold value are excluded from candidates for genotyping,
> ($\delta$) :
>
> > ($\delta$)-1 After the exclusion process of ($\gamma$), for the case that there is one allele that was selected for genotyping the gene locus of the particular gene loci or individual gene locus, if the individual depth of coverage of the allele is twice or more of the rejection threshold value described before, it is determined that the genotype is homozygous of the allele, and if it is less than twice of the rejection threshold, it is determined that the genotype is heterozygous of the allele and another allele is not determined, and
> > ($\delta$)-2 After the exclusion process of ($\gamma$), for the case that there are two alleles that were selected for genotyping the gene locus of the particular gene loci or individual gene locus, if the individual depth of coverage of the one whose individual depth of coverage is larger is less than twice of the smaller one, it is determined that the genotype is heterozygous of the two alleles, or if the individual depth of coverage of the one whose individual depth of coverage is larger is twice or more of the smaller one, it is determined that the genotype is homozygous of the allele whose individual depth of coverage is larger.

In addition to the above, for example, it is also possible to carry out the setting in the computer system of the present invention, in which for the case that there is no allele found for the genotype of each locus of the particular gene loci or individual gene locus after execution of the exclusion process of ($\gamma$), the genotype of the gene locus based on the allele is not determined.

The categories of these computer systems are "objects" and can be replaced with "devices".

[D] The program of the present invention

The program of the present invention is a computer program implementing an algorithm run in the computer system of the present invention to execute the method of the present invention, and the same terms overlap as a concept unless otherwise noted.

The program of the present invention is a computer program for optimizing the read mapping information of the particular gene loci or individual gene locus, which is characterized by including algorithms to realize all or a part of the following functions (1) to (7):

(A) The function (1) in which read information of DNA derived from the subject recorded in the recording unit as data of nucleotide sequence of read and alleles of the gene loci or particular gene locus to which the read is mapped is retrieved,

(B) The function (2) in which based on the read information retrieved in the function (1), for each read, the expected number of mappings for each allele of the gene loci or individual gene locus is calculated by executing numerical processing,

(C) The function (3) in which for each allele, the expected number of mappings quantified in the function (2) is summed for each allele of the gene loci or individual gene locus to calculate the total number of expected mappings,

(D) The function (4) in which for each allele, the total number of expected mappings calculated in the function (3) is divided by the sum of the total number of expected mappings for all alleles of the gene loci or individual gene locus, and the process of calculating the fraction of reads allocated to each allele of the gene loci or the individual gene locus with respect to the total amount of reads mapped to alleles of the gene loci or individual gene locus is executed,

(E) The function (5) in which the fraction of reads calculated in the above function (4) is assigned as frequency to each allele of the gene loci or individual gene locus, and given the assignment frequency, the function (2) described above is executed again in which for each read, the expected number of mappings to each allele of the gene loci or individual gene locus is calculated,

(F) The function (6) in which the function (3) or (4) is executed again for the newly obtained expected number of mappings calculated by the function (5) described above, and the process of calculating the fraction of reads allocated to each allele of the gene loci or individual gene locus with respect to the total amount of reads mapped to alleles of the gene loci or the individual gene locus is executed,

(G) The function (7) in which the function (5) and (6) described above are repeatedly executed, until for all reads difference between the number of expected mappings to each allele of the gene loci or individual gene locus calculated in the function (5) and that calculated in the previous function (5) is not recognized, or for all alleles of the gene loci or individual gene locus difference between the value of the fraction of reads calculated in the function (6) and that calculated in the previous function (6) is not recognized, and the converged number of expected mappings for each read to each allele of the gene loci or individual gene locus, or the converged value of the fraction of reads for each allele of the gene loci or individual gene locus, is recognized as the optimized data.

Furthermore, the present invention provides the program of the present invention which is characterized by including algorithms of mapping of the read information of the gene of the subject to alleles of the particular gene loci or individual gene locus that are registered in the database by executing the following functions (a) and (b) to be realized in the computer.

(a) The function in which after mapping sequence information of the subject's reads obtained by a DNA sequencer to the reference nucleotide sequence of whole human genome, reads mapped to alleles of each locus of the gene loci or individual gene locus are extracted.

(b) The function in which after mapping the reads mapped to alleles of the gene loci or individual gene locus extracted from the function (a) to nucleotide sequences of alleles of the gene loci or individual gene locus registered in the database, mapping information and mapping states, that is, identified read information of mapping position, differences between the read sequence and the reference sequence, and a mapping score, for each read to each allele of the gene loci or individual gene locus, are obtained.

Furthermore, the present invention provides a computer program for determining the genotype of each gene locus of the particular gene loci or individual gene locus of a subject, which is characterized by including algorithms realizing the following functions ($\alpha$) to ($\delta$) to be executed in a computer.

($\alpha$) A function $\alpha$, in which at least allele frequencies and depth of coverage of total reads of the gene loci or individual gene locus of the subject that were obtained by executing the computer program described before are retrieved.

($\beta$) A function $\beta$, in which based on the allele frequencies of the gene loci or the individual gene locus retrieved by executing function $\alpha$, calculation of the individual depth of coverage of each allele of the gene loci or individual gene locus, and allocation of the calculated individual depth of coverage to the alleles of the gene loci or the individual gene locus, are executed.

(γ) A function γ, in which given a rejection threshold value, which is set as a frequency number of 5 to 50%, preferably 10 to 30% of the average depth of coverage of all reads, the process in which alleles of the gene loci or individual gene locus whose individual depth of coverage specified by the function β described before is smaller than the threshold value are excluded from candidates for genotyping.

(δ) : A function δ specified as (δ)-1 and (δ)-2 below.

(δ)-1 After the exclusion process in function(γ), for the case that there is one allele that was selected for genotyping the gene locus of the particular gene loci or individual gene locus, if the individual depth of coverage of the allele is twice or more of the rejection threshold value described before, the process in which it is determined that the genotype is homozygous of the allele, and if it is less than the twice of the rejection threshold, it is determined that the genotype is heterozygous of the allele and another allele is not determined, is executed, and

(δ)-2 After the exclusion process in function(γ), for the case that there are two alleles that were selected for genotyping the gene locus of the particular gene loci or individual gene locus, if the individual depth of coverage of the one whose individual depth of coverage is larger is less than twice of the smaller one, the process in which it is determined that the genotype is heterozygous of the two alleles, or if the individual depth of coverage of the one whose individual depth of coverage is larger is twice or more of the smaller one, it is determined that the genotype is homozygous of the allele whose individual depth of coverage is larger, is executed.

In addition to the processes described above, for example, it is also possible to add a function explicitly, in which for the case that there is no allele found for the genotype of each locus of the particular gene loci or individual locus after execution of exclusion process in the function (γ), the genotype of the gene locus based on the allele is not determined, is executed.

The computer program of the present invention can be described in, for example, C language, Java (registered trademark), Perl, Python, and other languages.

The present invention further provides a recording medium readable by a computer or a recording medium that can be connected to a computer (hereinafter also referred to as a recording medium of the present invention), which is characterized by that the program of the present invention is recorded. As the recording media, magnetic media such as flexible disks, flash memories, and hard disks, optical media such as CDs, DVDs, and BDs, magneto-optical media such as MO and MD, and other media can be mentioned, but the recording media of the present invention is not particularly limited to the examples. A typical computer system of the present invention is characterized by executing the program of the present invention.

Effect of the Invention

The present invention provides the method to optimize the read mapping information of the particular gene loci or individual gene locus derived from read information obtained with a high-throughput sequencer for determining genotype of the gene loci or individual gene locus, the computer system for the optimization, the computer program for the optimization, the determination method for genotyping each gene locus of the gene loci and individual gene locus by applying the optimization method, the computer system for the determination, and the computer program for the determination. The present invention enables genotyping of a gene locus of gene loci or individual gene locus that are known to have similar nucleotide sequence in a genome or known to be highly polymorphic, in a high accurate and efficient manner. As the gene loci, for example, HLA gene loci, HAL being human MHC, cytochrome P450 loci, immunoglobulin gene loci, T cell receptor gene loci, and olfactory receptor gene loci can be considered.

Brief Description of the Drawings

FIG. 1 is a diagram showing a process flow of the present invention.

FIG. 2-1 is a flow sheet showing one embodiment of the optimization process of HLA-read mapping information with a latent variable $Z_{nt}$.

FIG. 2-2 is a flow sheet of the EM algorithm showing one embodiment of the optimization process of HLA-read mapping information with a latent variable $Z_{nts}$.

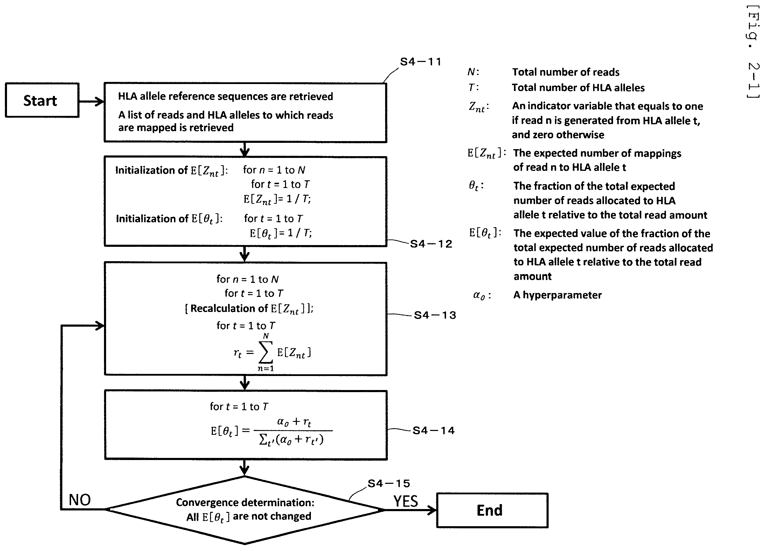

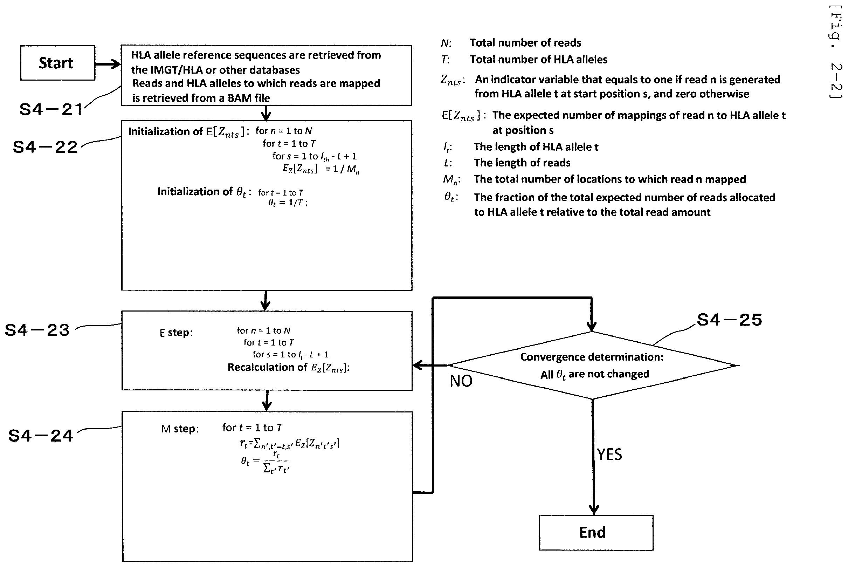

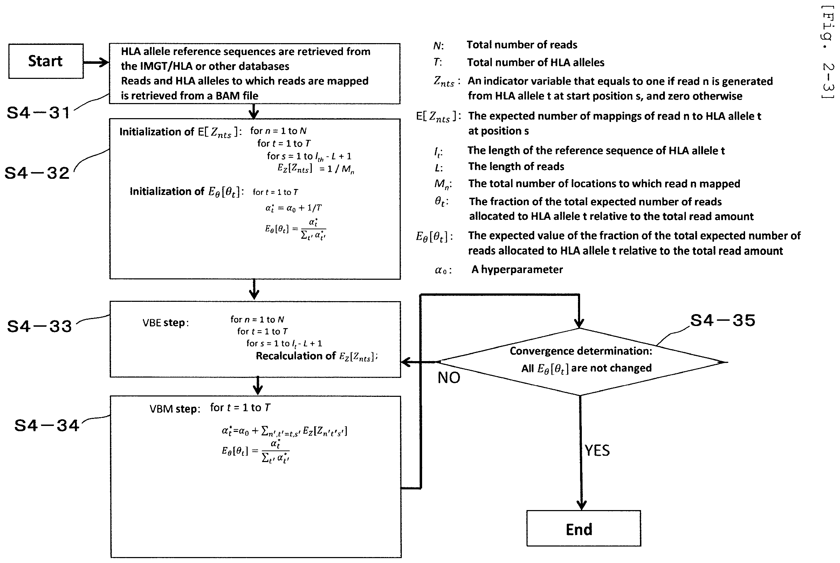

FIG. 2-3 is a flow sheet of the variational Bayesian algorithm showing one embodiment of the optimization process of HLA-read mapping information with a latent variable $Z_{nts}$.

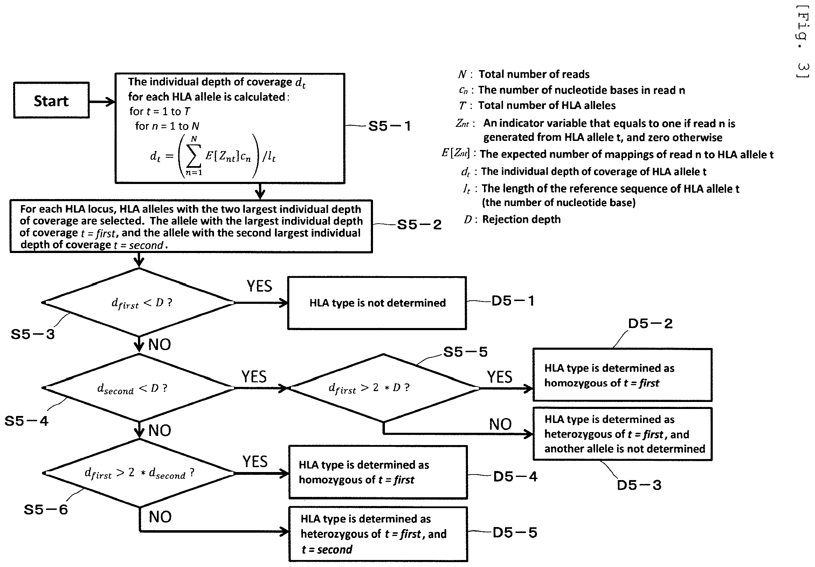

FIG. 3 is a flow sheet showing one example of the HLA typing process based on "the fraction of reads allocated to each HLA allele" obtained through the above optimization process.

16

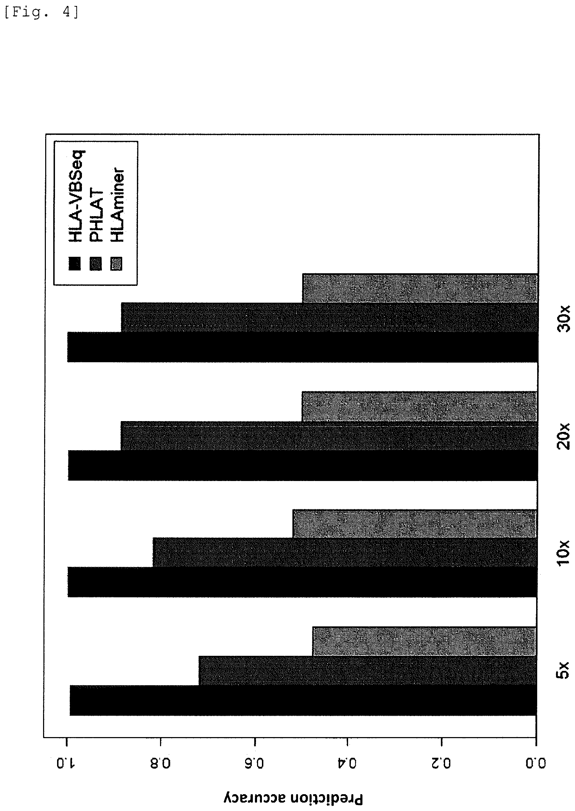

FIG. 4 is a figure showing prediction accuracy of HLA types at four-digit resolution at various depths of coverage using the simulation data.

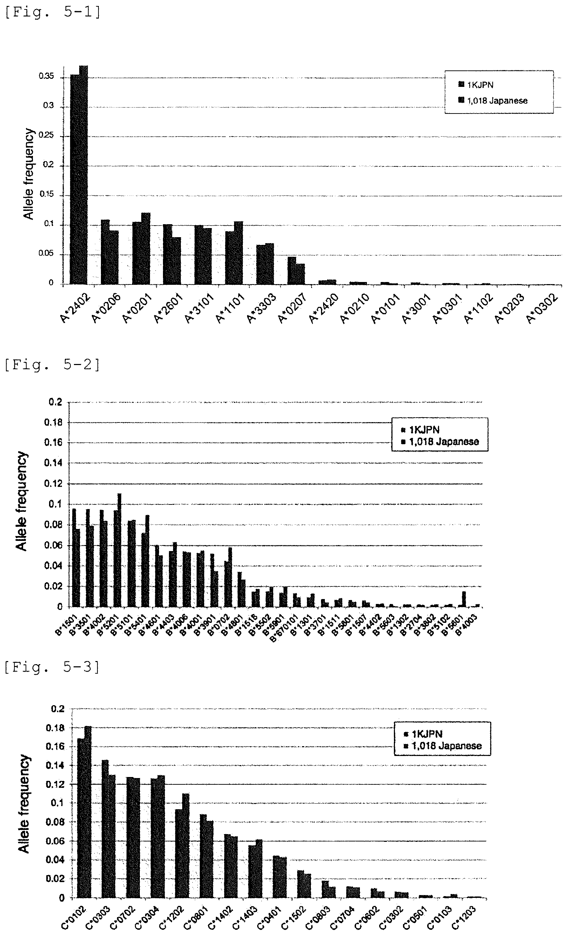

FIG. 5-1 is a graph showing the results of examining allele frequencies of HLA-A by the system of the present invention and another method.

FIG. 5-2 is a graph showing the results of examining allele frequencies of HLA-B by the system of the present invention and another method.

FIG. 5-3 is a graph showing the results of examining allele frequencies of HLA-C by the system of the present invention and another method. Modes of Carrying Out the Invention

[A] Estimation methods with the EM algorithm and variational Bayesian method

Here, the contents of the EM algorithm and the variational Bayesian method disclosed in the previous application will be described in more detail. Symbols to be used are as described before unless otherwise noted.

(1) EM (expectation-maximization) algorithm

The EM algorithm is a method for obtaining the maximum likelihood estimate of the parameter in the probability model in which the latent variable exists. In the estimation method of the present invention, for example, it can be done with the following contents.

That is, the estimation method of the present invention by the EM method is exemplified as the estimation method of the present invention characterized by performing the following steps (a) to (e) (this method is also referred to as "the EM method of the present invention").

(a) A step in which the first updated value of a posterior probability of $Z_{nts} = 1$ or $Z_{nt} = 1$ is calculated, based on a given initial value of $\theta_t$, and the first updated value of the maximum likelihood estimate of $\theta_t$ is further calculated, based on the first updated value of the posterior probability of $Z_{nts} = 1$ or $Z_{nt} = 1$, or,

(b) A step in which an updated value of the maximum likelihood estimate of $\theta_t$ is calculated, based on a given initial value of the posterior probability of the $Z_{nts} = 1$ or $Z_{nt} = 1$,

(c) A step in which an updated value of the posterior probability of $Z_{nts} = 1$ or $Z_{nt} = 1$ is calculated, based on the updated value of the maximum likelihood estimate of $\theta_t$ calculated by the previous step (d) (however, for the first time, it is step (a) or step (b)),

(d) A step in which an updated value of the maximum likelihood estimate of $\theta_t$ is calculated, based on the updated value of the posterior probability of $Z_{nts} = 1$ or $Z_{nt} = 1$ calculated in step (c), and

(e)

(i) A step in which convergence of the log likelihood is evaluated by calculating the log likelihood based on the posterior probability of $Z_{nts} = 1$ or $Z_{nt} = 1$ calculated in step (c) and $\theta_t$ calculated in step (d),

(ii) A step in which convergence of the updated posterior probability of $Z_{nts} = 1$ or $Z_{nt} = 1$ calculated in step (c) is evaluated, or

(iii) A step in which convergence of the updated maximum likelihood estimate of $\theta_t$ calculated in step (d) is evaluated, and,

if convergence is recognized, $\theta_t$ in each step is determined as the final estimate value, and if convergence is not recognized, iteration of steps (c), (d) and (e) is determined.

The step (a) described above is a step of defining the initial value of this method for iterative optimization.

The initial value of n in $Z_{nts} = 1$ or $Z_{nt} = 1$ is a number from 1 to N, t is a number from 1 to T. The value of s is a number $1 \leqq s \leqq l_t - L + 1$, where $l_t$ is the length of allele t of the particular gene locus, L is the read length. If a read is mapped to multiple locations, it is preferable to set the initial value of $Z_{nts} = 1$ or $Z_{nt} = 1$ as 1 / [The number of locations to which read n is mapped] by assuming that the mappings are equally likely. It is preferable to set the initial value of $\theta_t$ as 1/T by assuming that an allele frequency of each gene locus is the same. When the prior information of allele frequency of a specific gene locus is already known, it is also possible to use this information as the initial value of $\theta_t$, for example, when the subject population is a specific racial group.

The step (b) is a step in which $r_{nts}$, the posterior probability of $Z_{nts} = 1$ or $r_{nt}$, the posterior probability of $Z_{nt} = 1$, is calculated based on the current estimated value (for the first iteration, it is calculated based on the initial value obtained in the step (a)).

That is, it is a step of calculating $P (Z_{nts} = 1 \mid 0^*{}_t)$ or $P (Z_{nt} = 1 \mid \theta^*{}_t)$ (where * indicates that $\theta_t$ has been updated. The same notation follows below.), in which $r_{nts}$ is calculated as

[Mathematical 13]

$$r_{nts} = \begin{cases} \dfrac{\rho_{nts}}{\sum_{(t',s')\in\pi_n} \rho_{nt's'}} & \text{if } (t,s) \in \pi_n \\[2em] 0 & \text{otherwise.} \end{cases}$$

Here, for the single-end reads, $\rho_{nts}$ is calculated as

[Mathematical 14]

$$\log \rho_{nts} = \log\theta_t + \log p(S_n|T_n) + \log p(R_n|T_n, S_n)$$

(where the first term on the right side can be calculated by taking the logarithm of Expression (1) (a), the second term can be calculated by taking the logarithm of Expression (1) (c), and the third term can be calculated by taking the logarithm of Expression (2).

For the paired-end reads, $\rho_{nts}$ is calculated as

[Mathematical 15]

$$\log\rho_{nts} = \sum_{f=L}^{l_t-s+1} \{\log\theta_t + \log p(F_n = f|T_n) + \log p(S_n|T_n, F_n = f) + \log p(R_{na}|T_n, S_n)$$

$$+ \log p(R_{nb}|T_n, F_n, S_n)\}$$

(where the first term within the brackets on the right side can be calculated by taking the logarithm of Expression (3) (a), the second term can be calculated by taking the logarithm of Expression (3) (a'), the third term can be calculated by taking the logarithm of Expression (3) (b), the fourth term can be calculated by taking the logarithm of Expression (4-1), and the fifth term can be calculated by taking the logarithm of Expression (4-2).)

On the other hand, because $Z_{nt}$ considers all the $Z_{nts}$ described above with respect to all the possible s for each t, $r_{nt}$ can be calculated as

[Mathematical 16]

$$r_{nt} = \sum_{s} r_{nts}$$

This corresponds to the E step of the EM method of the present invention.

The step (c) is a step corresponding to the M step of EM method of the present invention, in which based on $r_{nts}$ or $r_{nt}$ which is the posterior probability of $Z_{nts} = 1$ or $Z_{nt} = 1$ obtained in step (b), the maximum likelihood estimate $\theta_t$ that maximizes the log likelihood is calculated as follows.

[Mathematical 17]

$$\theta_t = \frac{\displaystyle\sum_{n',t'=t,\,s'} r_{n't's'}}{N}$$

or,

[Mathematical 18]

$$\theta_t = \frac{\displaystyle\sum_{n',t'=t} r_{n't'}}{N}$$

The steps (d) and (e) are as described above. As a preferred example of the convergence criterion, the relative change $10^{-3}$ is used as the convergence criterion for allele frequencies of the particular gene loci or individual gene locus for the abundance parameter $\theta_t > 10^{-7}$, but different convergence criteria can be also used.

Usually, "iteration of steps (c), (d), and (e)" in step (e) is performed once or more, but it is also possible to do genotyping of alleles of the particular gene loci or individual gene locus without executing the iterative step, or with terminating the step when parameters are not converged.

(2) Variational Bayesian method

Variational Bayesian method is a method for estimating the posterior probability distribution of parameters by Bayesian estimation method, which enables robust estimation of parameters against noise.

The mathematical expression of the complete likelihood and posterior joint distribution of the estimation method of the present invention represented by the expression (1) involves integration over all possible latent variables Z and can not be solved analytically. Therefore, in the complete likelihood and posterior joint distribution, to obtain approximate solutions, the factorization of the latent variables and the model parameters is assumed as below.

[Mathematical 19]

$$q\,(\theta,\;Z)\approx q\,(\theta)\cdot q\,(Z)$$

In the present invention, we use Dirichlet distribution as a prior distribution of $\theta$, as described above.

[Mathematical 20]

$$p(\boldsymbol{\theta}) = \frac{1}{G(\boldsymbol{\alpha})} \prod_{t=1}^{T} \theta_t^{\alpha_t - 1} \qquad (\text{III})'$$

[where $\prod_{t=1}^{T}\theta_t=1$, T is the number of alleles of the particular gene loci, $\alpha_t$ is a hyperparameter,

[Mathematical 21]

$$G(\boldsymbol{\alpha}) = \frac{\prod_t \Gamma(\alpha_t)}{\Gamma(\sum_t \alpha_t)} \quad ,$$

and $\Gamma(\cdot)$ is gamma function.]

In the present invention, based on the assumption that there is no prior knowledge on the relative differences in the allele frequencies of particular gene loci or an individual gene locus such as HLA loci or locus, HLA being human a MHC, it is preferred that a single hyperparameter $\alpha_0$ is used for all the alleles of the particular gene loci or individual gene locus. The single hyperparameter $\alpha_0$ controls the complexity of the target parameter, that is, the number of $\theta_t > 0$. When $\alpha_0 \cong 1$, $\alpha_0 - 1$ can be interpreted as a prior count of the read assigned to the allele of the particular gene loci or individual gene locus, and when $\alpha_0 < 1$, the prior distribution favors a tendency for some of the allele frequencies at the particular gene loci or individual gene locus to be zero. Specifically, it is preferable to set $0 < \alpha_0 \leq 0.1$, or $\alpha_0$ that maximizes the lower bound of the log marginal likelihood. When prior information of allele frequencies is known, it is also possible to weight $\alpha_t > 0$ so that $\alpha_t$ whose allele frequency is higher in prior information is given a larger value in descending order for each t. Given the hyperparameter $\alpha_0$, the lower bound of the log marginal likelihood is iteratively maximized by the variational Bayesian estimation algorithm.

The estimation method of the present invention by variational Bayesian method is exemplified as the estimation method of the present invention characterized by carrying out the following steps (a) to (d) (this method is referred to as a "variational Bayesian method of the present invention").

(a) A step in which the first update posterior distribution of $Z_{nts}$ or $Z_{nt}$ is calculated, based on a given initial posterior distribution of $\theta_t$ based on a given initial value of the hyperparameter $\alpha_0$ representing prior information of allele frequencies of the particular gene loci or individual gene locus, and the first updated posterior distribution of $\theta_t$ is further calculated, based on the first updated posterior distribution of $Z_{nts}$ or $Z_{nt}$, or, (b) A step in which the first updated posterior distribution of $\theta_t$ is calculated, based on a given initial distribution of $Z_{nts}$ or $Z_{nt}$,

(c) A step in which an updated posterior distribution of $Z_{nts}$ or $Z_{nt}$ is calculated, based on the updated posterior distribution of $\theta_t$ calculated by the previous step (d) (however, for the first time, it is step (a) or step (b)),

(d) A step in which an updated posterior distribution of $\theta_t$ is calculated, based on the updated posterior distribution of $Z_{nts}$ or $Z_{nt}$ calculated in step (c), and

(e) A step in which convergence of an expected value of the updated posterior distribution of $\theta_t$ calculated in step (d) is evaluated, and if convergence is recognized, the expected value of $\theta_t$ is determined as the final estimate value, and if convergence is not recognized, iteration of steps (c), (d) and (e) is determined.

The step (a) described above is a step of defining the initial value of this method for iterative optimization. Specifically, it is preferable to set $\alpha^*_t = (N / T + \alpha_0) / \sum_t (N / T + \alpha_0)$ for each allele t of the particular gene loci or individual gene locus, where $\alpha^*_t$ is a parameter of $q^*(\theta)$, assuming that each allele frequency of the particular gene loci or individual gene locus is equal.

The step (b) is a step to calculate $E_z [Z_{nts}]$ or $E_z [Z_{nt}]$ given the current estimate of $q^*(\theta)$. The value for n in $E_z [Z_{nts}]$ is the number from 1 to N, the value for t is the number from 1 to T, the value for s is the number $1 \leq s \leq l_t - L + 1$, where $l_t$ is the length of allele t of the particular gene loci or individual gene locus, and L is the read length. $E_z[Z_{nts}]$ is calculated based on the current estimate of $q^*(\theta)$ as below.

[Mathematical 22]

$$E_Z[Z_{nts}] = \begin{cases} \dfrac{\rho_{nts}}{\sum_{(t',s') \in \pi_n} \rho_{nt's'}} & \text{if } (t,s) \in \pi_n \\[2ex] 0 & \text{otherwise} \end{cases}$$

Here, for the single-end reads, $\rho_{nts}$ is calculated as

[Mathematical 23]

$$\log \rho_{nts} = E_\theta[\log\theta_t] + \log p(S_n|T_n) + \log p(R_n|T_n, S_n)$$

(where the first term on the right side can be calculated by taking an expected value of the logarithm of Expression (1)(a), the second term can be calculated by taking the logarithm of Expression (1)(c), and the third term can be calculated by taking the logarithm of Expression (2).)

For the paired-end reads, $\rho_{nts}$ is calculated as

[Mathematical 24]

$$\log \rho_{nts} = \sum_{f=L}^{l_t - s + 1} \{E_\theta[\log\theta_t] + \log p(F_n = f|T_n) + \log p(S_n|T_n, F_n = f) + \log p(R_{na}|T_n, S_n)$$

$$+ \log p(R_{nb}|T_n, F_n, S_n)\}$$

(where the first term within the brackets on the right side can be calculated by taking an expected value of the logarithm of Expression (3)(a), the second term can be calculated by taking the logarithm of Expression (3)(a'), the third term can be calculated by taking the logarithm of Expression (3)(b), the fourth term can be calculated by taking the logarithm of Expression (4-1), and the fifth term can be calculated by taking the logarithm of Expression (4-2).)

On the other hand, because $Z_{nt}$ considers all the $Z_{nts}$ described above with respect to all the possible s for each t, $r_{nt}$ can be calculated as

[Mathematical 25]

$$r_{nt} = \sum_s r_{nts}$$

It is also possible to perform a series of calculations using $Z_{nt}$ instead of $Z_{nts}$, and to execute the subsequent steps.

Here,

[Mathematical 26]

$$E_\theta[\log\theta_t] = \psi(\alpha_t^*) - \psi(\sum_t \alpha_t^*)$$

(where $\psi(\cdot)$ is digamma function.)

The step (c) is a step of calculating $E_\theta[\theta_t]$ given the current estimate of q*(Z).

$E_\theta[\theta_t]$ is calculated based on the current estimate of q*(Z) as follows.

[Mathematical 27]

$$E_\theta[\theta_t] = \frac{\alpha_t^*}{\sum_{t'} \alpha_{t'}^*}$$

$$\alpha_t^* = \alpha_0 + \sum_{n',t'=t,\,s'} E_Z[Z_{n't's'}]$$

(where                                                    )

Therefore, q*(θ) is also a Dirichlet distribution, and the prior distribution p(θ) is a conjugate prior distribution.

In the steps (d), as a preferred example of the convergence criterion, the relative change $10^{-3}$ is used as the convergence criterion for allele frequencies of the particular gene loci or individual gene locus for the expected value of the abundance parameter, $E_\theta[\theta_t] > 10^{-7}$, but different convergence criteria can be also used.

Here, we investigate the lower bound of the log marginal likelihood and show that the converged value obtained by updating the variational Bayesian method of the present invention approximates the true objective parameter θ.

The log marginal likelihood of the present invention is decomposed as

[Mathematical 28]

$$\log p(\boldsymbol{R}) = L(\boldsymbol{q}) + KL(\boldsymbol{q} \| \boldsymbol{p})$$

(where

[Mathematical 29]

$$L(\boldsymbol{q}) = \iint q(\boldsymbol{\theta}, \boldsymbol{Z}) \log \frac{p(\boldsymbol{R}, \boldsymbol{\theta}, \boldsymbol{Z})}{q(\boldsymbol{\theta}, \boldsymbol{Z})} \, d\boldsymbol{\theta} d\boldsymbol{Z} \;,$$

$$KL(\boldsymbol{q} \| \boldsymbol{p}) = -\iint q(\boldsymbol{\theta}, \boldsymbol{Z}) \log \frac{p(\boldsymbol{\theta}, \boldsymbol{Z} | \boldsymbol{R})}{q(\boldsymbol{\theta}, \boldsymbol{Z})} \, d\boldsymbol{\theta} d\boldsymbol{Z} \;)$$

Since KL (q ‖ p) is the Kullback-Leibler divergence between q(θ, Z) and p(θ, Z | R), the lower bound of the log marginal likelihood is given by L(q). That is, since the Kullback-Leibler divergence is always 0 or more, L(q) constitutes the lower bound of the log marginal likelihood. It is generally proved that L(q) (the lower bound of the log marginal likelihood) is increased each time the steps (b) and (c) are repeatedly updated (Bishop CM. Pattern Recognition and Machine Learning. Springer Science:Business Media, LLC, New York, NY, USA; 2006).

Based on the factorization assumption

[Mathematical 30]

$$q(\theta, Z) \approx q(\theta) \, q(Z)$$

as described above, L(q) is calculated as follows.

[Mathematical 31]

$$
\begin{aligned}
L(\boldsymbol{q}) &= E[\log p(\boldsymbol{R}, \boldsymbol{\theta}, \boldsymbol{Z})] - E[\log q(\boldsymbol{\theta}, \boldsymbol{Z})] \\
&= E[\log p(\boldsymbol{R}, \boldsymbol{Z} | \boldsymbol{\theta})] + E[\log p(\boldsymbol{\theta})] - E[\log q(\boldsymbol{\theta})] - E[\log q(\boldsymbol{Z})]
\end{aligned}
$$

(where

[Mathematical 32]

$$E[\log p(\boldsymbol{R}, \boldsymbol{Z}|\boldsymbol{\theta})] = \sum_{n,t,s} E_Z[Z_{nts}]\log\rho_{nts} \quad,$$

$$E[\log p(\boldsymbol{\theta})] = \sum_{t}(\alpha_0 - 1)E_\theta[\log\theta_t] - \log G(\boldsymbol{\alpha}) \quad,$$

$$E[\log q(\boldsymbol{\theta})] = \sum_{t}(\alpha_t^* - 1)E_\theta[\log\theta_t] - \log G(\boldsymbol{\alpha}^*) \quad,$$

$$E[\log q(\boldsymbol{Z})] = \sum_{n,t,s} E_Z[Z_{nts}]\log E_Z[Z_{nts}] \quad )$$

Usually, "iteration of steps (c), (d), and (e)" in step (e) is performed once or more, but it is also possible to do genotyping of alleles of the particular gene loci or individual gene locus without executing the iterative step, or with terminating the step when parameters are not converged.

(3) The computer system and computer program using the EM algorithm

(i) As a computer system using the EM algorithm described above, for example, the following computer system can be described. An embodiment of the computer system of the present invention is a computer system characterized in that a computer executes an estimation method using the EM algorithm described above.
 The computer system of the present invention is a computer system for optimizing, for each read, the number of expected mappings to each allele of the gene loci or individual gene locus for the data where read mapping information of the particular gene loci or individual gene locus is mixed, comprising a recording unit and an arithmetic processing unit, which is characterized in that all or a part of the following processes (A) to (E), is executed:

(A) In the recording unit, read information of DNA derived from the subject is recorded as observed data of nucleotide sequence of read and alleles of the gene loci or individual gene locus to which the read is mapped to,
(B) In the arithmetic processing unit, based on the observed data retrieved from the recording unit, either one of the following initialization processes (B)-1 and (B)-2 is executed:

(B)-1: The process of calculating the initial value of $\theta$, which represents allele frequency of the gene loci or individual gene locus, and
(B)-2: The process of calculating the initial values of two latent variables, (a) and (b) described below, that are related to the $\theta$ described above and the data of DNA read information of the subject, which is the observed data:

(a) A variable $T_n$ that is dependent on $\theta$, which represents allele selection of read n in the gene loci or individual gene locus, and
(b) The posterior probability of an indicator variable $Z_{nts}$ ($Z_{nts}$ equals to one if $(T_n, S_n) = (t, s)$, and zero otherwise), which summarizes $S_n$, the start position of read n that is dependent on $T_n$, or $Z_{nt}$ ($Z_{nt}$ equals to one if $T_n = t$, and zero otherwise), which summarizes the latent variable $T_n$,

(C) In the arithmetic processing unit, based on the parameter $\theta$ calculated in the process (B)-1 described above, the calculation process of the posterior probability of indicator variable $Z_{nts}$ or $Z_{nt} = 1$ is exerted,
(D) In the arithmetic processing unit, the first updated value of the maximum likelihood estimate of parameter $\theta$ is calculated based on the posterior probability of indicator variable $Z_{nts}$ or $Z_{nt} = 1$ calculated in the process (B)-2 or (C),
(E) In the arithmetic processing unit, the processes (C) and (D) described above are executed again based on the first updated value of the maximum likelihood estimate of $\theta$ calculated in the process (D) described above, and the iterative loop process for calculating the second updated value of $\theta$ is repeatedly executed, until difference between the new updated value of $\theta$ and the previous updated value of $\theta$ is not recognized, and the converged parameter $\theta$ is recognized as the optimized value and recorded in the recording unit described above.

(ii) As a computer program using the EM algorithm described above, for example, the following computer program can be considered. The embodiment of the computer program of the present invention is a computer program characterized by realizing the estimation method using the EM algorithm in a computer.

The computer program is a computer program for optimizing, for each read, the number of expected mappings to each allele of the gene loci or locus for the data where read mapping information of the particular gene loci or individual locus is mixed, which is characterized in that all or a part of the following functions 1 to 5, is realized:

(A) Function 1 in which read information of DNA derived from the subject recorded as observed data of nucleotide sequence of read and alleles of the loci or locus to which the read is mapped to, is retrieved from the recording unit,
(B) Function 2 in which based on the observed data retrieved by function 1 described above, either one of the following initialization processes (B)-1 and (B)-2 is executed:

(B)-1: A process of calculating the initial value of $\theta$, which represents allele frequency of the gene loci or individual gene locus, and
(B)-2: A process of calculating the initial values of two latent variables, (a) and (b) described below, that are related to the $\theta$ described above and the data of DNA read information of the subject, which is observed data:

(a) A variable $T_n$ that is dependent on $\theta$, which represents allele selection of read n in the gene loci or individual gene locus, and
(b) The posterior probability of an indicator variable $Z_{nts}$ ($Z_{nts}$ equals to one if $(T_n, S_n) = (t, s)$, and zero otherwise), which summarizes $S_n$, the start position of read n that is dependent on $T_n$, or $Z_{nt}$ ($Z_{nt}$ equals to one if $T_n = t$, and zero otherwise), which summarizes the latent variable $T_n$,

(C) Function 3 in which based on the parameter $\theta$ calculated in the process (B)-1 described above, the calculation process of the posterior probability of indicator variable $Z_{nts}$ or $Z_{nt}$ is executed,
(D) Function 4 in which the first updated value of the maximum likelihood estimate of parameter $\theta$ is calculated based on the posterior probability of indicator variable $Z_{nts} = 1$ or $Z_{nt} = 1$ calculated in the process (B)-2 or function 3,
(E) Function 5 in which function 3 and 4 described above are executed again based on the first updated value of the maximum likelihood estimate of $\theta$ calculated in the function 4 described above, and the iterative loop process for calculating the second updated value of $\theta$ is repeatedly executed, until difference between the new updated value of $\theta$ and the previous updated value of $\theta$ is not recognized, and the converged parameter $\theta$ is recognized as the optimized value and recorded in the recording unit described above.

(4) The computer system and computer program using the variational Bayesian method

(i) As a computer system using the variational Bayesian method described above, for example, the following computer system can be considered. The embodiment of the computer system of the present invention is a computer system characterized by realizing the estimation method using the variational Bayesian method in a computer.

The computer system of the present invention is a computer system for optimizing, for each read, the number of expected mappings to each allele of the gene loci or locus for the data where read mapping information of the particular gene loci or individual gene locus is mixed, comprising a recording unit and an arithmetic processing unit, which is characterized in that all or a part of the following processes (A) to (E), is executed:

(A) In the recording unit, read information of DNA derived from the subject is recorded as observed data of nucleotide sequence of read and alleles of the loci or locus to which the read is mapped to,
(B) In the arithmetic processing unit, based on the observed data retrieved from the recording unit, either one of the following initialization processes (B)-1 and (B)-2 is executed:

(B)-1: A process of calculating the updated value of the posterior distribution of $\theta$, based on the initial value of hyperparameter $\alpha_t$ which represents prior information of allele frequency of the gene loci or individual gene locus, and

(B)-2: A process of calculating the initial distribution of two latent variables, (a) and (b) described below, that are related to the distribution of θ described above and the data where DNA read information of the subject is mixed, which is the observed data:

(a) A variable $T_n$ that is dependent on θ, which represents allele selection of read n in the gene loci or individual gene locus,
(b) The posterior distribution of an indicator variable $Z_{nts}$ ($Z_{nts}$ equals to one if ($T_n$, $S_n$) = (t, s), and zero otherwise), which summarizes $S_n$, the start position of read n that is dependent on $T_n$, or $Z_{nt}$ ($Z_{nt}$ equals to one if $T_n$ = t, and zero otherwise), which summarizes the latent variable $T_n$,

(C) In the arithmetic processing unit, based on the distribution of parameter θ calculated in the process (B)-1 described above, the calculation process of the posterior distribution of indicator variable $Z_{nts}$ or $Z_{nt}$ is exerted,
(D) In the arithmetic processing unit, the first updated posterior distribution of parameter θ is calculated based on the updated posterior distribution of $Z_{nts}$ or $Z_{nt}$ calculated in the process (B)-2 or (C),
(E) In the arithmetic processing unit, the processes (C) and (D) described above are executed again based on the first updated posterior distribution of θ calculated in the process (D), and the iterative loop process in which the second updated posterior distribution of θ is calculated is repeatedly executed, until difference between the expected value of the new updated posterior distribution of θ and the expected value of the previous updated posterior distribution of θ is not recognized, and the converged expected value of the posterior distribution of θ is recognized as the optimized value and recorded in the recording unit described above.

(ii) As a computer program using the variational Bayesian method described above, for example, the following computer program can be considered. The embodiment of the computer program of the present invention is a computer program characterized by realizing the estimation method using the variational Bayesian method in a computer.

The computer program is a computer program for optimizing, for each read, the number of expected mappings to each allele of particular gene loci or individual gene locus for the data where read mapping information of the particular gene loci or individual gene locus is mixed, which is characterized in that all or a part of the following functions 1 to 5, is realized:

(A) Function 1 in which read information of DNA derived from the subject recorded as observed data of nucleotide sequence of read and alleles of the loci or locus to which the read is mapped, is retrieved from the recording unit,
(B) Function 2 in which based on the observed data retrieved by function 1 described above, either one of the following initialization processes (B)-1 and (B)-2 is executed:

(B)-1: A process of calculating the updated posterior distribution of θ, based on the initial value of hyper-parameter $α_t$ which represents the prior information of allele frequency of the gene loci or individual gene locus, and
(B) -2: A process of calculating the posterior distribution of two latent variables, (a) and (b) described below, that are related to the distribution of θ described above and the data where DNA read information of the subject is mixed, which is the observed data:

(a) A variable $T_n$ that is dependent on θ, which represents allele selection of read n in the gene loci or individual gene locus,
(b) The initial posterior distribution of an indicator variable $Z_{nts}$ ($Z_{nts}$ equals to one if ($T_n$, $S_n$) = (t, s), and zero otherwise), which summarizes $S_n$, the start position of read n that is dependent on $T_n$, or $Z_{nt}$ ($Z_{nt}$ equals to one if $T_n$ = t, and zero otherwise), which summarizes the latent variable $T_n$,

(C) Function 3 in which based on the distribution of θ calculated in the process (B)-1 described above, the calculation process of the posterior distribution of indicator variable $Z_{nts}$ or $Z_{nt}$ is executed,
(D) Function 4 in which the first updated value of the posterior distribution of θ is calculated based on the updated posterior distribution of $Z_{nts}$ or $Z_{nt}$ calculated in the process (B)-2 or function 3,
(E) Function 5 in which function 3 and 4 described above are executed again based on the first updated value of the posterior distribution of θ calculated in the function 4 described above, and the iterative loop process in which the second updated value of the posterior distribution of θ is calculated is repeatedly executed, until difference between the expected value of the new updated posterior distribution of θ and the expected value of the previous updated posterior distribution of θ is not recognized, and the converged expected value of θ is recognized as the optimized value and recorded in the recording unit described above.

[B] A more specific form of the method of the present invention

Here, we disclose an example where the particular gene loci are HLA loci, HLA being a human MHC, but the algorithm disclosed herein may be applied to other loci, cytochrome P450 loci, immunoglobulin gene loci, T cell receptor gene loci, and olfactory receptor gene loci. The method of the present invention is used as a generic name of the optimization method and determination method, as described above.

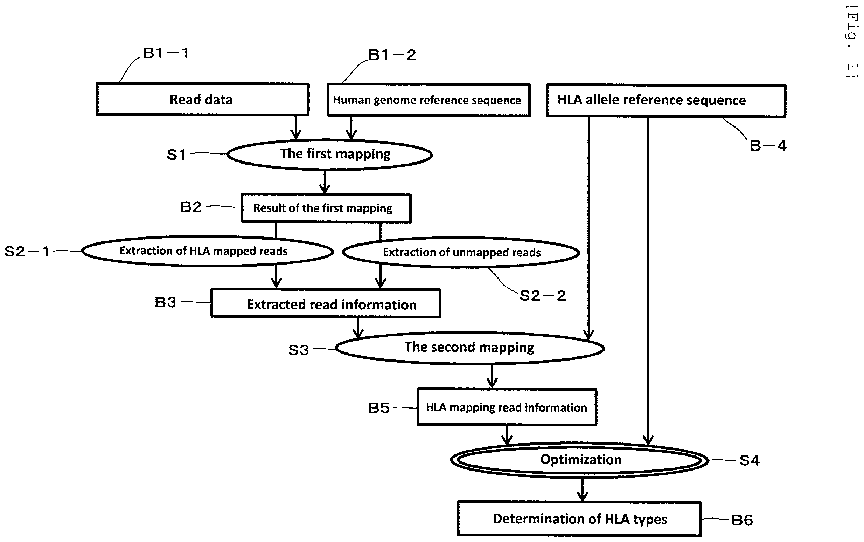

FIG. 1 is a process flow of the method of the present invention for predicting the genotype of a subject's HLA locus. Each element of this flow of processing is all performed electronically on the computer based on an instruction to realize an algorithm of computer software via a computer network or a database as necessary.

Box B1-1 indicates the presence of read data and box B1-2 indicates the presence of a human genome reference sequence. As described above, the "read data" (box B1-1) is electronic information of nucleotide sequence of a part or all of the individual DNA fragments (reads) obtained by processing the subject's DNA sample with a high-throughput sequencer, and normally, information indicating the reading accuracy is also added (FASTQ: a term derived from the FASTA format representing the nucleotide sequence of DNA). At this stage, "depth of coverage of total reads" is determined. The "human genome reference sequence" (box B1-2) is constructed from one or multiple human genome sequence information as described above, and its source of supply is not particularly limited here. It is also assumed that the ethnicity of the subject and that of individuals from which the human genome reference sequence is constructed are different, and there exists heterogeneity with respect to the genome sequence in HLA genes. The substantial effect of the present invention does not depend on the version change of the reference sequence, and it is always possible to determine HLA types of the subject in correspondence with the changes of the reference sequence, and as the latest reference sequence provided at present or the one that will be provided in the future can be used. Examples of human genome reference sequences include the human genome reference sequence (such as hgl9) managed by UCSC (University of California Santa Cruz), the human genomic reference sequence (such as GRCh37) managed by the Genome Reference Consortium, and others, but it is not limited to these examples. As described above, for example, sequence data obtained from targeted sequencing, Exome sequencing, RNA sequencing, and long-read sequencing such as with PacBio RSII and Oxford Nanopore and others can also be used as a reference sequence.

The step S1 is a step of performing the first mapping, or, a step of mapping the "read data" of the box B1-1 to the "human genome reference sequence" of the box B1-2. With regard to this mapping, those skilled in this field can create computer programs including algorithms capable of realizing the mapping with computers, but it is also possible to use existing software. The existing mapping software includes, for example, BWA-MEM (http://bio-bwa.source.net/), Bowtie 2 (http://bowtie-bio.sourceforge.net/bowtie 2/index.shtml), Novoalign (http://www.novocraft.com/products/novoalign/), and others. Also, when the read data is paired-end sequence data, if one of the nucleotide sequences (pairs) of both ends of the read is mapped to the HLA locus and the other is not mapped, it is preferable to extract both reads of the pair and use them in the following processes.

Furthermore, it is preferable that a "decoy sequence" is included in addition to the human genome reference sequence described above. A "decoy sequence" is a genomic sequence that does not exist in a reference sequence prepared in advance. This is because, without using a decoy sequence, there is a high risk that reads that are not derived from sequences registered in the human genome reference sequence will be mapped to some places in the existing human genomic reference sequences, possibly reducing mapping accuracy. As a decoy sequence, hs37d5 (ftp://ftp.1000genomes.ebi.ac.uk/vol1/ftp/technical/referenc e/phase2_reference_assembly_sequence/hs37d5.fa.gz) and others can be used.

The box B2 is a box showing the existence of "the result of the first mapping" in the above S1, and the mapping result exists in the format such as the SAM format or the BAM format.

Step S2-1 is a step of "extracting reads mapped to HLA loci" from "the result of the first mapping" in the box B2, and step S2-2 is a step of performing "extraction of unmapped reads" that are not mapped to any portion of the human genome reference sequence. With respect to the execution of these extraction, it is possible for those skilled in the field to create computer programs including algorithms that can realize the extraction with a computer, but it is also possible to use existing software. As existing extracting software, for example, SAMtools (http://samtools.sourceforge.net/) can be mentioned. In this extraction step, it is preferable to carry out calculation as efficient as possible because the necessary processing to be performed spans at least all HLA loci, which are many loci. Therefore, it is preferable that the computer program mentioned above is designed so that the extraction is carried out on all HLA loci (currently, HLA-A, -B, -C, -DM, -DO, -DP, -DQ, -DR, -E, -F, -G, -H, -J , - K, - L, - P, - V, - MIC, and -TAP are registered in the IMGT / HLA database). With SAMtools, extraction processing can be easily performed. It is preferable that "Extraction of unmapped reads" in step S2-2 is set as a step to prevent the loss of reads derived from HLA loci that were not mapped to the reference genomic sequence due to polymorphism in the subject's genomic sequence as described above.

Through these steps in S2, the information of reads that were mapped to regions other than HLA loci are excluded.

The box B3 is a box showing the presence of the read information (extracted reads) extracted in the above step S2 and the box B4 is a box showing the existence of the information of "reference sequence of HLA allele". Reference sequence information of HLA allele is electronic information derived from a database in which genome information of HLA allele is stored. As the database, for example, IMGT/HLA (http://www.ebi.ac.uk/ipd/imgt/hla/), and others can be mentioned. It is preferable to acquire the latest update information from these database information as much as possible. In addition, it is preferable that HLA alleles of "pseudogenes" which are genes that do not actually code for proteins are also included in the genomic information of HLA allele.

Step S3 is a step in which "second mapping" to "reference sequence of HLA allele" in box B4 of "extracted read information" in box B3 is executed. It is preferable that the mapping software is designed so that the execution mode of the second mapping is performed so as to allow one read to be mapped to multiple HLA alleles, and it is possible by specifying "-a option" with BWA-MEM mentioned above and "-k option" with Bowtie 2, which make it possible for the computer to realize the multiple mapping of reads to HLA alleles.

Box B5 is a box showing "the result of the second mapping" in step S3, that is, the presence of "HLA mapping read information", and the information exists in a format such as the SAM format or BAM format.

The "mapping process of the present invention" described above is shown until this step in FIG. 1.

Hereinafter, the step S4 is a step of optimizing the "HLA mapping read information" described above, and details thereof are shown in FIG. 2 (FIGS. 2-1, 2-2, 2-3) as a flow sheet. FIG. 2-1 shows embodiments (variational Bayesian method and EM algorithm) of the optimizing method of the present invention using the latent variable $Z_{nt}$, FIG. 2-2 shows an embodiment using the EM algorithm in the optimization method of the present invention using the latent variable $Z_{nts}$, and FIG. 2-3 shows an embodiment using the variational Bayesian method in the optimization method of the present invention using the latent variable $Z_{nts}$. The detailed calculation steps involved in these embodiments are as described above for alleles of the particular gene loci. Furthermore, the box B6 is a box showing the presence of electronic information of the desired "Determination of HLA type", but in the process from step S4 to box B6, the HLA typing process is preferably executed using the rejection threshold shown in FIG. 3 (described later).

[An embodiment of the optimization method using the latent variable $Z_{nt}$]

FIG. 2-1 is a flow sheet showing one embodiment of the optimization process using the latent variable $Z_{nt}$ of "HLA mapping read information" as described above. This flow sheet is an embodiment in which prediction by the variational Bayesian method is performed for Bayesian estimation to obtain expected read counts on HLA alleles, but it is also possible to perform prediction by the EM algorithm in order to perform maximum likelihood estimation, which does not need incorporation of the hyperparameter $\alpha_0$ as described below.

Step S4-11 is a step of retrieving "HLA allele reference sequence" shown in the box B4 and retrieving "HLA mapping read information" shown in the box B5.

Step S4-12 is a step in which the number of expected mappings $E[Z_{nt}]$ to each HLA allele of the read n used in the optimization process by executing the computer program of the present invention, and the expected value of the fraction of the total number of expected mappings allocated to each HLA allele relative to the total read amount, $E[\theta_t]$, are initialized.

Step S4-13 is a step in which the total number of expected mappings for each HLA allele for all reads based on the initialized values obtained in step S4-12 described above is calculated. The definition of the mathematical expression described here is as described above. The $r_t$ is "the total number of expected mappings assigned to HLA allele t".

Step S4-14 is a step in which the "total number of expected mappings" for each HLA allele, calculated in step S4-13 described above is divided by the sum of the total number of expected mappings for all HLA alleles, and the expected value of the fraction of the total number of expected mappings allocated to each HLA allele relative to the total read amount, $E[\theta_t]$, is calculated. In this step S4-14, the hyperparameter $\alpha_0$ has been incorporated as described above, in which an embodiment of the variational Bayesian method is described. For example, $\alpha_0 = 1$ which gives a uniform distribution is used as the hyper parameter $\alpha_0$ assuming that there is no prior knowledge, but $\alpha_0$ that maximizes the lower bound of the log likelihood, for example, $\alpha_0 = 0.01$ is also preferably used. Furthermore, as described above, it is also possible to use it as a flow sheet of an embodiment of the EM algorithm for performing maximum likelihood estimation of the parameter $\theta_t$ without incorporating this hyperparameter.

Step S4-15 is a step related to loop processing, and

(i) The function in which the distribution of the fraction of the total number of expected mappings calculated in step S4-14 described above is assigned to each HLA allele as a frequency distribution, and based on the allocated frequency distribution, again in step S4-13 described above, the number of expected mappings for each HLA allele for each read is calculated,

(ii) The function in which based on the number of expected mappings for each HLA allele for each read calculated in the function (i) described above, step S4-13 or S4-14 is executed again for calculating the updated distribution of the fraction of reads assigned to each HLA allele relative to the total read amount mapped to HLA alleles,

(iii) The function in which the calculation processes in the (i) and (ii) described above are repeatedly executed, until the difference between the number of expected mappings to each HLA allele for each read calculated by executing the current (i) and that calculated by executing the previous (i) is not recognized for all the reads, or, the difference between the expected value of the distribution of the fraction of the total number of expected mappings calculated by executing the current (ii) and that calculated by executing the previous (ii) is not recognized for all HLA alleles, and the converged expected number of mappings to each HLA allele for each read or the converged expected value of the distribution of the fraction of reads for each HLA allele is recognized as the optimized data as "convergence value";

are included in the description of step S4-15.

[Embodiment of the optimization method using latent variable $Z_{nts}$ (1)]

FIG. 2-2 is a flow sheet showing an embodiment of the optimization process using the latent variable $Z_{nts}$ of "HLA mapping read information" as described above. This flow sheet describes prediction of the expected read counts on HLA alleles by maximum likelihood estimation with the EM algorithm.

Step S4-21 is a step of retrieving the "HLA mapping read information" in the same manner as in step S4-11 described above.

Step S4-22 is a step in which a latent variable $Z_{nts}$ (a latent variable taking 1 in the case where the read n is generated from the position s of the HLA allele t) and $\theta_t$, the fraction of reads allocated to each HLA allele relative to the total read amount, used in the optimization process by executing the computer program, are initialized. The expected value of the latent variable $Z_{nts}$ is described as $E[Z_{nts}]$. Here, N is the "total number of reads", and T is the "total number of HLA alleles". $l_t$ is the length of the HLA allele t, and L is the read length. $M_n$ is the total number of observed mapped locations of the read n. $E[Z_{nts}]$ is initialized as $1 / M_n$, given an assumption of no prior information / uniform distribution. $\theta_t$ is given as $1 / T$ as an initial value, given an assumption of no prior information / uniform distribution.

Step S4-23 is a step in which for all reads, the number of expected mappings to each HLA allele based on the initialized information retrieved in step S4-22 is calculated. That is, this is a step in which the expected value of the latent variable $Z_{nts}$ ($E_z[Z_{nts}]$) indicating the allocation of read n to the position s of the HLA allele t, based on the $\theta_t$ updated immediately before (for the first time, it is the initial values in step S4-22 described above), that is, the "expected number of mappings", is calculated, which corresponds to the E step of the EM method.

Step S4-24 is a step in which the "expected number of mappings" calculated in step S4-23 described above is summed for each HLA allele and the "total number of expected mappings" ($r_t = \sum_{n',t'=t,s'} E_z[Z_{n't's'}]$) is calculated, and the process in which the total number of expected mappings is divided by the sum of the total number of expected mappings for all HLA alleles to calculate the maximum likelihood estimate of the fraction of reads assigned to each allele relative to the total read amount "$\theta_t$" ($\theta_t = r_t / \sum_t \cdot r_{t'}$), is executed, which corresponds to the M step of the EM method. This step corresponds to calculating $\theta_t$ that locally maximizes the log likelihood.

Step S4-25 is a step related to loop processing. That is, steps S4-23 and S4-24 are executed again based on the first updated value of the maximum likelihood estimate of $\theta_t$ calculated in step S4-24, and the loop processing in which the second updated value of the parameter $\theta$ is calculated is repeatedly executed until the significant difference between the new updated value and the previously updated value is not recognized for all HLA alleles, and the converged parameter $\theta$ is recorded on the recording unit as the optimized $\theta$.

[Embodiment of the optimization method using latent variable $Z_{nts}$ (2)]

FIG. 2-3 is a flow sheet showing an embodiment of the optimization process using the latent variable $Z_{nts}$ of "HLA mapping read information" as described above. This flow sheet describes prediction of the expected read counts on HLA alleles and HLA allele frequency by variational Bayesian method.

Step S4-31 is a step of retrieving the "HLA mapping read information" in the same manner as in step S4-11 described above.

Step S4-32 is a step in which the posterior distribution of a latent variable $Z_{nts}$ (a latent variable taking 1 in the case where the read n is generated from the position s of the HLA allele t, and 0 otherwise) and the posterior distribution of $\theta_t$ (the fraction of reads allocated to each HLA allele relative to the total read amount), used in the optimization process by executing the computer program, are initialized. The expected value of the posterior distribution of the latent variable $Z_{nts}$ is described as $E[Z_{nts}]$. Here, N is the "total number of reads", and T is the "total number of HLA alleles". $l_t$ is the length of the HLA allele t, and L is the read length. $M_n$ is the total number of observed mapped locations of the read n. $E[Z_{nts}]$ is initialized as $1 / M_n$, given an assumption of no prior information / uniform distribution. The expected value of

$\theta_t$, $E_\theta[\theta_t]$, is given as $\alpha^*_t/\sum_{t'}\alpha^*_{t'}$. Here, $\alpha^*t=\alpha_0+N/T$. As described above, $\alpha_0$ is a hyperparameter. For example, assuming that there is no prior information, $\alpha_0 = 1$ that gives a uniform distribution is used, but it is also preferable to use $\alpha_0$, which maximizes the lower bound of log likelihood, for example, $\alpha_0 = 0.01$.

Step S4-33 is a step in which for all reads, the number of expected mappings to each HLA allele based on the initialized information retrieved in step S4-32 is calculated. That is, this is a step in which the expected value of the posterior distribution of the latent variable $Z_{nts}$, $E_z[Z_{nts}]$, indicating the allocation of read n to the position s of the HLA allele t, based on the posterior distribution of $\theta_t$ updated immediately before (for the first time, it is the initial values in step S4-32 described above), that is, the "expected number of mappings", is calculated, which corresponds to the E step of the variational Bayesian method.

Step S4-34 is a step in which the posterior distribution of $\theta_t$ is calculated based on the "expected number of mappings" ($E_z[Z_{nts}]$) calculated in step S4-33 described above, which corresponds to the M step of the variational Bayesian method, and the expected value of the posterior distribution of $\theta_t$, $E_\theta[\theta_t]$, is calculated based on the $E_z[Z_{nts}]$ calculated in step S4-34 as

[Mathematical 33]

$$E_\theta[\theta_t] = \frac{\alpha^*_t}{\sum_{t'}\alpha^*_{t'}}$$

$$\left(\text{where } \alpha^*_t = \alpha_0 + \sum_{n',t'=t,\,s'} E_Z[Z_{n't's'}]\right)$$

Step S4-35 is a step of selecting whether to repeat steps S4-33 and S4-34 described above, or to end the loop process.

That is, steps S4-33 and S4-34 are executed again based on the first updated posterior distribution of the parameter $\theta_t$ calculated in step S4-24, and the loop processing of calculating the second updated posterior distribution of the parameter $\theta$ is iteratively executed until the significant difference between the expected value of the newly updated posterior distribution and the expected value of the posterior distribution updated last time is not recognized, and the converged expected value of $\theta$ is recorded in the recording unit as the data of optimized $\theta$.

It is possible to use the obtained "$r_t$" or "$\theta_t$" as an indicator of HLA typing, but it is preferable that the following HLA typing process is performed.

FIG. 3 is a flow sheet showing an example of the HLA typing process using a rejection threshold for the individual depth of coverage that is determined relative to the depth of coverage of all reads, based on "the fraction of reads assigned to each HLA allele t "$\theta_t$", or the expected value of the read fraction "$\theta_t$" ", obtained through the optimization step described above.

Step S5-1 is a step in which the "individual depth of coverage" of each HLA allele is calculated from "the fraction of reads assigned to each HLA allele". The variables "t" and "n" are initialized, and the process of calculating the individual depth "dt" is performed using the mathematical expression indicated in box S5-1. The details of the calculation process here have already been described.

Step S5-2 is a step in which, as described in the box, for each HLA locus, two of the HLA alleles with the two largest individual depth of coverage "dt" are selected, and the allele with the largest individual depth of coverage, and the allele with the second largest individual depth of coverage are specified.

Step S5-3 is a step in which a selection process to determine whether the largest individual depth of coverage "$d_{first}$" of the allele described above is smaller than the rejection depth "D" or not is executed. The rejection depth "D" is a numerical value selected between 5 and 50%, preferably between 10 and 30% of the "depth of coverage of total reads" as described above. If the individual depth of coverage "$d_{first}$" is smaller than the rejection depth "D", a decision is made that "HLA type is not determined" (conclusion D5-1). If it is not smaller, the next selection step S5-4 is executed.

Step S5-4 is a step in which a selection process to determine whether the second largest individual depth of coverage "$d_{second}$" of the allele described above is smaller than the rejection depth "D" or not is executed. If the individual depth of coverage "$d_{second}$" is smaller than the rejection depth "D", a decision is made to go to the selection step S5-5, and if it is not smaller, a decision is made to go to the selection step S5-6.

Step S5-5 is a step that is applied when the individual depth of coverage "$d_{second}$" is smaller than the rejection depth "D". That is, a determination process in which if the "$d_{first}$" described above is greater than twice the rejection depth "D", "the HLA type is homozygous of the HLA allele with the largest individual depth of coverage" (conclusion D5-2), and if it is not larger than 2 times, it is assumed that "the HLA type is determined as heterozygous of the HLA allele with the largest individual depth of coverage and the other allele is not determined" (conclusion D5-3), is performed.

Step S5-6 is a further selection step that is applied when "$d_{second} < D$" is not satisfied in step S5-4, and a determination process in which if "$d_{first}$" is more than twice the individual depth of coverage "$d_{second}$", "the HLA type is homozygous of the HLA allele with the highest individual depth of coverage" (conclusion D5-4), and if it is not more than twice, "the HLA type is heterozygous of the HLA alleles with the largest and the second largest individual depth of coverage" (conclusion D5-5), is performed.

As described above, the system of the present invention can determine the HLA type in the preferred form (box B6 in FIG. 1).

[Examples]

Hereinafter, examples of the present invention performed in which the target is HLA, which is human MHC, are disclosed.

[A] The source used in the examples

The sources used in executing the computer system and the computer program (hereinafter collectively referred to as the "system of the present invention") used in the present examples are as follows.

(1) HLA mapping process of the present invention

HiSeq2000 (Illumina, Inc.) was used as the next-generation sequencer that provides the "read information" of box B1-1 in FIG. 1. The read information is provided in the FASTAQ format, and the subsequent read information is likewise in the FASTAQ format.

As the "human genome reference sequence" in box B1-2, hgl9 (UCSC) or GRCh37 (Genome Reference Consortium) was used in conjunction with decoy sequence (hs37d5).

The mapping of step S1/S3 was performed by BWA-MEM. For step S3, the option "-a" was specified and all possible mapping locations were output for each read.

The "first mapping result" in box B2 and the "second mapping result" in box B5 were used in the BAM format.

As software for extracting the mapping result in step S2, "SAMtools" was used.

"Reference sequence of HLA allele" in box B4 was provided in the FASTA format obtained from the IMGT/HLA database.

(2) The optimization process

The optimization process in step S4 is executed by applying the algorithm described in flow sheet in FIG. 2-1 using the variational Bayesian method to the paired-end data, the rejection depth is set to 20% of the depth of coverage of total reads, and the rejection process was executed by applying the algorithm described in the flow sheet in FIG. 3.

[B]Performance measurement of HLA typing

(1) Overview of the simulation experiment

The prediction performance of the system of the present invention was evaluated from the viewpoint of prediction accuracy. The prediction accuracy is defined as the fraction of the true positive prediction relative to the predicted HLA types. In this simulation experiment, two HLA alleles (either heterozygous or homozygous) for the six HLA loci (HLA-A, -B, -C, -DQA1, -DQB1, -DRB1) were evaluated in each individual. Predictive performance was evaluated separately for each method with two-digit, four-digit, six-digit and eight-digit resolution.

(2)Simulation data analysis

With the simulation data analysis, the performance of the system of the present invention to predict HLA types was evaluated compared to other systems. As the comparable systems, (a) PHLAT (Bai et al., BMC genomics, 15: 325 (2014)) and (b) HLAminer (Warren et al., Genome medicine, 4 (12): 102 (2013)) were used. It is known that these comparable systems can classify HLA class I loci (HLA-A, -B, and -C) and the class II loci (HLA-DQA1, -DQB1, and -DRB1) at a four-digit resolution from human whole-genome sequence data.

First, simulation data of 1,000 individuals of human samples was prepared. For each individual, HLA diplotype of the six HLA loci was randomly selected from HLA alleles registered in the IMGT/HLA database release 3.15.0. Once the HLA type was established for each individual, one SNP for 1,000 bp was incorporated in each individual's HLA allele based on the average polymorphism in the human genome. Next, 100 bp of the paired-end read data (average depths of coverage were 5×, 10×, 20× and 30×, and the mean fragment length and standard deviation of the distribution were set as 300 bp and 40 bp, respectively) was uniformly produced across the HLA allele sequence with 0.1% substitution, deletion and insertion errors.

Table 1 shows the prediction accuracy of the system of the present invention and the existing tool using the 30× simulation data.

[Table 1]

| HLA resolution | System of the present invention | PHLAT | HLAminer |
| --- | --- | --- | --- |
| 8-digit | 99.94% | - | - |
| 6-digit | 99.95% | 80.80% | - |
| 4-digit | 99.95% | 88.75% | 50.12% |
| 2-digit | 100% | 96.39% | 77.82% |

In Table 1, the system of the present invention was able to estimate the HLA type with an especially high accuracy of 99.94% at eight-digit resolution which can not be conducted with existing PHLAT and HLAminer. Comparing the estimation accuracy of the system of the present invention with that of the existing PHLAT and HLAminer, it became clear that the system of the present invention achieved better prediction performance at the four-digit resolution and the six-digit resolution than the existing software. FIG. 4 is a drawing showing prediction accuracy of HLA types at four-digit resolution at various depth of coverage in the simulation data. In FIG. 4, the prediction accuracy when using the system of the present invention was always better than the accuracy with PHLAT and HLAminer throughout the examined depth of coverage.

In particular, in the system of the present invention, HLA type prediction was made at an accuracy of 99.36% at four-digit resolution using the simulation data of average depth of 5×. In PHLAT, only SNP sites are examined in order to compute likelihood, but with only the limited information, in case if other polymorphic sites (such as deletions or insertions) are important for determining the HLA type, it is not effective. Another possible disadvantage of PHLAT is that the system needs prior information about HLA allele frequency. However, the HLA allele frequency is particularly diverse among human populations and it is not always possible to guess the racial roots of the provided gene specimens. In contrast, the system of the present invention does not require prior information on HLA allele frequency.

(3) Real data analysis (1)

The system of the present invention is applied to whole-genome sequencing data of CEU trio samples (NA12878 (child), NA12891 (father) and NA12892 (mother)) used in the international HapMap project, which were not amplified by PCR method. Here, 100 bp paired-end data was generated using HiSeq2000, the average insertion length was 300 bp, and the depth of coverage of these data was 45× for each sample (all data was provided by Illumina, Inc.). Table 2 shows the predicted HLA types for the CEU trio.

[Table2]

| Sample | Predicted HLA types | |
| --- | --- | --- |
| NA 12878 (child) | **A*01:01**:01:01 | **A*11:01**:01 |
| | **B*08:01**:01 | **B*56:01**:01 |
| | **C*01:02**:01 | **C*07:01**:01:01 |
| | DQA1*01:01:02 | DQA1*05:01:01:02 |
| | DQB1*02:01:01 | DQB1*05:01:01:02 |
| | DRB1*03:01:01:01 | DRB1*01:01:01 |
| NA 12891 (father) | **A*01:01**:01:01 | **A*24:02**:01:01 |
| | **B*07:02**:01 | **B*08:01**:01 |
| | **C*07:01**:01:01 | **C*07:02**:01:03 |
| | DQA1*01:02:01:01 | DQA1*05:01:01:02 |
| | DQB1*02:01:01 | DQB1*06:02:01 |

(continued)

| Sample | Predicted HLA types | |
|---|---|---|
| | DRB1*03:01:01:01 | DRB1*15:01:01:02 |
| NA12892 (mother) | **A*02:01**:01:01 | **A*11:01**:01 |
| | **B*15:01**:01:01 | **B*56:01**:01 |
| | **C*01:02**:01 | **C*04:01**:01:01 |
| | DQA1*01:01:02 | DQA1*01:01:02 |
| | DQB1*05:01:01:02 | DQB1*05:01:01:01 |
| | DRB1*01:01:01 | DRB1*01:01:01 |

In Table 2, the HLA types for the class I locus (HLA-A, -B, and -C) predicted by using the system of the present invention are matched with HLA types experimentally verified at four-digit resolution. These are shown in bold text in Table 2. Many of the HLA types were predicted at 8-digit resolution by the system of the present invention. The HLA types of HLA-A, -B and -C loci predicted in the system of the present invention are consistent with HLA types predicted with PHLAT at six-digit resolution except for "B*07:02:01" (one allele in NA12891). Another literature (Major et al., PloS one, 8(11): e78410 (2013)) also reported one of the HLA-B alleles of NA12891 as "B*07:02:01", which is consistent with the prediction with the system of the present invention. PHLAT, on the other hand, predicted this HLA type as "B*07:02:29". Overall, the HLA types of the trio (child, father and mother) predicted in the system of the present invention was more consistent than those predicted by PHLAT.

The HLA types of the HLA-DQA1, -DQB1 and -DRB1 loci predicted with the system of the present invention are consistent with those predicted with PHLAT at six-digit resolution, except for DQA1*01:01:02 (one allele in NA12878 and two alleles in NA12892). PHLAT predicted the HLA allele as "DQA 1*01:01:01", which was predicted as "DQA1*01:01:02" with the system of the present invention. However, its genomic sequence itself was missing in the IMGT database release 3.15.0, so it was not possible to determine the success or failure between the two systems.

(4) Real data analysis (2)

The system of the present invention was applied to the 1KJPN population (1,070 healthy Japanese who participated in the cohort study of Tohoku Medical Megabank Project) and HLA allele at HLA-A, HLA-B and HLA-C loci were estimated. This example is based on mapping of sequence reads to genomic HLA allele sequences registered in the IMGT/HLA database. This analysis confirmed that it is possible to classify HLA-A alleles for 2,063 alleles out of 2,140 alleles at full resolution (eight-digits in HLA nomenclature).

Also, it is confirmed that the allele frequencies of HLA-A, HLA-B, and HLA-C at the predicted four-digit resolution (nucleotide difference that changes the amino acid sequence) are very similar to those determined at 4-digit resolution using the method PCR-SSOP (Itoh, Y. et al., Immunogenetics 57, 717-729 (2005)) in another Japanese population of 1018 people. Since both of the two Japanese populations which were compared include a sufficient number of samples of 1,000 or more, it can be assumed that the allele frequency distribution is close to the actual HLA allele frequency of a Japanese population. The fact that the estimated result with the system of the present invention and the estimated result with PCR-SSOP are very similar suggests that the allele frequency of a Japanese population could be correctly estimated at four-digit resolution whichever method was used.

FIG. 5-1 is a graph showing the results of examining HLA allele frequencies calculated for HLA-A, FIG. 5-2 for HLA-B, and FIG. 5-3 for HLA-C with both methods. The vertical axis shows allele frequency and the horizontal axis shows HLA alleles at four-digit resolution, and a graph bar on the left side of the same HLA allele type is the analysis result estimated using the system of the present invention in this 1KJPN population, and a graph bar on the right shows the analysis result with PCR-SSOP applied to another Japanese population of 1,018 people.

Industrial Applicability

The system of the present invention realizes efficient and accurate genotyping of the gene loci in which there are similar nucleotide sequences in multiple locations in genome or there are many polymorphisms known, such as HLA loci, HLA being a human MHC, cytochrome P450 loci, gene loci encoding an immunoglobulin, gene loci encoding T cell receptors, gene loci encoding olfactory receptors, using whole-genome sequencing data without the need of primer design that is specific to the gene locus or prior knowledge of the allele frequency of the gene loci. Regardless of individual or population scale sequence data, it is possible to easily apply the system of the present invention for genotyping each locus at any selected gene loci, which is useful for studies to identify the relationship between genotype and phenotype and for clinical applications such as HLA type matching of donors and recipients during organ transplantation. In addition, by applying the present invention to other MHC other than HLA, for example, mammalian MHC such as H-2 (histocompatibility-2) which is a mouse MHC, and avian MHC such as chicken B locus, the present invention can be used as providing a more accurate and detailed variety discrimination, and further, providing basic knowledge for establishing therapeutic guidelines for diseases of pets and protected animals.

Explanation of Reference Numerals

B1-1 A box indicating the presence of read data
B1-2 A box indicating the presence of human genome reference
S1 A step of performing the first mapping
B2 A box indicating the presence of the result from the first mapping
S2-1 A step of extracting reads from the result of the first mapping
S2-2 A step of extracting unmapped reads
B3 A box indicating the presence of the read information extracted by S2
B4 A box indicating the presence of HLA allele reference sequence
S3 A step of performing the second mapping
B5 A box indicating the presence of HLA mapping read information
B6 A box indicating the presence of electronic information in which HLA type determination has been made
S4-11 A step of describing a function of retrieval for performing optimization
S4-12 A step of initializing parameters for performing optimization processes
S4-13 A step of describing a function of E step for optimization
S4-14 A step of describing a function of M step for optimization
S4-15 A step of describing a function of loop and convergence for optimization
S4-21 A step of describing a function of retrieval for performing optimization
S4-22 A step of initializing parameters for performing optimization processes
S4-23 A step of describing a function of E step for optimization
S4-24 A step of describing a function of M step for optimization
S4-25 A step of describing a function of loop and convergence for optimization
S4-31 A step of describing a function of retrieval for performing optimization
S4-32 A step of initializing parameters for performing optimization processes
S4-33 A step of describing a function of E step for optimization
S4-34 A step of describing a function of M step for optimization
S4-35 A step of describing a function of loop and convergence for optimization
S5-1 A step of performing calculation of individual depth of coverage
S5-2 A step of performing selection of the two with the largest individual depth of coverage
S5-3 A step of performing selection based on the rejection depth and the largest individual depth of coverage
D5-1 A box indicating a conclusion that the HLA type is not determined
S5-4 A step of performing selection based on the rejection depth and the second largest individual depth of coverage
S5-5 A step performed when the second largest individual depth of coverage is smaller than the rejection depth
D5-2 A box indicating a conclusion that it is homozygous of the HLA allele with the largest individual depth of coverage
D5-3 A box indicating a conclusion that it is heterozygous of the HLA alleles with the largest individual depth of coverage and another allele that is not determined
S5-6 A step performed when the second largest individual depth of coverage is not smaller than the rejection depth
D5-4 A box indicating a conclusion that it is homozygous of the HLA allele with the largest individual depth of coverage
D5-5 A box indicating a conclusion that it is heterozygous of the HLA alleles with the largest and the second largest individual depth of coverage

**Claims**

1. A method for optimizing read information of DNA, in which:

   (I) the following steps (1) to (6) are executed using data in which read information of DNA derived from alleles of selected gene loci or an individual gene locus is mixed, where there are multiple loci having similar nucleotide base sequences in a genome or a large number of genetic polymorphisms exits in the loci:

(1) a step in which, for each read, quantification of an expected number of mappings to each allele of the gene loci or individual gene locus, is performed;

(2) a step in which the expected number of mappings quantified in step (1) is summed for each allele of the gene loci or individual gene locus to calculate a total number of expected mappings;

(3) a step in which the total number of expected mappings calculated in step (2) is divided by the sum of the total number of expected mappings of all the alleles of the gene loci or individual gene locus, to calculate a fraction of reads allocated to each allele of the gene loci or individual gene locus relative to a total read amount mapped to alleles of the gene loci or individual gene locus;

(4) a step in which the fraction of reads obtained in step (3) is assigned to each allele of the gene loci or individual gene locus as frequency, and based on the assigned frequencies, for each read, the expected number of mappings to each allele of the gene loci or individual gene locus is calculated again;

(5) a step in which step (2) or (3) is executed again based on the updated expected number of mappings obtained in step (4), to calculate a fraction of the number of reads allocated to an allele of the gene loci or individual gene locus relative to the total read amount mapped to alleles of the gene loci or individual gene locus; and

(6) a step in which steps (4) and (5) are repeatedly executed, until difference between the expected number of mappings to each allele of the gene loci or individual gene locus calculated in step (4) and that calculated in the previous step (4) is not recognized for all the reads, or difference between the fraction of reads calculated in step (5) and that calculated in the previous step (5), is not recognized for all the alleles in the gene loci or individual gene locus, and the converged expected number of mappings for each read for each allele of the gene loci or individual gene locus, or, the converged fraction of reads for alleles of the gene loci or individual gene locus, is recognized as optimized data,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following steps (a) and (b):

(a) a step in which with respect to nucleotide sequence information of reads in the whole genome obtained from the subject, reads are mapped to a human genome reference sequence, and then those mapped to the gene loci or individual gene locus are extracted; and

(b) a step in which the read sequence information mapped to the gene loci or individual gene locus obtained in step (a) is mapped to the reference nucleotide sequences of alleles of the gene loci or individual gene locus registered in a database, and reads mapped to each allele of the gene loci or individual locus are extracted, and read information is obtained, in which mapping of each read to each allele of the gene loci or individual gene locus is identified; or

(II) mapping of read information of DNA from a subject to alleles of selected gene loci or an individual gene locus is optimized by a computer, where there are multiple loci having similar nucleotide base sequences in a genome or a large number of genetic polymorphisms exits in the loci, which includes a step to determine for each read an expected number of mappings to alleles of the gene loci or individual gene locus, and a step to estimate allele frequencies $\theta$ of the gene loci or individual gene locus, $\theta$ being an objective parameter, where $\theta$ is T dimensional vector, the T being the number of alleles of the gene loci or individual gene locus, given nucleotide sequences of all reads in data in which read information of DNA derived from alleles of the gene loci or individual gene locus is mixed as observed data R, and which is **characterized in that**, with respect to (1) a latent variable $T_n$ dependent on $\theta$ with respect to allele selection of the gene loci or individual gene locus of read n, and (2) a latent variable $S_n$ dependent on $T_n$ with respect to starting position of read n, and given nucleotide sequence of read n as observed data $R_n$, at least (i) variables $T_n$ and $S_n$ or (ii) variable $T_n$ is incorporated to calculate an estimate of the parameter in an estimation process of the objective parameter $\theta$, so that observed data $R_n$ depends on the latent variables during the estimation process of the objective parameter $\theta$ from observed data $R_n$,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following steps (a) and (b):

(a) a step in which with respect to nucleotide sequence information of reads in the whole genome obtained from the subject, reads are mapped to a human genome reference sequence, and then those mapped to the gene loci or individual gene locus are extracted; and

(b) a step in which the read sequence information mapped to the gene loci or individual gene locus obtained in step (a) is mapped to the reference nucleotide sequences of alleles of the gene loci or individual gene locus registered in a database, and reads mapped to each allele of the gene loci or individual locus are extracted, and read information is obtained, in which mapping of each read to each allele of the gene loci or individual gene locus is identified.

2. The optimization method according to claim 1, wherein the read information is single-end read information or paired-end read information.

3. The optimization method according to claim 1 or 2, which is **characterized by** calculating for each read the expected number of mappings to each allele of the selected gene loci or individual gene locus, and calculating estimated value of allele frequency $\theta$ of the gene loci or individual gene locus, $\theta$ being an objective parameter, by executing steps based on a maximum likelihood estimation method, or Bayesian estimation method.

4. The optimization method according to any one of claims 1 to 3, which is **characterized by** using an indicator variable $Z_{nts}$ ($Z_{nts}$ equals to one if $(T_n, S_n) = (t, s)$, and zero otherwise), which summarizes latent variables $T_n$ and $S_n$, or $Z_{nt}$ ($Z_{nt}$ equals to one if $T_n = t$, and zero otherwise), which summarizes the latent variable $T_n$, in variant (II).

5. The optimization method according to claim 4, which is **characterized by** executing the following steps (1) to (5):

(1) a step in which a first update value of a posterior probability of $Z_{nts} = 1$ or $Z_{nt} = 1$ is calculated based on a given initial value of $\theta_t$, and a first updated value of a maximum likelihood estimate of $\theta_t$ is further calculated, based on the first updated value of the posterior probability of $Z_{nts} = 1$ or $Z_{nt} = 1$, or,
(2) a step in which a first updated value of a maximum likelihood estimate of $\theta_t$ is calculated, based on a given initial value of a posterior probability of $Z_{nts} = 1$ or $Z_{nt} = 1$;
(3) a step in which an updated value of the posterior probability of $Z_{nts} = 1$ or $Z_{nt} = 1$ is calculated, based on the updated value of the maximum likelihood estimate of $\theta_t$ calculated by a previous step (4) (however, for the first time, by step (1) or step (2));
(4) a step in which an updated value of the maximum likelihood estimate of $\theta_t$ is calculated, based on the updated value of the posterior probability of $Z_{nts} = 1$ or $Z_{nt} = 1$ calculated in step (3); and
(5)

(i) a step in which log likelihood is calculated based on the updated value of the posterior probability of $Z_{nts} = 1$ or $Z_{nt} = 1$ calculated in step (3) and the updated value of the maximum likelihood estimate of $\theta_t$ calculated in step (4), to evaluate convergence of the log likelihood,
(ii) a step in which convergence of the updated value of the posterior probability of $Z_{nts} = 1$ or $Z_{nt} = 1$ calculated in step (3) is evaluated, or
(iii) a step in which convergence of the updated value of the maximum likelihood estimate of $\theta_t$ calculated in step (4), and,
if convergence is recognized, $\theta_t$ in each step is determined as a final estimate value, and if convergence is not recognized, iteration of steps (3), (4) and (5) is determined.

6. The optimization method according to claim 4, which is **characterized by** executing the following steps (1) to (5):

(1) a step in which a first update posterior distribution of $Z_{nts}$ or $Z_{nt}$ is calculated based on a given initial posterior distribution of $\theta_t$ based on the hyperparameter $\alpha_0$ representing prior information of allele frequencies of the selected gene loci or individual gene locus, and a first updated posterior distribution of $\theta_t$ is further calculated based on the first updated posterior distribution of $Z_{nts}$ or $Z_{nt}$, or, (2) a step in which an updated posterior distribution of $\theta_t$ is calculated based on a given initial distribution of the $Z_{nts}$ or $Z_{nt}$;
(3) a step in which an updated posterior distribution of $Z_{nts}$ or $Z_{nt}$ is calculated, based on the updated posterior distribution of $\theta_t$, calculated by the previous step (4) (however, for the first time, by step (1) or step (2));
(4) a step in which an updated posterior distribution of $\theta_t$ is calculated, based on the updated posterior distribution of $Z_{nts}$ or $Z_{nt}$ calculated in step (3); and
(5) a step in which convergence of the updated posterior distribution of $\theta_t$ calculated in step (4) is evaluated, and if convergence is recognized, the expected value of $\theta_t$ is determined as a final estimate value, and if convergence is not recognized, iteration of steps (3), (4) and (5) is determined.

7. The optimization method according to any one of claims 1 to 6, which is **characterized in that** the mapping performed in steps (a) and (b) allows one read to be mapped to multiple alleles of the selected gene loci or individual gene locus.

8. The optimization method according to claim 7, which is **characterized in that** in addition to the reads mapped to alleles of the selected gene loci or individual gene locus obtained in step (a), reads that are not mapped to whole human genome are extracted, and it becomes the target of the re-mapping in step (b).

9. The optimization method according to any one of claims 1 to 8, which is **characterized in that** the selected gene loci or individual gene locus are MHC gene loci or individual MHC gene locus.

10. The optimization method according to claim 9, which is **characterized in that** MHC is HLA.

11. A genotype determination method for selected gene loci or an individual gene locus, in which:

(I) individual depth of coverage of reads for each allele of the gene loci or individual gene locus is calculated from allele frequency of the selected gene loci or individual gene locus obtained from the optimization method recited in any one of claims 1 to 10, top two or less than two alleles of the gene loci or individual gene locus are selected from a list of alleles that are sorted based on the individual depth of coverage by descending order, and the alleles are determined as candidates for genotyping of each gene locus of the selected gene loci or the individual gene locus; or
(II) individual depth of coverage of reads for each allele of the gene loci or individual gene locus is calculated from allele frequency of the gene loci or individual gene locus obtained from the optimization method recited in any one of claims 1 to 10, top two or less than two alleles of the gene loci or individual gene locus are selected from a list of alleles that are sorted based on the individual depth of coverage by descending order, and the alleles are determined as candidates for genotyping of each gene locus of the selected gene loci or the individual gene locus, and which is **characterized by** setting a threshold of depth of coverage of alleles at 5-50% of the depth of coverage of total reads as a rejection threshold, and eliminating alleles of the gene loci or gene locus whose individual depth of coverage are less than the threshold from candidates for genotyping of each gene locus of the selected gene loci or the individual gene locus.

12. The determination method according to claim 11, which is **characterized in that**, in variant (II), after eliminating the alleles from candidates for genotyping of each gene locus of the selected gene loci or the individual gene locus, the following (i) or (ii) is determined:

(i) for a case that there is one allele that is selected for genotyping of the gene locus of the gene loci or the individual gene locus, if the individual depth of coverage of the allele is twice or more of the rejection threshold, it is determined that the genotype is homozygous of the allele, and if it is less than twice of the rejection threshold, it is determined that the genotype is heterozygous of the allele and another allele is not determined, and
(ii) for the case that there are two alleles that are selected for genotyping of the gene locus of the gene loci or the individual gene locus, if the individual depth of coverage of the one whose individual depth of coverage is larger is less than twice of the smaller one, it is determined that the genotype is heterozygous of the two alleles, or if the individual depth of coverage of the one whose individual depth of coverage is larger is twice or more of the smaller one, it is determined that the genotype is homozygous of the allele whose individual depth of coverage is larger.

13. A computer system:

(I) for optimizing read information of DNA derived from alleles of gene loci or an individual gene locus to be optimized, where there are multiple loci having similar nucleotide base sequences in a genome or a large number of genetic polymorphisms exits in the loci, comprising a recording unit and an arithmetic processing unit, which is **characterized in** the following processes (A) to (G) are executed:

(A) in the recording unit, read information of DNA derived from a subject is recorded as data of nucleotide sequence of read and alleles of the gene loci or individual gene locus to which the read is mapped;
(B) in the arithmetic processing unit, based on the information of the recording section, for each read, an expected number of mappings for each allele of the gene loci or individual gene locus is calculated by executing numerical processing;
(C) for each allele, the expected number of mappings calculated in the process (B) is summed for each allele of the gene loci or individual gene locus to calculate a total number of expected mappings;

(D) for each allele, the total number of expected mappings calculated in the process (C) is divided by the sum of the total number of expected mappings for all alleles of the gene loci or individual gene loci, and the process of calculating the fraction of reads allocated to each allele of the gene loci or individual gene locus with respect to the total amount of reads mapped to alleles of the gene loci or individual gene locus is executed;

(E) the fraction of reads calculated in the above process (C) is assigned as frequency to each allele of the gene loci or individual gene locus, and given the assignment frequency, the process (B) is executed again in which for each read, the expected number of mappings to each allele of the gene loci or individual gene locus is calculated;

(F) the process (C) or (D) is executed again for the new expected number of mappings calculated by the process (E), and the process of calculating the fraction of reads allocated to each allele of the gene loci or individual gene locus with respect to the total amount of reads mapped to alleles of the gene loci or the individual locus is executed; and

(G) the processes (E) and (F) are repeatedly executed, until for all reads, difference between the number of expected mappings to each allele of the gene loci or individual gene locus calculated in the process (E) and that calculated in the previous process (E) is not recognized, or for all alleles of the gene loci or individual gene locus, difference between the fraction of reads calculated in the process (F) and that calculated in the previous process (F) is not recognized, and a converged number of expected mappings for each read to each allele of the gene loci or individual gene locus, or the converged value of the fraction of reads for each allele of the gene loci or individual gene locus, is recognized as the optimized data,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following processes (a) and (b):

(a) a process in which with respect to nucleotide sequence information of reads in the whole genome obtained from the subject, reads are mapped to a human genome reference sequence, and then those mapped to the gene loci or individual gene locus are extracted; and

(b) a process in which the read sequence information mapped to the gene loci or individual gene locus obtained in process (a) is mapped to the reference nucleotide sequences of alleles of the gene loci or individual gene locus registered in a database, and read information is obtained, in which mapping of each read to each allele of the gene loci or individual gene locus and mapping states are identified;

(II) for optimizing, for each read, the number of expected mappings to each allele of selected gene loci or individual gene locus for data in which read information of DNA derived from alleles of the gene loci or individual locus is mixed, where there are multiple loci having similar nucleotide base sequences in a genome or a large number of genetic polymorphisms exits in the loci, comprising a recording unit and an arithmetic processing unit, which is **characterized in that** the following processes (A) to (E) are executed:

(A) in the recording unit, read information of DNA derived from a subject is recorded as observed data of nucleotide sequence of read and alleles of the gene loci or individual gene locus to which the read is mapped;

(B) in the arithmetic processing unit, based on the information of the recording unit, either one of the following initialization processes (B)-1 and (B)-2 is executed:

(B)-1: the process of calculating an initial value of $\theta$, which represents allele frequency of the gene loci or individual gene locus, and

(B)-2: the process of calculating initial values of two latent variables, (i) and (ii) described below, that are related to the $\theta$ described above and the data of DNA read information of the subject, which is the observed data:

(i) a variable $T_n$ that is dependent on $\theta$, which represents allele selection of read n in the gene loci or individual gene locus,

(ii) a posterior probability of an indicator variable $Z_{nts}$ ($Z_{nts}$ equals to one if $(T_n, S_n) = (t, s)$, and zero otherwise), which summarizes $S_n$ and a start position of read n that is dependent on $T_n$, or $Z_{nt}$ ($Z_{nt}$ equals to one if $T_n = t$, and zero otherwise), which summarizes the latent variable $T_n$;

(C) in the arithmetic processing unit, based on the parameter $\theta$ calculated in the process (B)-1, the calculation process of the posterior probability of indicator variable $Z_{nts}$ or $Z_{nt} = 1$ is executed;

(D) in the arithmetic processing unit, a first updated value of the maximum likelihood estimate of parameter $\theta$ is calculated based on the posterior probability of indicator variable $Z_{nts}$ or $Z_{nt} = 1$ calculated in the process (B)-2 or (C); and

(E) in the arithmetic processing unit, the processes (C) and (D) are executed again based on the first updated value of the maximum likelihood estimate of parameter $\theta$ calculated in the process (D), and an iterative loop process for calculating a second updated value of parameter $\theta$ is repeatedly executed, until difference between the new updated value of $\theta$ and the previous updated value of $\theta$ is not recognized, and the converged parameter $\theta$ is recorded as an optimized value in the recording unit,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following processes (a) and (b):

(a) a process in which with respect to nucleotide sequence information of reads in the whole genome obtained from the subject, reads are mapped to a human genome reference sequence, and then those mapped to the gene loci or individual gene locus are extracted; and

(b) a process in which the read sequence information mapped to the gene loci or individual gene locus obtained in process (a) is mapped to the reference nucleotide sequences of alleles of the gene loci or individual gene locus registered in a database, and read information is obtained, in which mapping of each read to each allele of the gene loci or individual gene locus and mapping states are identified;

(III) for optimizing, for each read, the number of expected mappings to each allele of selected gene loci or an individual gene locus for data where read mapping information of the gene loci or individual gene locus is mixed, where there are multiple loci having similar nucleotide base sequences in a genome or a large number of genetic polymorphisms exits in the loci, comprising a recording unit and an arithmetic processing unit, which is **characterized in that** the following processes (A) to (E) are executed:

(A) in the recording unit, read information of DNA derived from the subject is recorded as observed data of nucleotide sequence of read and alleles of the gene loci or individual gene locus to which the read is mapped,

(B) in the arithmetic processing unit, based on the observed data retrieved from the recording unit, either one of the following initialization processes (B)-1 and (B)-2 is executed:

(B)-1: the process of calculating an initial value of posterior distribution of $\theta$, based on an initial value of hyperparameter $\alpha_t$ which represents prior information of allele frequency of the gene loci or individual gene locus, and

(B)-2: the process of calculating initial distribution of two latent variables, (i) and (ii) described below, that are related to the distribution of $\theta$ described above and the data of DNA read information of the subject, which is observed data:

(i) a variable $T_n$ that is dependent on $\theta$, which represents allele selection of read n in the gene loci or individual gene locus, and

(ii) posterior distribution of an indicator variable $Z_{nts}$ ($Z_{nts}$ equals to one if $(T_n, S_n) = (t, s)$, and zero otherwise), which summarizes $S_n$, the start position of read n that is dependent on $T_n$, or $Z_{nt}$ ($Z_{nt}$ equals to one if $T_n = t$, and zero otherwise), which summarizes the latent variable $T_n$;

(C) in the arithmetic processing unit, based on the distribution of parameter $\theta$ calculated in the process (B)-1, the calculation process of the posterior distribution of indicator variable $Z_{nts}$ or $Z_{nt}$ is executed;

(D) in the arithmetic processing unit, the first updated posterior distribution of parameter $\theta$ is calculated based on the posterior distribution of $Z_{nts}$ or $Z_{nt}$ calculated in the process (B)-2 or (C),

(E) in the arithmetic processing unit, the processes (C) and (D) are executed again based on the first updated value of the posterior distribution of $\theta$, and an iterative loop process for calculating a second updated posterior distribution of $\theta$ is repeatedly executed, until difference between the expected value of the new updated posterior distribution of $\theta$ and the expected value of the previous updated posterior distribution of $\theta$ is not recognized, and the converged expected value of the posterior distribution of $\theta$ is recorded as the optimized value in the recording unit,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following processes (a) and (b):

(a) a process in which with respect to nucleotide sequence information of reads in the whole genome obtained from the subject, reads are mapped to a human genome reference sequence, and then those mapped to the gene loci or individual gene locus are extracted; and
(b) a process in which the read sequence information mapped to the gene loci or individual gene locus obtained in process (a) is mapped to the reference nucleotide sequences of alleles of the gene loci or individual gene locus registered in a database, and read information is obtained, in which mapping of each read to each allele of the gene loci or individual gene locus and mapping states are identified; or

(IV) for determining genotype of each gene locus of selected gene loci or an individual gene locus of a subject, where there are multiple loci having similar nucleotide base sequences in a genome or a large number of genetic polymorphisms exits in the loci, comprising a recording unit and an arithmetic processing unit, which is **characterized in that** the following processes ($\alpha$) to ($\delta$) are executed:

($\alpha$) in the recording unit, at least allele frequencies and depth of coverage of total reads of the gene loci or individual gene locus of the subject that are obtained by the optimization method recited in any one of claims 1 to 10 are recorded;
($\beta$) in the arithmetic processing unit, based on the allele frequencies of the gene loci or the individual gene locus recorded in the recording unit, processing of calculating individual depth of coverage of each allele of the gene loci or individual gene locus, and processing of allocating the calculated individual depth of coverage to the alleles of the gene loci or the individual gene locus, are executed;
($\gamma$) given a rejection threshold value, which is set as a frequency number of 5 to 50% of the average depth of coverage of all reads, a process in which alleles of the gene loci or individual gene locus whose individual depth of coverage is smaller than the threshold value are excluded from candidates for genotyping, is executed;
($\delta$) :

($\delta$)-1 after the exclusion process of ($\gamma$), for a case that there is one allele that is selected for genotyping the gene locus of the selected gene loci or the individual gene locus, if the individual depth of coverage of the allele is twice or more of the rejection threshold value, it is determined that the genotype is homozygous of the allele, and if it is less than twice of the rejection threshold value, it is determined that the genotype is heterozygous of the allele and another allele is not determined, and
($\delta$)-2 after the exclusion process of ($\gamma$), for a case that there are two alleles that are selected for genotyping the gene locus of the selected gene loci or the individual gene locus, if the individual depth of coverage of the one whose individual depth of coverage is larger is less than twice of the smaller one, it is determined that the genotype is heterozygous of the two alleles, or if the individual depth of coverage of the one whose individual depth of coverage is larger is twice or more of the smaller one, it is determined that the genotype is homozygous of the allele whose individual depth of coverage is larger.

14. The computer system according to claim 13, wherein, in any one of variants (I) to (III), the read information is single-end read information or paired-end read information.

15. The computer system according to claim 13 or 14, which is **characterized in that**, in any one of variants (I) to (III), the mapping performed in process (b) allows one read to be mapped to multiple alleles of the selected gene loci or individual gene locus.

16. The computer system according to claim 15, which is **characterized in that** in addition to the reads mapped to alleles of the selected gene loci or individual gene locus obtained in process (a), reads that are not mapped to the whole human genome are extracted, and it becomes the target of the re-mapping in process (b).

17. The computer system according to any one of claims 13 to 16, which is **characterized in that** the selected gene loci or individual gene locus is MHC gene loci or individual MHC gene locus.

**18.** The computer system according to claim 17, which is **characterized in that** MHC is HLA.

**19.** A computer program comprising instructions which, when the computer program is executed by a computer, cause the computer to:

(I) optimize read information of DNA derived from alleles of selected gene loci or an individual gene locus, where there are multiple loci having similar nucleotide base sequences in a genome or a large number of genetic polymorphisms exits in the loci, by realizing the following functions (1) to (7):

(A) the function (1) in which read information of DNA derived from a subject recorded in a recording unit as data of nucleotide sequence of read and alleles of the gene loci or individual gene locus to which the read is mapped is retrieved from the recording unit;

(B) the function (2) in which based on the read information retrieved in the function (1), for each read, an expected number of mappings for each allele of the gene loci or individual gene locus is calculated by executing numerical processing;

(C) the function (3) in which for each allele, the expected number of mappings quantified in the function (2) is summed for each allele of the gene loci or individual gene locus to calculate the total number of expected mappings;

(D) the function (4) in which for each allele, the total number of expected mappings calculated in the function (3) is divided by the sum of the total number of expected mappings for all alleles of the gene loci or individual gene loci, and the process of calculating the fraction of reads allocated to each allele of the gene loci or individual gene locus with respect to the total amount of reads mapped to alleles of the gene loci or individual gene locus is executed;

(E) the function (5) in which the fraction of reads calculated in the function (4) is assigned as frequency to each allele of the gene loci or individual gene locus, and given the assignment frequency, the function (2) is executed again in which for each read, the expected number of mappings to each allele of the gene loci or individual gene locus is calculated;

(F) the function (6) in which the function (3) or (4) is executed again for the newly obtained expected number of mappings calculated by the function (5), and the process of calculating the fraction of reads allocated to each allele of the gene loci or individual gene locus with respect to the total amount of reads mapped to alleles of the gene loci or the individual locus is executed; and

(G) the function (7) in which the functions (5) and (6) are repeatedly executed, until for all reads, difference between the number of expected mappings to each allele of the gene loci or individual gene locus calculated in the function (5) and that calculated in the previous function (5), is not recognized, or for all alleles of the gene loci or individual gene locus, difference between the value of the fraction of reads calculated in the function (6) and that calculated in the previous function (6), is not recognized, and the converged number of expected mappings for each read to each allele of the gene loci or individual gene locus, or the converged value of the fraction of reads for each allele of the gene loci or individual gene locus, is recognized as an optimized data,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following processes (a) and (b):

(a) a process in which with respect to nucleotide sequence information of reads in the whole genome obtained from the subject, reads are mapped to a human genome reference sequence, and then those mapped to the gene loci or individual gene locus are extracted; and

(b) a process in which the read sequence information mapped to the gene loci or individual gene locus obtained in process (a) is mapped to the reference nucleotide sequences of alleles of the gene loci or individual gene locus registered in a database, and read information is obtained, in which mapping of each read to each allele of the gene loci or individual gene locus and mapping states are identified;

(II) to optimize, for each read, the number of expected mappings to each allele of selected gene loci or an individual gene locus for data in which read information of DNA derived from alleles of the gene loci or gene locus is mixed, where there are multiple loci having similar nucleotide base sequences in a genome or a large number of genetic polymorphisms exits in the loci, by realizing the following functions 1 to 5:

(A) function 1 in which read information of DNA derived from a subject recorded in a recording unit as observed data of nucleotide sequence of read and alleles of the gene loci or individual gene locus to which the read is mapped, is retrieved from the recording unit;

(B) function 2 in which based on the observed data retrieved by function 1, either one of the following initialization processes (B)-1 and (B)-2 is executed:

(B)-1: the process of calculating an initial value of $\theta$, which represents allele frequency of the gene loci or individual gene locus, and

(B)-2: the process of calculating initial values of two latent variables, (i) and (ii) described below, that are related to the $\theta$ described above and the data of DNA read information of the subject, which is the observed data:

(i) a variable $T_n$ that is dependent on $\theta$, which represents allele selection of read n in the gene loci or individual gene locus, and

(ii) posterior probability of an indicator variable $Z_{nts}$ ($Z_{nts}$ equals to one if $(T_n, S_n) = (t, s)$, and zero otherwise), which summarizes $S_n$ which is start position of read n that is dependent on $T_n$, or $Z_{nt}$ ($Z_{nt}$ equals to one if $T_n = t$, and zero otherwise), which summarizes a latent variable $T_n$;

(C) function 3 in which based on the parameter $\theta$ calculated in the process (B)-1, the calculation process of the posterior probability of indicator variable $Z_{nts}$ or $Z_{nt} = 1$ is executed;

(D) function 4 in which a first updated value of maximum likelihood estimate of parameter $\theta$ is calculated based on the posterior probability of $Z_{nts}$ or $Z_{nt} = 1$ calculated in process (B)-2 or function 3; and

(E) function 5 in which function 3 and 4 are executed again based on the first updated value of the maximum likelihood estimate of $\theta$, and the iterative loop process for calculating a second updated value of $\theta$, is repeatedly executed, until difference between the new updated value of $\theta$ and the previous updated value of $\theta$ is not recognized, and the converged parameter $\theta$ is recorded as an optimized value in the recording unit, wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following processes (a) and (b):

(a) a process in which with respect to nucleotide sequence information of reads in the whole genome obtained from the subject, reads are mapped to a human genome reference sequence, and then those mapped to the gene loci or individual gene locus are extracted; and

(b) a process in which the read sequence information mapped to the gene loci or individual gene locus obtained in process (a) is mapped to the reference nucleotide sequences of alleles of the gene loci or individual gene locus registered in a database, and read information is obtained, in which mapping of each read to each allele of the gene loci or individual gene locus and mapping states are identified;

(III) to optimize, for each read, the number of expected mappings to each allele of selected gene loci or an individual gene locus for data in which read information of DNA derived from alleles of the gene loci or gene locus is mixed, where there are multiple loci having similar nucleotide base sequences in a genome or a large number of genetic polymorphisms exits in the loci, by realizing the following functions 1 to 5:

(A) function 1 in which read information of DNA derived from a subject recorded in a recording unit as observed data of nucleotide sequence of read and alleles of the gene loci or individual gene locus to which the read is mapped, is retrieved from the recording unit;

(B) function 2 in which based on the observed data retrieved by function 1, either one of the following initialization processes (B)-1 and (B)-2 is executed:

(B)-1: a process of calculating an updated value of posterior distribution of $\theta$, based on an initial value of hyperparameter $\alpha_t$ which represents prior information of allele frequency of the gene loci or individual gene locus, and

(B)-2: a process of calculating initial distributions of two latent variables, (i) and (ii) described below, that are related to the distribution of $\theta$ described above and the data of DNA read information of the subject, which is observed data:

(i) a variable $T_n$ that is dependent on $\theta$, which represents allele selection of read n in the gene loci or individual gene locus,

(ii) posterior distribution of indicator variable $Z_{nts}$ ($Z_{nts}$ equals to one if $(T_n, S_n) = (t, s)$, and zero otherwise), which summarizes $S_n$ which is start position of read n that is dependent on $T_n$, or $Z_{nt}$ ($Z_{nt}$ equals to one if $T_n = t$, and zero otherwise), which summarizes latent variable $T_n$;

(C) function 3 in which based on the distribution $\theta$ calculated in the process (B)-1, the calculation process of the posterior distribution of indicator variable $Z_{nts}$ or $Z_{nt}$ is executed;

(D) function 4 in which a first updated value of the posterior distribution of $\theta$ is calculated based on the posterior distribution of $Z_{nts}$ or $Z_{nt}$ calculated in function (B)-2 or function 3; and

(E) function 5 in which function 3 and 4 are executed again based on the first updated value of the posterior distribution of $\theta$, and an iterative loop process for calculating a second updated value of the posterior distribution of $\theta$ is repeatedly executed, until difference between an expected value of the new updated posterior distribution of $\theta$ and an expected value of the previous updated posterior distribution of $\theta$ is not recognized, and the converged expected value of posterior distribution of $\theta$ is recorded as an optimized value in the recording unit,

wherein the data in which read information of DNA derived from alleles of the selected gene loci or individual gene locus is mixed, is read information in which mapping of each read to each allele of the gene loci or individual gene locus is identified by mapping read information in a whole genome of a subject to reference nucleotide sequences of alleles of the gene loci or individual gene locus registered in a database, and the mapping is **characterized by** executing the following processes (a) and (b):

(a) a process in which with respect to nucleotide sequence information of reads in the whole genome obtained from the subject, reads are mapped to a human genome reference sequence, and then those mapped to the gene loci or individual gene locus are extracted; and

(b) a process in which the read sequence information mapped to the gene loci or individual gene locus obtained in process (a) is mapped to the reference nucleotide sequences of alleles of the gene loci or individual gene locus registered in a database, and read information is obtained, in which mapping of each read to each allele of the gene loci or individual gene locus and mapping states are identified; or

(IV) to determine genotype of a gene locus of selected gene loci or an individual gene locus of a subject, where there are multiple loci having similar nucleotide base sequences in a genome or a large number of genetic polymorphisms exits in the loci, by realizing the following functions ($\alpha$) to ($\delta$):

($\alpha$) a function $\alpha$, in which at least allele frequencies and depth of coverage of total reads of the gene loci or individual gene locus of the subject that are obtained by executing the computer program recited in any one of variants (I) to (III), are retrieved;

($\beta$) a function $\beta$, in which based on the allele frequencies of the gene loci or the individual gene locus retrieved by executing function $\alpha$, calculation of individual depth of coverage of each allele of the gene loci or individual gene locus, and allocation of the calculated individual depth of coverage to the alleles of the gene loci or the individual gene locus, are executed;

($\gamma$) a function $\gamma$, in which given a rejection threshold value, which is set as a frequency number of 5 to 50% of average depth of coverage of all reads, a process in which alleles of the gene loci or individual gene locus whose individual depth of coverage specified by the function $\beta$ is smaller than the threshold value are excluded from candidates for genotyping, is executed; and

($\delta$): a function $\delta$ specified as ($\delta$)-1 and ($\delta$)-2 below:

($\delta$)-1 after the exclusion process in function($\gamma$), for a case that there is one allele that is selected for genotyping the gene locus of the selected gene loci or the individual gene locus, if the individual depth of coverage of the allele is twice or more of the rejection threshold value, a process in which it is determined that the genotype is homozygous of the allele, and if it is less than twice of the rejection threshold value, it is determined that the genotype is heterozygous of the allele and another allele is not determined, and

($\delta$)-2 after the exclusion process in function($\gamma$), for a case that there are two alleles that are selected for genotyping the gene locus of the selected gene loci or the individual gene locus, if the individual depth of coverage of the one whose individual depth of coverage is larger is less than twice of the smaller one, a process in which it is determined that the genotype is heterozygous of the two alleles, or if the individual depth of coverage of the one whose individual depth of coverage is larger is twice or more of the smaller one, it is determined that the genotype is homozygous of the allele whose individual depth of coverage is larger, is executed.

20. The computer program according to claim 19, wherein, in any one of variants (I) to (III), the read information is single-end read information or paired-end read information.

21. The computer program according to claim 19 or 20, which is **characterized in that**, in any one of variants (I) to (III), generation of the data in which read information of DNA derived from alleles of the selected gene loci or individual gene locus is mixed, is realizing by executing the following functions (a) and (b):

(a) a function in which with respect to nucleotide sequence information of reads obtained from the subject, reads are mapped to human genome nucleotide sequence, and then those mapped to alleles of the gene loci or individual gene locus are extracted; and
(b) a function in which with respect to the read sequence information mapped to alleles of the gene loci or individual gene locus obtained in function (a), reads are mapped to reference nucleotide sequences of alleles of the gene loci or individual gene locus that are registered in a database, and reads mapped to each allele of the gene loci or individual locus are extracted, and read information is obtained, in which mapping of each read to each allele of the gene loci or individual gene locus is identified.

22. The computer program according to any one of claims 19 to 21, which is **characterized in that**, in any one of variants (I) to (III), the mapping performed in function (b) allows one read to be mapped to multiple alleles of the selected gene loci or individual gene locus.

23. The computer program according to claim 22, which is **characterized in that** in addition to the reads mapped to alleles of the selected gene loci or individual gene locus obtained in function (a), reads that are not mapped to the whole human genome are extracted, and it becomes the target of the re-mapping in function (b).

24. The computer program according to any one of claims 19 to 23, which is **characterized in that** the selected gene loci or individual gene locus is MHC gene loci or individual MHC gene locus.

25. The computer program according to claim 24, which is **characterized in that** MHC is HLA.

26. A computer-readable recording medium, wherein the computer program recited in any one of claims 19 to 25 is recorded.

**Patentansprüche**

1. Verfahren zur Optimierung der Ableseinformationen von DNA, in welchem:

(I) die nachfolgenden Schritte (1) bis (6) unter Verwendung von Daten durchgeführt werden, in welchen die Ableseinformationen einer aus Allelen ausgewählter Genloci oder eines einzelnen Genlocus erhaltenen DNA vermischt sind, wobei mehrere Loci mit ähnlichen Nukleotidbasensequenzen in einem Genom vorliegen oder eine große Anzahl von genetischen Polymorphismen in den Loci existiert:

(1) ein Schritt, in welchem für jede Ablesung eine Quantifizierung einer erwarteten Anzahl von Alignments mit jedem Allel der Genloci oder des einzelnen Genlocus durchgeführt wird;
(2) ein Schritt, in welchem die in Schritt (1) quantifizierte erwartete Anzahl von Alignments für jedes Allel der Genloci oder des einzelnen Genlocus addiert wird, um auf diese Weise eine Gesamtzahl von erwarteten Alignments zu berechnen;
(3) ein Schritt, in welchem die in Schritt (2) berechnete Gesamtzahl von erwarteten Alignments durch die Summe der Gesamtzahl von erwarteten Alignments für sämtliche Allele der Genloci oder des einzelnen Genlocus geteilt wird, um auf diese Weise einen Anteil an Ablesungen, welcher jedem Allel der Genloci oder des einzelnen Genlocus zugewiesen wird, in Bezug auf eine mit Allelen der Genloci oder des einzelnen Genlocus abgeglichene Gesamtablesemenge zu berechnen;
(4) ein Schritt, in welchem der in Schritt (3) erhaltene Anteil an Ablesungen jedem Allel der Genloci oder des einzelnen Genlocus als Frequenz zugeordnet wird, und auf Grundlage der zugeordneten Frequenzen für jede Ablesung die erwartete Anzahl von Alignments mit jedem Allel der Genloci oder des einzelnen Genlocus erneut berechnet wird;

(5) ein Schritt, in welchem auf Grundlage der in Schritt (4) erhaltenen aktualisierten erwarteten Anzahl von Alignments Schritt (2) oder (3) erneut ausgeführt wird, um auf diese Weise einen Anteil der Anzahl von Ablesungen, die einem Allel der Genloci oder des einzelnen Genlocus zugewiesen sind, in Bezug auf eine mit Allelen der Genloci oder des einzelnen Genlocus abgeglichene Gesamtablesemenge zu berechnen; und

(6) ein Schritt, in welchem die Schritte (4) und (5) wiederholt ausgeführt werden, bis zwischen der in Schritt (4) berechneten erwarteten Anzahl von Alignments mit jedem Allel der Genloci oder des einzelnen Genlocus und der im vorherigen Schritt (4) berechneten Anzahl für sämtliche Ablesungen kein Unterschied mehr festgestellt wird, oder zwischen dem in Schritt (5) berechneten Anteil an Ablesungen und dem im vorherigen Schritt (5) berechneten Anteil für sämtliche Allele der Genloci oder des einzelnen Genlocus kein Unterschied mehr festgestellt wird, und die konvergierte erwartete Anzahl von Alignments für jede Ablesung für jedes Allel der Genloci oder des einzelnen Genlocus, oder der konvergierte Anteil an Ablesungen für Allele der Genloci oder des einzelnen Genlocus, als optimierte Daten anerkannt wird,

wobei es sich bei den Daten, in welchen die Ableseinformationen der aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Schritte (a) und (b) gekennzeichnet ist:

(a) ein Schritt, in welchem, was Nukleotidsequenzinformationen aus Ablesungen in dem aus dem Subjekt erhaltenen Gesamtgenom betrifft, die Ablesungen mit einer menschlichen Referenzgenomsequenz abgeglichen werden, und anschließend jene, die mit den Genloci oder dem einzelnen Genlocus abgeglichen worden sind, extrahiert werden; und

(b) ein Schritt, in welchem die in Schritt (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und mit jedem Allel der Genloci oder des einzelnen Locus abgeglichene Ablesungen extrahiert werden, und Ableseinformationen erhalten werden, in welchen das Alignment einer jeden Ablesung mit jedem Allel der Genloci oder des einzelnen Genlocus erfasst ist; oder

(II) ein Alignment der Ableseinformationen von DNA aus einem Subjekt mit Allelen ausgewählter Genloci oder eines einzelnen Genlocus mittels eines Computers optimiert wird, wobei mehrere Loci mit ähnlichen Nukleotidbasensequenzen in einem Genom vorliegen oder eine große Anzahl von genetischen Polymorphismen in den Loci existiert, umfassend einen Schritt zur Bestimmung einer erwarteten Anzahl von Alignments mit Allelen der Genloci oder des einzelnen Genlocus für jede Ablesung, und einen Schritt zur Abschätzung von Allelfrequenzen $\theta$ der Genloci oder des einzelnen Genlocus, wobei $\theta$ ein objektiver Parameter ist, $\theta$ ein T-dimensionaler Vektor ist, T die Anzahl von Allelen der Genloci oder des einzelnen Genlocus darstellt, und vorgegebene Nukleotidsequenzen sämtlicher Ablesungen in Daten, in welchen Ableseinformationen einer aus Allelen der Genloci oder des einzelnen Genlocus erhaltenen DNA vermischt sind, als Beobachtungsdaten R definiert sind, und welches **dadurch gekennzeichnet ist, dass**, in Bezug auf (1) eine von $\theta$ abhängige latente Variable $T_n$ betreffend die Allelauswahl der Genloci oder des einzelnen Genlocus im Rahmen der Ablesung n, und (2) eine von $T_n$ abhängige latente Variable $S_n$ betreffend die Anfangsposition der Ablesung n, und eine vorgegebene Nukleotidsequenz der Ablesung n als Beobachtungsdaten $R_n$, zumindest (i) die Variablen $T_n$ und $S_n$ oder (ii) die Variable $T_n$ zur Berechnung einer Schätzung des Parameters in einem Verfahren zur Schätzung des objektiven Parameters $\theta$ miteinbezogen wird/werden, so dass die Beobachtungsdaten $R_n$ während des Verfahrens zur Schätzung des objektiven Parameters $\theta$ aus den Beobachtungsdaten $R_n$ von den latenten Variablen abhängen,

wobei es sich bei den Daten, in welchen Ableseinformationen von der aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Schritte (a) und (b) gekennzeichnet ist:

(a) ein Schritt, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, die mit den Genloci oder dem einzelnen Genlocus abgeglichen worden sind, extrahiert werden; und

(b) ein Schritt, in welchem die in Schritt (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, mit jedem Allel der Genloci oder des einzelnen Locus abgeglichene Ablesungen extrahiert werden, und Ableseinformationen erhalten werden, in welchen das Alignment einer jeden Ablesung mit jedem Allel der Genloci oder des einzelnen Genlocus erfasst ist.

2. Optimierungsverfahren gemäß Anspruch 1, wobei es sich bei den Ableseinformationen um "Single End"-Ableseinformationen oder um "Paired End"-Ableseinformationen handelt.

3. Optimierungsverfahren gemäß Anspruch 1 oder 2, welches **dadurch gekennzeichnet ist, dass** es bei jeder Ablesung die erwartete Anzahl von Alignments mit jedem Allel der ausgewählten Genloci oder des einzelnen Genlocus berechnet, und auf Grundlage eines Maximum-Likelihood-Schätzverfahrens oder eines Bayes'schen Schätzverfahrens den Schätzwert der Allelfrequenz $\theta$ der Genloci oder des einzelnen Genlocus durch Ausführen von Schritten berechnet, wobei $\theta$ ein objektiver Parameter ist.

4. Optimierungsverfahren gemäß einem der Ansprüche 1 bis 3, welches **dadurch gekennzeichnet ist, dass** es eine Indikatorvariable $Z_{nts}$ ($Z_{nts}$ ist eins, wenn $(T_n, S_n) = (t, s)$, und ist anderweitig null), welche die latenten Variablen $T_n$ und $S_n$ zusammenfasst, oder $Z_{nt}$ ($Z_{nt}$ ist eins, wenn $T_n = t$, und ist anderweitig null), welche in Variante (II) die latente Variable $T_n$ zusammenfasst, nutzt.

5. Optimierungsverfahren gemäß Anspruch 4, welches **dadurch gekennzeichnet ist, dass** es die nachfolgenden Schritte (1) bis (5) ausführt:

(1) ein Schritt, in welchem auf Grundlage eines vorgegebenen Anfangswerts für $\theta_t$ ein erster aktualisierter Wert für eine A-posteriori-Wahrscheinlichkeit von $Z_{nts} = 1$ oder $Z_{nt} = 1$ berechnet wird, und weiterhin auf Grundlage des ersten aktualisierten Werts für die A-posteriori-Wahrscheinlichkeit von $Z_{nts} = 1$ oder $Z_{nt} = 1$ ein erster aktualisierter Wert für eine Maximum-Likelihood-Schätzung von $\theta_t$ berechnet wird, oder,

(2) ein Schritt, in welchem auf Grundlage eines vorgegebenen Anfangswerts für eine A-posteriori-Wahrscheinlichkeit von $Z_{nts} = 1$ oder $Z_{nt} = 1$ ein erster aktualisierter Wert für eine Maximum-Likelihood-Schätzung von $\theta_t$ berechnet wird;

(3) ein Schritt, in welchem auf Grundlage des mittels eines vorherigen Schritts (4) (allerdings erstmals mittels Schritt (1) oder Schritt (2)) berechneten aktualisierten Werts für die Maximum-Likelihood-Schätzung von $\theta_t$ ein aktualisierter Wert für die A-posteriori-Wahrscheinlichkeit von $Z_{nts} = 1$ oder $Z_{nt} = 1$ berechnet wird;

(4) ein Schritt, in welchem auf Grundlage des in Schritt (3) berechneten aktualisierten Werts für die A-posteriori-Wahrscheinlichkeit von $Z_{nts} = 1$ oder $Z_{nt} = 1$ ein aktualisierter Wert für die Maximum-Likelihood-Schätzung von $\theta_t$ berechnet wird; und

(5)

(i) ein Schritt, in welchem auf Grundlage des in Schritt (3) berechneten aktualisierten Werts für die A-posteriori-Wahrscheinlichkeit von $Z_{nts} = 1$ oder $Z_{nt} = 1$ und dem in Schritt (4) berechneten aktualisierten Wert für die Maximum-Likelihood-Schätzung von $\theta_t$ die log-Wahrscheinlichkeit berechnet wird, um auf diese Weise die Konvergenz der log-Wahrscheinlichkeit zu evaluieren,

(ii) ein Schritt, in welchem die Konvergenz des in Schritt (3) berechneten aktualisierten Werts für die A-posteriori-Wahrscheinlichkeit von $Z_{nts} = 1$ oder $Z_{nt} = 1$ evaluiert wird, oder

(iii) ein Schritt, in welchem die Konvergenz des in Schritt (4) berechneten aktualisierten Werts für die Maximum-Likelihood-Schätzung von $\theta_t$ evaluiert wird, und,

wenn eine Konvergenz festgestellt wird, $\theta_t$ in jedem Schritt als finaler Schätzwert festgelegt wird, und, wenn keine Konvergenz festgestellt wird, eine Wiederholung der Schritte (3), (4) und (5) veranlasst wird.

6. Optimierungsverfahren gemäß Anspruch 4, welches **dadurch gekennzeichnet ist, dass** es die nachfolgenden Schritte (1) bis (5) ausführt:

(1) ein Schritt, in welchem auf Grundlage einer vorgegebenen A-posteriori-Anfangsverteilung von $\theta_t$ mittels des Hyperparameters $\alpha_0$, der frühere Informationen bezüglich von Allelfrequenzen der ausgewählten Genloci oder des einzelnen Genlocus repräsentiert, eine erste aktualisierte A-posteriori-Verteilung von $Z_{nts}$ oder $Z_{nt}$ berechnet wird, und weiterhin auf Grundlage der ersten aktualisierten A-posteriori-Verteilung von $Z_{nts}$ oder $Z_{nt}$ eine erste

aktualisierte A-posteriori-Verteilung von $\theta_t$ berechnet wird, oder,

(2) ein Schritt, in welchem auf Grundlage einer vorgegebenen Anfangsverteilung von $Z_{nts}$ oder $Z_{nt}$ eine aktualisierte A-posteriori-Verteilung von $\theta_t$ berechnet wird;

(3) ein Schritt, in welchem auf Grundlage der mittels des vorherigen Schritts (4) (allerdings erstmals mittels Schritt (1) oder Schritt (2)) berechneten aktualisierten A-posteriori-Verteilung von $\theta_t$ eine aktualisierte A-posteriori-Verteilung von $Z_{nts}$ oder $Z_{nt}$ berechnet wird;

(4) ein Schritt, in welchem auf Grundlage der in Schritt (3) berechneten aktualisierten A-posteriori-Verteilung von $Z_{nts}$ oder $Z_{nt}$ eine aktualisierte A-posteriori-Verteilung von $\theta_t$ berechnet wird; und

(5) ein Schritt, in welchem die Konvergenz der in Schritt (4) berechneten aktualisierten A-posteriori-Verteilung von $\theta_t$ evaluiert wird, und, wenn eine Konvergenz festgestellt wird, der erwartete Wert für $\theta_t$ als finaler Schätzwert festgelegt wird, und, wenn keine Konvergenz festgestellt wird, eine Wiederholung der Schritte (3), (4) und (5) veranlasst wird.

**7.** Optimierungsverfahren gemäß einem der Ansprüche 1 bis 6, welches **dadurch gekennzeichnet ist, dass** das in den Schritten (a) und (b) durchgeführte Alignment die Möglichkeit bietet, eine Ablesung mit mehreren Allelen der ausgewählten Genloci oder des einzelnen Genlocus abzugleichen.

**8.** Optimierungsverfahren gemäß Anspruch 7, welches **dadurch gekennzeichnet ist, dass** zusätzlich zu den mit Allelen der ausgewählten Genloci oder des einzelnen Genlocus abgeglichenen und in Schritt (a) erhaltenen Ablesungen Ablesungen extrahiert werden, welche nicht mit dem menschlichen Gesamtgenom abgeglichen worden sind, und dies das Ziel des erneuten Alignments in Schritt (b) darstellt.

**9.** Optimierungsverfahren gemäß einem der Ansprüche 1 bis 8, welches **dadurch gekennzeichnet ist, dass** es sich bei den ausgewählten Genloci oder dem einzelnen Genlocus um MHC-Genloci oder einen einzelnen MHC-Genlocus handelt.

**10.** Optimierungsverfahren gemäß Anspruch 9, welches **dadurch gekennzeichnet ist, dass** es sich bei MHC um HLA handelt.

**11.** Verfahren zur Bestimmung des Genotyps für ausgewählte Genloci oder einen einzelnen Genlocus, in welchem:

(I) auf Grundlage der Allelfrequenz der ausgewählten Genloci oder des einzelnen Genlocus, welche mittels des in einem der Ansprüche 1 bis 10 erwähnten Optimierungsverfahrens erhalten worden ist, die individuelle Sequenziertiefe von Ablesungen für jedes Allel der Genloci oder des einzelnen Genlocus berechnet wird, die obersten zwei oder weniger als zwei Allele der Genloci oder des einzelnen Genlocus aus einer Liste von Allelen ausgewählt werden, welche auf Grundlage der individuellen Sequenziertiefe in absteigender Reihenfolge angeordnet sind, und die Allele als Kandidaten für eine Genotypisierung eines jeden Genlocus der ausgewählten Genloci oder des einzelnen Genlocus festgelegt werden; oder

(II) auf Grundlage der Allelfrequenz der Genloci oder des einzelnen Genlocus, welche mittels des in einem der Ansprüche 1 bis 10 erwähnten Optimierungsverfahrens erhalten worden ist, die individuelle Sequenziertiefe von Ablesungen für jedes Allel der Genloci oder des einzelnen Genlocus berechnet wird, die obersten zwei oder weniger als zwei Allele der Genloci oder des einzelnen Genlocus aus einer Liste von Allelen ausgewählt werden, welche auf Grundlage der individuellen Sequenziertiefe in absteigender Reihenfolge angeordnet sind, und die Allele als Kandidaten für eine Genotypisierung eines jeden Genlocus der ausgewählten Genloci oder des einzelnen Genlocus festgelegt werden, und welches **dadurch gekennzeichnet ist, dass** ein Grenzwert der Sequenziertiefe von Allelen auf 5-50% der Sequenziertiefe der Gesamtzahl von Ablesungen als Ablehnungsgrenzwert festgelegt wird, und Allele der Genloci oder des Genlocus, deren individuelle Sequenziertiefe geringer ist als der Grenzwert von Kandidaten für die Genotypisierung eines jeden Genlocus der ausgewählten Genloci oder des einzelnen Genlocus, eliminiert werden.

**12.** Bestimmungsverfahren gemäß Anspruch 11, welches **dadurch gekennzeichnet ist, dass** in Variante (II) nach Eliminierung der Allele aus den Kandidaten für eine Genotypisierung eines jeden Genlocus der ausgewählten Genloci oder des einzelnen Genlocus die nachfolgenden Punkte (i) oder (ii) festgestellt werden:

(i) im Falle des Vorliegens eines für die Genotypisierung des Genlocus der Genloci oder des einzelnen Genlocus ausgewählten Allels wird, wenn die individuelle Sequenziertiefe des Allels das Doppelte oder mehr als das Doppelte des Ablehnungsgrenzwerts beträgt, festgelegt, dass der Genotyp bezüglich des Allels homozygot ist, und wird, wenn sie weniger als das Doppelte des Ablehnungsgrenzwerts beträgt, festgelegt, dass der Genotyp bezüglich des Allels heterozygot ist und kein weiteres Allel bestimmt wird, und

(ii) im Falle des Vorliegens zweier für die Genotypisierung des Genlocus der Genloci oder des einzelnen Genlocus ausgewählter Allele wird, wenn die individuelle Sequenziertiefe jenes Allels, dessen individuelle Sequenziertiefe größer ist, weniger als das Doppelte des kleineren beträgt, festgelegt, dass der Genotyp bezüglich der beiden Allele heterozygot ist, oder wird, wenn die individuelle Sequenziertiefe jenes Allels, dessen individuelle Sequenziertiefe größer ist, das Doppelte oder mehr als das Doppelte des kleineren beträgt, festgelegt, dass der Genotyp bezüglich des Allels, dessen individuelle Sequenziertiefe größer ist, homozygot ist.

**13.** Computersystem:

(I) zur Optimierung der Ableseinformationen von DNA, welche aus zu optimierenden Allelen von Genloci oder einem einzelnen Genlocus erhalten worden sind, wobei mehrere Loci mit ähnlichen Nukleotidbasensequenzen in einem Genom vorliegen oder eine große Anzahl von genetischen Polymorphismen in den Loci existiert, wobei das Computersystem eine Aufzeichnungseinheit und eine arithmetische Verarbeitungseinheit umfasst und **dadurch gekennzeichnet ist, dass** die nachfolgenden Prozesse (A) bis (G) ausgeführt werden:

(A) in der Aufzeichnungseinheit werden Ableseinformationen von einer aus einem Subjekt erhaltenen DNA als Daten bestehend aus der Nukleotidsequenz einer Ablesung sowie Allelen der Genloci oder des einzelnen Genlocus, mit welchen die Ablesung abgeglichen worden ist, aufgezeichnet;

(B) in der arithmetischen Verarbeitungseinheit wird auf Grundlage der Informationen des Aufzeichnungsabschnitts für jede Ablesung eine erwartete Anzahl von Alignments für jedes Allel der Genloci oder des einzelnen Genlocus durch Ausführen einer numerischen Verarbeitung berechnet;

(C) für jedes Allel wird die in Prozess (B) berechnete erwartete Anzahl von Alignments für jedes Allel der Genloci oder des einzelnen Gelocus addiert, um auf diese Weise eine Gesamtzahl von erwarteten Alignments zu berechnen;

(D) für jedes Allel wird die in Prozess (C) berechnete Gesamtzahl von erwarteten Alignments durch die Summe der Gesamtzahl von erwarteten Alignments für sämtliche Allele der Genloci oder des einzelnen Genlocus geteilt, und es wird der Prozess einer Berechnung des Anteils an Ablesungen, welcher jedem Allel der Genloci oder des einzelnen Genlocus zugewiesen wird, in Bezug auf die mit Allelen der Genloci oder des einzelnen Genlocus abgeglichene Gesamtzahl von Ablesungen ausgeführt;

(E) der im vorstehenden Prozess (C) berechnete Anteil an Ablesungen wird jedem Allel der Genloci oder des einzelnen Genlocus als Frequenz zugeordnet, und es wird der Prozess (B), in welchem für jede Ablesung die erwartete Anzahl von Alignments mit jedem Allel der Genloci oder des einzelnen Genlocus berechnet wird, bei vorgegebener Zuordnungsfrequenz erneut ausgeführt;

(F) der Prozess (C) oder (D) wird für die in Prozess (E) berechnete neue erwartete Anzahl von Alignments erneut ausgeführt, und es wird der Prozess einer Berechnung des Anteils an Ablesungen, welcher jedem Allel der Genloci oder des einzelnen Genlocus zugewiesen wird, in Bezug auf die mit Allelen der Genloci oder des einzelnen Genlocus abgeglichene Gesamtzahl von Ablesungen ausgeführt; und

(G) die Prozesse (E) und (F) werden wiederholt ausgeführt, bis zwischen der in Prozess (E) berechneten Anzahl von erwarteten Alignments mit jedem Allel der Genloci oder des einzelnen Genlocus und der im vorherigen Prozess (E) berechneten Anzahl für sämtliche Ablesungen kein Unterschied mehr festgestellt wird, oder zwischen dem in Prozess (F) berechneten Anteil an Ablesungen und dem im vorherigen Prozess (F) berechneten Anteil für sämtliche Allele der Genloci oder des einzelnen Genlocus kein Unterschied mehr festgestellt wird, und eine konvergierte Anzahl von erwarteten Alignments für jede Ablesung mit jedem Allel der Genloci oder des einzelnen Genlocus, oder der konvergierte Wert für den Anteil an Ablesungen für jedes Allel der Genloci oder des einzelnen Genlocus, als optimierte Daten anerkannt wird, wobei es sich bei den Daten, in welchen Ableseinformationen von der aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Prozesse (a) und (b) gekennzeichnet ist:

(a) ein Prozess, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, die mit den Genloci oder dem einzelnen Genlocus abgeglichen worden sind, extrahiert werden; und

(b) ein Prozess, in welchem die in Prozess (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und Ableseinformationen erhalten werden, in welchen das Alignment einer jeden Ablesung mit jedem Allel der Genloci oder des einzelnen Genlocus sowie Alignmentzustände erfasst sind;

(II) im Rahmen jeder Ablesung zur Optimierung der Anzahl von erwarteten Alignments mit jedem Allel ausgewählter Genloci oder eines einzelnen Genlocus in Bezug auf Daten, in welchen Ableseinformationen von einer aus Allelen der Genloci oder des einzelnen Genlocus erhaltenen DNA vermischt sind, wobei mehrere Loci mit ähnlichen Nukleotidbasensequenzen in einem Genom vorliegen oder eine große Anzahl von genetischen Polymorphismen in den Loci existiert, wobei das Computersystem eine Aufzeichnungseinheit und eine arithmetische Verarbeitungseinheit umfasst und **dadurch gekennzeichnet ist, dass** die nachfolgenden Prozesse (A) bis (E) ausgeführt werden:

(A) in der Aufzeichnungseinheit werden Ableseinformationen von einer aus einem Subjekt erhaltenen DNA als Beobachtungsdaten bestehend aus der Nukleotidsequenz einer Ablesung sowie Allelen der Genloci oder des einzelnen Genlocus, mit welchen die Ablesung abgeglichen worden ist, aufgezeichnet;
(B) in der arithmetischen Verarbeitungseinheit wird auf Grundlage der Informationen aus der Aufzeichnungseinheit einer der nachfolgenden Initialisierungsprozesse (B)-1 und (B)-2 ausgeführt:

(B)-1: der Prozess der Berechnung eines Anfangswerts für $\theta$, welches die Allelfrequenz der Genloci oder des einzelnen Genlocus repräsentiert, und
(B)-2: der Prozess der Berechnung von Anfangswerten für zwei latente, nachfolgend beschriebene Variablen (i) und (ii), welche mit dem vorstehend beschriebenen $\theta$ und den Daten der DNA-Ableseinformationen des Subjekts, bei denen es sich um die Beobachtungsdaten handelt, in Beziehung stehen:

(i) eine von $\theta$ abhängige Variable $T_n$, welche eine Allelauswahl im Rahmen der Ablesung n in den Genloci oder dem einzelnen Genlocus repräsentiert,
(ii) eine A-posteriori-Wahrscheinlichkeit einer Indikatorvariable $Z_{nts}$ ($Z_{nts}$ ist eins, wenn $(T_n, S_n) = (t, s)$, und ist anderweitig null), welche $S_n$, das eine Startposition der Ablesung n darstellt und von $T_n$ abhängig ist, zusammenfasst, oder $Z_{nt}$ ($Z_{nt}$ ist eins, wenn $T_n = t$, und ist anderweitig null), welche die latente Variable $T_n$ zusammenfasst;

(C) in der arithmetischen Verarbeitungseinheit wird auf Grundlage des in Prozess (B)-1 berechneten Parameters $\theta$ der Berechnungsprozess der A-posteriori-Wahrscheinlichkeit der Indikatorvariable $Z_{nts}$ oder $Z_{nt}$ = 1 ausgeführt;
(D) in der arithmetischen Verarbeitungseinheit wird auf Grundlage der in Prozess (B)-2 oder (C) berechneten A-posteriori-Wahrscheinlichkeit der Indikatorvariable $Z_{nts}$ oder $Z_{nt}$ = 1 ein erster aktualisierter Wert für die Maximum-Likelihood-Schätzung des Parameters $\theta$ berechnet; und
(E) in der arithmetischen Verarbeitungseinheit werden auf Grundlage des in Prozess (D) berechneten ersten aktualisierten Werts für die Maximum-Likelihood-Schätzung des Parameters $\theta$ die Prozesse (C) und (D) erneut ausgeführt, und wird ein Wiederholungsschleifenprozess zur Berechnung eines zweiten aktualisierten Werts des Parameters $\theta$ wiederholt ausgeführt, bis zwischen dem neuen aktualisierten Wert für $\theta$ und dem vorherigen aktualisierten Wert für $\theta$ kein Unterschied mehr festgestellt wird, und der konvergierte Parameter $\theta$ als optimierter Wert in der Aufzeichnungseinheit aufgezeichnet wird,
wobei es sich bei den Daten, in welchen Ableseinformationen von der aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Prozesse (a) und (b) gekennzeichnet ist:

(a) ein Prozess, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, welche mit den Genloci oder dem einzelnen Genlocus abgeglichen worden sind, extrahiert werden; und
(b) ein Prozess, in welchem die in Prozess (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und Ableseinformationen erhalten werden, in welchen das Alignment einer jeden Ablesung mit jedem Allel der Genloci oder des einzelnen Genlocus sowie Alignmentzustände erfasst sind;

(III) im Rahmen jeder Ablesung zur Optimierung der Anzahl von erwarteten Alignments mit jedem Allel ausgewählter Genloci oder eines einzelnen Genlocus in Bezug auf Daten, in welchen Alignmentableseinformationen der Genloci oder des einzelnen Genlocus vermischt sind, wobei mehrere Loci mit ähnlichen Nukleotidbasensequenzen in einem Genom vorliegen oder eine große Anzahl von genetischen Polymorphismen in den Loci existiert, und wobei das Computersystem eine Aufzeichnungseinheit und eine arithmetische Verarbeitungseinheit umfasst und **dadurch gekennzeichnet ist, dass** die nachfolgenden Prozesse (A) bis (E) ausgeführt werden:

(A) in der Aufzeichnungseinheit werden Ableseinformationen von einer aus einem Subjekt erhaltenen DNA als Beobachtungsdaten bestehend aus der Nukleotidsequenz einer Ablesung sowie Allelen der Genloci oder des einzelnen Genlocus, mit welchen die Ablesung abgeglichen worden ist, aufgezeichnet;

(B) in der arithmetischen Verarbeitungseinheit wird auf Grundlage der aus der Aufzeichnungseinheit abgerufenen Beobachtungsdaten einer der nachfolgenden Initialisierungsprozesse (B)-1 und (B)-2 ausgeführt:

(B)-1: der Prozess der Berechnung eines Anfangswerts für eine A-posteriori-Verteilung von $\theta$ auf Grundlage eines Anfangswerts für den Hyperparameter $\alpha_t$, welcher frühere Informationen bezüglich der Allelfrequenz der Genloci oder des einzelnen Genlocus repräsentiert, und

(B)-2: der Prozess der Berechnung einer Anfangsverteilung von zwei latenten, nachfolgend beschriebenen Variablen (i) und (ii), welche mit der vorstehend beschriebenen Verteilung von $\theta$ und den Daten der DNA-Ableseinformationen des Subjekts, bei denen es sich um Beobachtungsdaten handelt, in Beziehung stehen:

(i) eine von $\theta$ abhängige Variable $T_n$, welche eine Allelauswahl im Rahmen der Ablesung n in den Genloci oder dem einzelnen Genlocus repräsentiert, und

(ii) eine A-posteriori-Verteilung einer Indikatorvariable $Z_{nts}$ ($Z_{nts}$ ist eins, wenn $(T_n, S_n) = (t, s)$, und ist anderweitig null), welche $S_n$ und die von $T_n$ abhängige Startposition der Ablesung n zusammenfasst, oder $Z_{nt}$ ($Z_{nt}$ ist eins, wenn $T_n = t$, und ist anderweitig null), welche die latente Variable $T_n$ zusammenfasst;

(C) in der arithmetischen Verarbeitungseinheit wird auf Grundlage der in Prozess (B)-1 berechneten Verteilung des Parameters $\theta$ der Berechnungsprozess der A-posteriori-Verteilung der Indikatorvariable $Z_{nts}$ oder $Z_{nt}$ ausgeführt;

(D) in der arithmetischen Verarbeitungseinheit wird auf Grundlage der in Prozess (B)-2 oder (C) berechneten A-posteriori-Verteilung von $Z_{nts}$ oder $Z_{nt}$ eine erste aktualisierte A-posteriori-Verteilung des Parameters $\theta$ berechnet;

(E) in der arithmetischen Verarbeitungseinheit werden auf Grundlage des ersten aktualisierten Werts für die A-posteriori-Verteilung von $\theta$ die Prozesse (C) und (D) erneut ausgeführt, und es wird ein Wiederholungsschleifenprozess zur Berechnung einer zweiten aktualisierten A-posteriori-Verteilung von $\theta$ wiederholt ausgeführt, bis zwischen dem erwarteten Wert für die neue aktualisierte A-posteriori-Verteilung von $\theta$ und dem erwarteten Wert für die vorherige aktualisierte A-posteriori-Verteilung von $\theta$ kein Unterschied mehr festgestellt wird, und wird der konvergierte erwartete Wert für die A-posteriori-Verteilung von $\theta$ als optimierter Wert in der Aufzeichnungseinheit aufgezeichnet;

wobei es sich bei den Daten, in welchen Ableseinformationen von einer aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Prozesse (a) und (b) gekennzeichnet ist:

(a) ein Prozess, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, welche mit den Genloci oder dem einzelnen Genlocus abgeglichen worden sind, extrahiert werden; und

(b) ein Prozess, in welchem die in Prozess (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und Ableseinformationen erhalten werden, in welchen das Alignment einer jeden Ablesung mit jedem Allel der Genloci oder des einzelnen Genlocus sowie Alignmentzustände erfasst sind; oder

(IV) zur Bestimmung des Genotyps eines jeden Genlocus ausgewählter Genloci oder eines einzelnen Genlocus eines Subjekts, wobei mehrere Loci mit ähnlichen Nukleotidbasensequenzen in einem Genom vorliegen oder eine große Anzahl von genetischen Polymorphismen in den Loci existiert, und wobei das Computersystem eine Aufzeichnungseinheit und eine arithmetische Verarbeitungseinheit umfasst und **dadurch gekennzeichnet ist, dass** die nachfolgenden Prozesse ($\alpha$) bis ($\delta$) ausgeführt werden:

($\alpha$) in der Aufzeichnungseinheit werden zumindest die Allelfrequenzen und die Sequenziertiefe der Gesamtzahl von Ablesungen der Genloci oder des einzelnen Genlocus des Subjekts, welche mittels des in einem der Ansprüche 1 bis 10 erwähnten Optimierungsverfahrens erhalten worden sind, aufgezeichnet;

($\beta$) in der arithmetischen Verarbeitungseinheit werden auf Grundlage der in der Aufzeichnungseinheit aufgezeichneten Allelfrequenzen der Genloci oder des einzelnen Genlocus eine Verarbeitung der Berechnung der individuellen Sequenziertiefe eines jeden Allels der Genloci oder des einzelnen Genlocus, sowie eine Verarbeitung der Zuordnung der berechneten individuellen Sequenziertiefe mit den Allelen der Genloci oder des einzelnen Genlocus ausgeführt;

($\gamma$) bei einem vorgegebenen Ablehnungsgrenzwert, welcher als Frequenzzahl von 5 bis 50% der mittleren Sequenziertiefe sämtlicher Ablesungen festgelegt ist, wird ein Prozess ausgeführt, in welchem Allele der Genloci oder des einzelnen Genlocus, deren individuelle Sequenziertiefe geringer ist als der Grenzwert, aus den Kandidaten für die Genotypisierung ausgeschlossen werden;

($\delta$):

($\delta$)-1 nach dem Ausschlussprozess ($\gamma$) wird im Falle des Vorliegens eines für die Genotypisierung des Genlocus der ausgewählten Genloci oder des einzelnen Genlocus ausgewählten Allels, wenn die individuelle Sequenziertiefe des Allels das Doppelte oder mehr als das Doppelte des Ablehnungsgrenzwerts beträgt, festgelegt, dass der Genotyp bezüglich des Allels homozygot ist, und wird, wenn sie weniger als das Doppelte des Ablehnungsgrenzwerts beträgt, festgelegt, dass der Genotyp bezüglich des Allels heterozygot ist und kein weiteres Allel bestimmt wird, und

($\delta$)-2 nach dem Ausschlussprozess ($\gamma$) wird im Falle des Vorliegens zweier für die Genotypisierung des Genlocus der ausgewählter Genloci oder des einzelnen Genlocus ausgewählten Allele, wenn die individuelle Sequenziertiefe jenes Allels, dessen individuelle Sequenziertiefe größer ist, weniger als das Doppelte des kleineren beträgt, festgelegt, dass der Genotyp bezüglich der beiden Allele heterozygot ist, oder wird, wenn die individuelle Sequenziertiefe jenes Allels, dessen individuelle Sequenziertiefe größer ist, das Doppelte oder mehr als das Doppelte des kleineren beträgt, festgelegt, dass der Genotyp bezüglich des Allels, dessen individuelle Sequenziertiefe größer ist, homozygot ist.

14. Computersystem gemäß Anspruch 13, wobei in jeder der Varianten (I) bis (III) die Ableseinformationen "Single End"-Ableseinformationen oder "Paired End"-Ableseinformationen sind.

15. Computersystem gemäß Anspruch 13 oder 14, welches **dadurch gekennzeichnet ist, dass** in jeder der Varianten (I) bis (III) das in Prozess (b) durchgeführte Alignment die Möglichkeit bietet, eine Ablesung mit mehreren Allelen der ausgewählten Genloci oder des einzelnen Genlocus abzugleichen.

16. Computersystem gemäß Anspruch 15, welches **dadurch gekennzeichnet ist, dass** zusätzlich zu den mit Allelen der ausgewählten Genloci oder des einzelnen Genlocus abgeglichenen und in Prozess (a) erhaltenen Ablesungen Ablesungen extrahiert werden, welche nicht mit dem menschlichen Gesamtgenom abgeglichen worden sind, und dies das Ziel des erneuten Alignments in Prozess (b) darstellt.

17. Computersystem gemäß einem der Ansprüche 13 bis 16, welches **dadurch gekennzeichnet ist, dass** es sich bei den ausgewählten Genloci oder dem einzelnen Genlocus um MHC-Genloci oder einen einzelnen MHC-Genlocus handelt.

18. Computersystem gemäß Anspruch 17, welches **dadurch gekennzeichnet ist, dass** es sich bei MHC um HLA handelt.

19. Computerprogramm, umfassend Befehle, welche bei Ausführung des Computerprogramms auf einem Computer diesen veranlassen:

(I) Ableseinformationen einer aus Allelen von ausgewählten Genloci oder einem einzelnen Genlocus erhaltenen DNA, wobei mehrere Loci mit ähnlichen Nukleotidbasensequenzen in einem Genom vorliegen oder eine große Anzahl von genetischen Polymorphismen in den Loci existiert, durch Realisierung der nachfolgenden Funktionen (1) bis (7) zu optimieren:

(A) die Funktion (1), in welcher Ableseinformationen von einer aus einem Subjekt erhaltenen DNA, die als Daten bestehend aus der Nukleotidsequenz einer Ablesung sowie Allelen der Genloci oder des einzelnen Genlocus, mit welchen die Ablesung abgeglichen worden ist, in einer Aufzeichnungseinheit aufgezeichnet worden sind, aus der Aufzeichnungseinheit abgerufen werden;

(B) die Funktion (2), in welcher auf Grundlage der in der Funktion (1) abgerufenen Ableseinformationen für jede Ablesung eine erwartete Anzahl von Alignments für jedes Allel der Genloci oder des einzelnen Genlocus durch Ausführen einer numerischen Verarbeitung berechnet wird;

(C) die Funktion (3), in welcher für jedes Allel die in der Funktion (B) quantifizierte erwartete Anzahl von Alignments für jedes Allel der Genloci oder des einzelnen Gelocus addiert wird, um auf diese Weise die Gesamtzahl von erwarteten Alignments zu berechnen;

(D) die Funktion (4), in welcher für jedes Allel die in der Funktion (3) berechnete Gesamtzahl von erwarteten Alignments durch die Summe der Gesamtzahl von erwarteten Alignments für sämtliche Allele der Genloci oder des einzelnen Genlocus geteilt wird, und der Prozess der Berechnung des Anteils an Ablesungen, welcher jedem Allel der Genloci oder des einzelnen Genlocus zugewiesen wird, in Bezug auf die mit Allelen der Genloci oder des einzelnen Genlocus abgeglichene Gesamtzahl von Ablesungen ausgeführt wird;

(E) die Funktion (5), in welcher der in der Funktion (4) berechnete Anteil an Ablesungen jedem Allel der Genloci oder des einzelnen Genlocus als Frequenz zugeordnet wird, und die Funktion (2), in welcher für jede Ablesung die erwartete Anzahl von Alignments mit jedem Allel der Genloci oder des einzelnen Genlocus berechnet wird, bei vorgegebener Zuordnungsfrequenz erneut ausgeführt wird;

(F) die Funktion (6), in welcher die Funktion (3) oder (4) für die mittels der Funktion (5) berechnete, neu erhaltene erwartete Anzahl von Alignments erneut ausgeführt wird, und der Prozess der Berechnung des Anteils an Ablesungen, welcher jedem Allel der Genloci oder des einzelnen Genlocus zugewiesen wird, in Bezug auf die mit Allelen der Genloci oder des einzelnen Genlocus abgeglichene Gesamtzahl von Ablesungen ausgeführt wird; und

(G) die Funktion (7), in welcher die Funktionen (5) und (6) wiederholt ausgeführt werden, bis zwischen der in der Funktion (5) berechneten Anzahl von erwarteten Alignments mit jedem Allel der Genloci oder des einzelnen Genlocus und der in der vorherigen Funktion (5) berechneten Anzahl für sämtliche Ablesungen kein Unterschied mehr festgestellt wird, oder zwischen dem in der Funktion (6) berechneten Wert für den Anteil an Ablesungen und dem in der vorherigen Funktion (6) berechneten Wert für sämtliche Allele der Genloci oder des einzelnen Genlocus kein Unterschied mehr festgestellt wird, und die konvergierte Anzahl von erwarteten Alignments für jede Ablesung mit jedem Allel der Genloci oder des einzelnen Genlocus, oder der konvergierte Wert des Anteils an Ablesungen für jedes Allel der Genloci oder des einzelnen Genlocus, als optimierte Daten anerkannt wird,

wobei es sich bei den Daten, in welchen Ableseinformationen von der aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Prozesse (a) und (b) gekennzeichnet ist:

(a) ein Prozess, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, welche mit den Genloci oder dem einzelnen Genlocus abgeglichen worden sind, extrahiert werden; und

(b) ein Prozess, in welchem die in Prozess (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und Ableseinformationen erhalten werden, in welchen das Alignment einer jeden Ablesung mit jedem Allel der Genloci oder des einzelnen Genlocus sowie Alignmentzustände erfasst sind;

(II) im Rahmen jeder Ablesung die Anzahl von erwarteten Alignments mit jedem Allel ausgewählter Genloci oder eines einzelnen Genlocus in Bezug auf Daten, in welchen Ableseinformationen von einer aus Allelen der Genloci oder des Genlocus erhaltenen DNA vermischt sind, wobei mehrere Loci mit ähnlichen Nukleotidbasensequenzen in einem Genom vorliegen oder eine große Anzahl von genetischen Polymorphismen in den Loci existiert, durch Realisierung der nachfolgenden Funktionen 1 bis 5 zu optimieren:

(A) Funktion 1, in welcher Ableseinformationen von einer aus einem Subjekt erhaltenen DNA, die als Beobachtungsdaten bestehend aus der Nukleotidsequenz einer Ablesung sowie Allelen der Genloci oder des einzelnen Genlocus, mit welchen die Ablesung abgeglichen worden ist, in einer Aufzeichnungseinheit aufgezeichnet worden sind, aus der Aufzeichnungseinheit abgerufen werden;

(B) Funktion 2, in welcher auf Grundlage der mittels Funktion 1 abgerufenen Beobachtungsdaten einer der nachfolgenden Initialisierungsprozesse (B)-1 und (B)-2 ausgeführt wird:

(B)-1: der Prozess der Berechnung eines Anfangswerts für $\theta$, welches die Allelfrequenz der Genloci oder des einzelnen Genlocus repräsentiert, und

(B)-2: der Prozess der Berechnung von Anfangswerten für zwei latente, nachfolgend beschriebene Variablen (i) und (ii), welche mit dem vorstehend beschriebenen $\theta$ und den Daten der DNA-Ableseinformationen des Subjekts, bei denen es sich um die Beobachtungsdaten handelt, in Beziehung stehen:

(i) eine von $\theta$ abhängige Variable $T_n$, welche eine Allelauswahl im Rahmen der Ablesung n in den Genloci oder dem einzelnen Genlocus repräsentiert, und

(ii) eine A-posteriori-Wahrscheinlichkeit einer Indikatorvariable $Z_{nts}$ ($Z_{nts}$ ist eins, wenn $(T_n, S_n) = (t, s)$, und ist anderweitig null), welche $S_n$, das eine Startposition der Ablesung n darstellt und von $T_n$ abhängig ist, zusammenfasst, oder $Z_{nt}$ ($Z_{nt}$ ist eins, wenn $T_n = t$, und ist anderweitig null), welche eine latente Variable $T_n$ zusammenfasst;

(C) Funktion 3, in welcher auf Grundlage des in Prozess (B)-1 berechneten Parameters $\theta$ der Berechnungsprozess der A-posteriori-Wahrscheinlichkeit der Indikatorvariable $Z_{nts}$ oder $Z_{nt} = 1$ ausgeführt wird;

(D) Funktion 4, in welcher auf Grundlage der in Prozess (B)-2 oder Funktion 3 berechneten A-posteriori-Wahrscheinlichkeit von $Z_{nts}$ oder $Z_{nt} = 1$ ein erster aktualisierter Wert der Maximum-Likelihood-Schätzung des Parameters $\theta$ berechnet wird;

(E) Funktion 5, in welcher auf Grundlage des ersten aktualisierten Werts für die Maximum-Likelihood-Schätzung von $\theta$ Funktion 3 und 4 erneut ausgeführt werden, und der Wiederholungsschleifenprozess zur Berechnung eines zweiten aktualisierten Werts für $\theta$ wiederholt ausgeführt wird, bis zwischen dem neuen aktualisierten Wert für $\theta$ und dem vorherigen aktualisierten Wert für $\theta$ kein Unterschied mehr festgestellt wird, und der konvergierte Parameter $\theta$ als optimierter Wert in der Aufzeichnungseinheit aufgezeichnet wird, wobei es sich bei den Daten, in welchen Ableseinformationen von der aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Prozesse (a) und (b) gekennzeichnet ist:

(a) ein Prozess, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, welche mit den Genloci oder dem einzelnen Genlocus abgeglichen worden sind, extrahiert werden; und

(b) ein Prozess, in welchem die in Prozess (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und Ableseinformationen erhalten werden, in welchen das Alignment einer jeden Ablesung mit jedem Allel der Genloci oder des einzelnen Genlocus sowie Alignmentzustände erfasst sind;

(III) im Rahmen jeder Ablesung die Anzahl von erwarteten Alignments mit jedem Allel ausgewählter Genloci oder eines einzelnen Genlocus in Bezug auf Daten, in welchen Ableseinformationen von einer aus Allelen der Genloci oder des Genlocus erhaltenen DNA vermischt sind, wobei mehrere Loci mit ähnlichen Nukleotidbasensequenzen in einem Genom vorliegen oder eine große Anzahl von genetischen Polymorphismen in den Loci existiert, durch Realisierung der nachfolgenden Funktionen 1 bis 5 zu optimieren:

(A) Funktion 1, in welcher Ableseinformationen von einer aus einem Subjekt erhaltenen DNA, die als Beobachtungsdaten bestehend aus der Nukleotidsequenz einer Ablesung sowie den Allelen der Genloci oder des einzelnen Genlocus, mit welchen die Ablesung abgeglichen worden ist, in einer Aufzeichnungseinheit aufgezeichnet worden sind, aus der Aufzeichnungseinheit abgerufen werden;

(B) Funktion 2, in welcher auf Grundlage der mittels Funktion 1 abgerufenen Beobachtungsdaten einer der nachfolgenden Initialisierungsprozesse (B)-1 und (B)-2 ausgeführt wird:

(B)-1: ein Prozess der Berechnung eines aktualisierten Werts für die A-posteriori-Verteilung von $\theta$ auf Grundlage eines Anfangswerts für den Hyperparameter $\alpha_t$, welcher frühere Informationen bezüglich der Allelfrequenz der Genloci oder des einzelnen Genlocus repräsentiert, und

(B)-2: ein Prozess der Berechnung von Anfangsverteilungen für zwei latente, nachfolgend beschriebene Variablen (i) und (ii), welche mit der vorstehend beschriebenen Verteilung von $\theta$ und den Daten der DNA-Ableseinformationen des Subjekts, bei denen es sich um Beobachtungsdaten handelt, in Beziehung stehen:

(i) eine von $\theta$ abhängige Variable $T_n$, welche eine Allelauswahl im Rahmen der Ablesung n in den Genloci oder dem einzelnen Genlocus repräsentiert, und

(ii) eine A-posteriori-Verteilung einer Indikatorvariable $Z_{nts}$ ($Z_{nts}$ ist eins, wenn $(T_n, S_n) = (t, s)$, und ist anderweitig null), welche $S_n$, das eine Startposition der Ablesung n darstellt und von $T_n$ abhängig ist, zusammenfasst, oder $Z_{nt}$ ($Z_{nt}$ ist eins, wenn $T_n = t$, und ist anderweitig null), welche die latente Variable $T_n$ zusammenfasst;

(C) Funktion 3, in welcher auf Grundlage der in Prozess (B)-1 berechneten Verteilung von $\theta$ der Berechnungsprozess der A-posteriori-Verteilung der Indikatorvariable $Z_{nts}$ oder $Z_{nt}$ ausgeführt wird;

(D) Funktion 4, in welcher auf Grundlage der in Funktion (B)-2 oder Funktion 3 berechneten A-posteriori-Verteilung von $Z_{nts}$ oder $Z_{nt}$ eine erster aktualisierter Wert für die A-posteriori-Verteilung von $\theta$ berechnet wird; und

(E) Funktion 5, in welcher auf Grundlage des ersten aktualisierten Werts für die A-posteriori-Verteilung von $\theta$ Funktion 3 und 4 erneut ausgeführt werden, und ein Wiederholungsschleifenprozess zur Berechnung eines zweiten aktualisierten Werts für die A-posteriori-Verteilung von $\theta$ wiederholt ausgeführt wird, bis zwischen einem erwarteten Wert für die neue aktualisierte A-posteriori-Verteilung von $\theta$ und einem erwarteten Wert für die vorherige aktualisierte A-posteriori-Verteilung von $\theta$ kein Unterschied mehr festgestellt wird, und der konvergierte erwartete Wert für die A-posteriori-Verteilung von $\theta$ als optimierter Wert in der Aufzeichnungseinheit aufgezeichnet wird;

wobei es sich bei den Daten, in welchen Ableseinformationen von einer aus Allelen der ausgewählten Genloci oder des einzelnen Genlocus erhaltenen DNA vermischt sind, um Ableseinformationen handelt, in welchen ein Alignment einer jeden Ablesung mit jedem Allel der Genloci oder des einzelnen Genlocus durch Abgleichen von Ableseinformationen in einem Gesamtgenom eines Subjekts mit in einer Datenbank eingetragenen Referenznukleotidsequenzen von Allelen der Genloci oder des einzelnen Genlocus erfasst ist, und das Alignment durch Ausführen der nachfolgenden Prozesse (a) und (b) gekennzeichnet ist:

(a) ein Prozess, in welchem, was Nukleotidsequenzinformationen aus Ablesungen in dem aus dem Subjekt erhaltenen Gesamtgenom betrifft, Ablesungen mit einer menschlichen Referenzgenomsequenz abgeglichen werden, und anschließend jene, welche mit den Genloci oder dem einzelnen Genlocus abgeglichen worden sind, extrahiert werden; und

(b) ein Prozess, in welchem die in Prozess (a) erhaltenen und mit den Genloci oder dem einzelnen Genlocus abgeglichenen Ablesesequenzinformationen mit den in einer Datenbank eingetragenen Referenznukleotidsequenzen von Allelen der Genloci oder des einzelnen Genlocus abgeglichen werden, und Ableseinformationen erhalten werden, in welchen das Alignment einer jeden Ablesung mit jedem Allel der Genloci oder des einzelnen Genlocus sowie Alignmentzustände erfasst sind; oder

(IV) den Genotyp eines Genlocus ausgewählter Genloci oder eines einzelnen Genlocus eines Subjekts, wobei mehrere Loci mit ähnlichen Nukleotidbasensequenzen in einem Genom vorliegen oder eine große Anzahl von genetischen Polymorphismen in den Loci existiert, durch Realisierung der nachfolgenden Funktionen ($\alpha$) bis ($\delta$) zu bestimmen:

($\alpha$) eine Funktion $\alpha$, in welcher zumindest die Allelfrequenzen und die Sequenziertiefe aus der Gesamtzahl von Ablesungen der Genloci oder des einzelnen Genlocus des Subjekts, die durch Ausführen des in einer der Varianten (I) bis (III) erwähnten Computerprogramms erhalten worden sind, abgerufen werden;

(β) eine Funktion β, in welcher auf Grundlage der durch Ausführen der Funktion α abgerufenen Allelfrequenzen der Genloci oder des einzelnen Genlocus eine Berechnung der individuellen Sequenziertiefe eines jeden Allels der Genloci oder des einzelnen Genlocus, sowie eine Zuordnung der berechneten individuellen Sequenziertiefe zu den Allelen der Genloci oder des einzelnen Genlocus ausgeführt wird;

(γ) eine Funktion γ, in welcher bei einem vorgegebenen Ablehnungsgrenzwert, welcher als Frequenzzahl von 5 bis 50% der mittleren Sequenziertiefe sämtlicher Ablesungen festgelegt ist, ein Prozess ausgeführt wird, in welchem Allele der Genloci oder des einzelnen Genlocus, deren durch die Funktion β spezifizierte individuelle Sequenziertiefe geringer ist als der Grenzwert, aus den Kandidaten für die Genotypisierung ausgeschlossen werden; und

(δ): eine Funktion δ, welche nachfolgend als (δ)-1 und (δ)-2 spezifiziert ist:

(δ)-1 nach dem Ausschlussprozess in Funktion (γ) wird im Falle des Vorliegens eines für die Genotypisierung des Genlocus der ausgewählten Genloci oder des einzelnen Genlocus ausgewählten Allels, wenn die individuelle Sequenziertiefe des Allels das Doppelte oder mehr als das Doppelte des Ablehnungsgrenzwerts beträgt, ein Prozess durchgeführt, welcher festlegt, dass der Genotyp bezüglich des Allels homozygot ist, und, wenn sie weniger als das Doppelte des Ablehnungsgrenzwerts beträgt, festlegt, dass der Genotyp bezüglich des Allels heterozygot ist und kein weiteres Allel bestimmt wird, und

(δ)-2 nach dem Ausschlussprozess in Funktion (γ) wird im Falle des Vorliegens zweier für die Genotypisierung des Genlocus der ausgewählten Genloci oder des einzelnen Genlocus ausgewählter Allele, wenn die individuelle Sequenziertiefe jenes Allels, dessen individuelle Sequenziertiefe größer ist, weniger als das Doppelte des kleineren beträgt, ein Prozess durchgeführt, welcher festlegt, dass der Genotyp bezüglich der beiden Allele heterozygot ist, oder, wenn die individuelle Sequenziertiefe jenes Allels, dessen individuelle Sequenziertiefe größer ist, das Doppelte oder mehr als das Doppelte des kleineren beträgt, festlegt, dass der Genotyp bezüglich des Allels, dessen individuelle Sequenziertiefe größer ist, homozygot ist.

20. Computerprogramm gemäß Anspruch 19, wobei in jeder der Varianten (I) bis (III) die Ableseinformationen "Single End"-Ableseinformationen oder "Paired End"-Ableseinformationen sind.

21. Computerprogramm gemäß Anspruch 19 oder 20, welches **dadurch gekennzeichnet ist, dass** in jeder der Varianten (I) bis (III) die Erzeugung der Daten, in welchen Ableseinformationen von einer aus Allelen der ausgewählten Genloci oder des einzelnen Genlocus erhaltenen DNA vermischt sind, durch Ausführen der nachfolgenden Funktionen (a) und (b) realisiert wird:

(a) eine Funktion, in welcher, was Nukleotidsequenzinformationen aus den aus dem Subjekt erhaltenen Ablesungen betrifft, Ablesungen mit einer menschlichen Nukleotidgenomsequenz abgeglichen werden, und anschließend jene, welche mit Allelen der Genloci oder des einzelnen Genlocus abgeglichen worden sind, extrahiert werden; und

(b) eine Funktion, in welcher, was die in Funktion (a) erhaltenen und mit Allelen der Genloci oder des einzelnen Genlocus abgeglichenen Ablesesequenzinformationen betrifft, Ablesungen mit den in einer Datenbank eingetragenen Referenznukleotidsequenzen von Allelen der Genloci oder des einzelnen Genlocus abgeglichen werden, mit jedem Allel der Genloci oder des einzelnen Genlocus abgeglichene Ablesungen extrahiert werden, und Ableseinformationen erhalten werden, in welchen das Alignment einer jeden Ablesung mit jedem Allel der Genloci oder des einzelnen Genlocus erfasst ist.

22. Computerprogramm gemäß einem der Ansprüche 19 bis 21, welches **dadurch gekennzeichnet ist, dass** in jeder der Varianten (I) bis (III) das in Funktion (b) durchgeführte Alignment die Möglichkeit bietet, eine Ablesung mit mehreren Allelen der ausgewählten Genloci oder des einzelnen Genlocus abzugleichen.

23. Computerprogramm gemäß Anspruch 22, welches **dadurch gekennzeichnet ist, dass** zusätzlich zu den mit Allelen der ausgewählten Genloci oder des einzelnen Genlocus abgeglichenen und in Funktion (a) erhaltenen Ablesungen Ablesungen extrahiert werden, welche nicht mit dem menschlichen Gesamtgenom abgeglichen worden sind, und dies das Ziel des erneuten Alignments in Funktion (b) darstellt.

24. Computerprogramm gemäß einem der Ansprüche 19 bis 23, welches **dadurch gekennzeichnet ist, dass** es sich bei den ausgewählten Genloci oder dem einzelnen Genlocus um MHC-Genloci oder einen einzelnen MHC-Genlocus handelt.

**25.** Computerprogramm gemäß Anspruch 24, welches **dadurch gekennzeichnet ist, dass** es sich bei MHC um HLA handelt.

**26.** Computerlesbares Aufzeichnungsmedium, auf welchem das in einem der Ansprüche 19 bis 25 erwähnte Computerprogram aufgezeichnet ist.

**Revendications**

**1.** Procédé d'optimisation d'informations de lectures d'ADN, dans lequel :

(I) les étapes (1) à (6) suivantes sont exécutées en utilisant des données dans lesquelles les informations de lectures d'ADN issues d'allèles de loci génétiques sélectionnés ou d'un locus génétique individuel sont mélangées, où il y a de multiples loci ayant des séquences de bases nucléotidiques similaires dans un génome ou un grand nombre de polymorphismes génétiques existe dans les loci :

(1) une étape dans laquelle, pour chaque lecture, la quantification d'un nombre attendu de correspondances avec chaque allèle des loci génétiques ou du locus génétique individuel, est réalisée ;
(2) une étape dans laquelle le nombre attendu de correspondances quantifié à l'étape (1) est additionné pour chaque allèle des loci génétiques ou du locus génétique individuel pour calculer un nombre total de correspondances attendues ;
(3) une étape dans laquelle le nombre total de correspondances attendues calculé à l'étape (2) est divisé par la somme du nombre total de correspondances attendues de tous les allèles des loci génétiques ou du locus génétique individuel, pour calculer une fraction de lectures attribuées à chaque allèle des loci génétiques ou du locus génétique individuel par rapport à une quantité de lectures totales mises en correspondance avec les allèles des loci génétiques ou du locus génétique individuel ;
(4) une étape dans laquelle la fraction de lectures obtenue à l'étape (3) est assignée à chaque allèle des loci génétiques ou du locus génétique individuel en tant que fréquence, et sur la base des fréquences assignées, pour chaque lecture, le nombre attendu de correspondances avec chaque allèle des loci génétiques ou du locus génétique individuel est de nouveau calculé ;
(5) une étape dans laquelle l'étape (2) ou (3) est de nouveau exécutée sur la base du nombre attendu mis à jour de correspondances obtenu à l'étape (4), pour calculer une fraction du nombre de lectures attribuées à un allèle des loci génétiques ou du locus génétique individuel par rapport à une quantité de lectures totale mise en correspondance avec les allèles des loci génétiques ou du locus génétique individuel ; et
(6) une étape dans laquelle les étapes (4) et (5) sont exécutées de manière répétée, jusqu'à ce qu'une différence entre le nombre attendu de correspondances avec chaque allèle des loci génétiques ou du locus génétique individuel calculé à l'étape (4) et celui calculé à l'étape (4) précédente ne soit pas reconnue pour toutes les lectures, ou une différence entre la fraction de lectures calculée à l'étape (5) et celle calculée à l'étape (5) précédente, ne soit pas reconnue pour tous les allèles dans les loci génétiques ou le locus génétique individuel, et le nombre attendu convergent de correspondances pour chaque lecture pour chaque allèle des loci génétiques ou du locus génétique individuel, ou, la fraction convergente de lectures pour les allèles des loci génétiques ou du locus génétique individuel, est reconnue en tant que données optimisées, dans lequel les données dans lesquelles les informations de lecture d'ADN issues d'allèles des loci génétiques sélectionnés ou de locus génétique individuel sont mélangées, sont des informations de lecture dans lesquelles la mise en correspondance de chaque lecture avec chaque allèle des loci génétiques ou du locus génétique individuel est identifiée par mise en correspondance d'informations de lectures dans un génome entier d'un sujet avec des séquences nucléotidiques de référence d'allèles des loci génétiques ou du locus génétique individuel enregistrées dans une base de données, et la mise en correspondance est **caractérisée par** exécution des étapes (a) et (b) suivantes :

(a) une étape dans laquelle par rapport aux informations de séquences nucléotidiques de lectures dans le génome entier obtenu du sujet, les lectures sont mises en correspondance avec une séquence de référence du génome humain, puis celles mises en correspondance avec les loci génétiques ou le locus génétique individuel sont extraites ; et
(b) une étape dans laquelle les informations de séquences de lectures mises en correspondance avec les loci génétiques ou le locus génétique individuel obtenues à l'étape (a) sont mises en correspondance avec les séquences nucléotidiques de référence d'allèles des loci génétiques ou du locus génétique individuel enregistrées dans une base de données, et les lectures mises en correspondance avec chaque allèle des loci ou du locus individuel génétique(s) sont extraites, et les informations de lectures sont obtenues, dans laquelle la correspondance de chaque lecture avec chaque allèle des loci génétiques ou du locus génétique individuel est identifiée ; ou

(II) la correspondance des informations de lectures d'ADN d'un sujet avec des allèles de loci génétiques sélectionnés ou d'un locus génétique individuel est optimisée par un ordinateur, où il y a de multiples loci ayant des séquences de bases nucléotidiques similaires dans un génome ou un grand nombre de polymorphismes génétiques existe dans les loci, ce qui inclut une étape de détermination pour chaque lecture d'un nombre attendu de correspondances avec des allèles des loci génétiques ou du locus génétique individuel, et une étape d'estimation des fréquences d'allèles $\theta$ des loci génétiques ou du locus génétique individuel, $\theta$ étant un paramètre objectif, où $\theta$ est un vecteur T dimensionnel, le T étant le nombre d'allèles des loci génétiques ou du locus génétique individuel, étant donné les séquences nucléotidiques de toutes les lectures dans les données dans lesquelles les informations de lectures d'ADN issues d'allèles des loci génétiques ou du locus génétique individuel sont mélangées en tant que données observées R, et qui sont **caractérisées en ce que**, par rapport à (1) une variante latente $T_n$ dépendante de $\theta$ par rapport à la sélection d'allèles des loci génétiques ou locus génétique individuel de lecture n, et (2) une variable latente $S_n$ dépendante de $T_n$ par rapport à une position de départ de lecture n, et étant donné une séquence nucléotidique de lecture n en tant que donnée observée $R_n$, au moins (i) les variables $T_n$ et $S_n$ ou (ii) la variable $T_n$ sont incorporées pour calculer une estimation du paramètre dans un processus d'estimation du paramètre objectif $\theta$, de telle sorte que les données observées $R_n$ dépendent des variables latentes durant le processus d'estimation du paramètre objectif $\theta$ à partir des données observées Rn,

dans lequel les données dans lesquelles les informations de lectures d'ADN issues d'allèles des loci génétiques sélectionnés ou de locus génétique individuel sont mélangées, sont des informations de lectures dans lesquelles la correspondance de chaque lecture avec chaque allèle des loci génétiques ou du locus génétique individuel est identifiée par mise en correspondance d'informations de lectures dans un génome entier d'un sujet avec des séquences nucléotidiques de référence d'allèles des loci génétiques ou du locus génétique individuel enregistrées dans une base de données, et la mise en correspondance est **caractérisée par** exécution des étapes (a) et (b) suivantes :

(a) une étape dans laquelle par rapport aux informations de séquences nucléotidiques de lectures dans le génome entier obtenu du sujet, les lectures sont mises en correspondance avec la séquence de référence du génome humain, puis celles mises en correspondance avec les loci génétiques ou le locus génétique individuel sont extraites ; et
(b) une étape dans laquelle les informations de séquences de lectures mises en correspondance avec les loci génétiques ou le locus génétique individuel obtenues à l'étape (a) sont mises en correspondance avec les séquences nucléotidiques de référence d'allèles des loci génétiques ou du locus génétique individuel enregistrées dans une base de données, et les lectures mises en correspondance avec chaque allèle des loci ou du locus individuel génétique(s) sont extraites, et les informations de lectures sont obtenues, dans laquelle la correspondance de chaque lecture avec chaque allèle des loci génétiques ou du locus génétique individuel est identifiée.

2. Procédé d'optimisation selon la revendication 1, dans lequel les informations de lectures sont des informations de lectures à extrémité unique ou des informations de lectures à extrémités appariées.

3. Procédé d'optimisation selon la revendication 1 ou 2, qui est **caractérisé par** le calcul pour chaque lecture du nombre attendu de correspondances avec chaque allèle des loci génétiques sélectionnés ou du locus génétique individuel, et le calcul d'une valeur estimée de la fréquence d'allèles $\theta$ des loci génétiques ou du locus génétique individuel, $\theta$ étant un paramètre objectif, par l'exécution d'étapes sur la base d'une méthode d'estimation de la vraisemblance maximale, ou d'une méthode d'estimation bayésienne.

4. Procédé d'optimisation selon l'une quelconque des revendications 1 à 3, qui est **caractérisé par** l'utilisation d'une variable d'indicateur $Z_{nts}$ ($Z_{nts}$ est égal à un si $(T_n, S_n) = (t, s)$, et zéro autrement), qui résume les variables latentes $T_n$ et $S_n$, ou $Z_{nt}$ ($Z_n$ test égal à un si $T_n = t$, et zéro autrement), qui résume la variable latente $T_n$, dans la variante (II).

5. Procédé d'optimisation selon la revendication 4, qui est **caractérisé par** l'exécution des étapes (1) à (5) suivantes :

(1) une étape dans laquelle une première valeur de mise à jour d'une probabilité postérieure de $Z_{nts} = 1$ ou $Z_{nt} = 1$ est calculée sur la base d'une valeur initiale donnée de $\theta_t$, et une première valeur mise à jour d'une estimation de vraisemblance maximale de $\theta_t$ est en outre calculée, sur la base de la première valeur mise à jour de la probabilité postérieure de $Z_{nts} = 1$ ou $Z_{nt} = 1$, ou,

(2) une étape dans laquelle une première valeur mise à jour d'une estimation de vraisemblance maximale de $\theta_t$ est calculée, sur la base d'une valeur initiale donnée d'une probabilité postérieure de $Z_{nts} = 1$ ou $Z_{nt} = 1$ ;

(3) une étape dans laquelle une valeur mise à jour de la probabilité postérieure de $Z_{nts} = 1$ ou de $Z_{nt} = 1$ est calculée, sur la base de la valeur mise à jour de l'estimation de vraisemblance maximale de $\theta_t$ calculée par une étape (4) précédente (cependant, pour la première fois, par l'étape (1) ou l'étape (2)) ;

(4) une étape dans laquelle une valeur mise à jour d'une estimation de vraisemblance maximale de $\theta_t$ est calculée, sur la base de la valeur mise à jour de la probabilité postérieure de $Z_{nts} = 1$ ou $Z_{nt} = 1$ à l'étape (3) ; et

(5)

(i) une étape dans laquelle le logarithme du rapport de vraisemblance est calculé sur la base de la valeur mise à jour de la probabilité postérieure de $Z_{nts} = 1$ ou de $Z_{nt} = 1$ calculée à l'étape (3) et la valeur mise à jour de l'estimation de vraisemblance maximale de $\theta_t$ calculée à l'étape (4), pour évaluer la convergence de la vraisemblance logarithmique,

(ii) une étape dans laquelle la convergence de la valeur mise à jour de la probabilité postérieure de $Z_{nts} = 1$ ou de $Z_{nt} = 1$ calculée à l'étape (3) est évaluée, ou

(iii) une étape dans laquelle la convergence de la valeur mise à jour de l'estimation de vraisemblance maximale de $\theta_t$ est calculée à l'étape (4), et,

si la convergence est reconnue, $\theta_t$ à chaque étape est déterminée en tant que valeur d'estimation finale, et si la convergence n'est pas reconnue, la répétition des étapes (3), (4) et (5) est déterminée.

6. Procédé d'optimisation selon la revendication 4, qui est **caractérisé par** l'exécution des étapes (1) à (5) suivantes :

(1) une étape dans laquelle une première distribution postérieure mise à jour de $Z_{nts}$ ou $Z_{nt}$ est calculée sur la base d'une distribution postérieure initiale donnée de $\theta_t$ sur la base de l'hyperparamètre $\alpha 0$ représentant des informations préalables de fréquences d'allèles des loci génétiques sélectionnés ou du locus génétique individuel, et une première distribution postérieure mise à jour de $\theta_t$ est en outre calculée sur la base de la première distribution postérieure mise à jour de $Z_{nts}$ ou $Z_{nt}$, ou,

(2) une étape dans laquelle une distribution postérieure mise à jour de $\theta_t$ est calculée sur la base d'une distribution initiale donnée de $Z_{nts}$ ou $Z_{nt}$ ;

(3) une étape dans laquelle une distribution postérieure mise à jour de $Z_{nts}$ ou de $Z_{nt}$ est calculée, sur la base de la distribution postérieure mise à jour de $\theta t$, calculée par l'étape (4) précédente (cependant, pour la première fois, par l'étape (1) ou l'étape (2)) ;

(4) une étape dans laquelle une distribution postérieure mise à jour de $\theta_t$ est calculée, sur la base de la distribution postérieure mise à jour de $Z_{nts}$ ou $Z_{nt}$ calculée à l'étape (3) ; et

(5) une étape dans laquelle la convergence de la distribution postérieure mise à jour de $\theta_t$ calculée à l'étape (4) est évaluée, et si la convergence est reconnue, la valeur attendu de $\theta_t$ est déterminée en tant que valeur d'estimation finale, et si la convergence n'est pas reconnue, la répétition des étapes (3), (4) et (5) est déterminée.

7. Procédé d'optimisation selon l'une quelconque des revendications 1 à 6, qui est **caractérisé en ce que** la mise en correspondance réalisée dans les étapes (a) et (b) permet la mise en correspondance d'une lecture avec de multiples allèles des loci génétiques sélectionnés ou du locus génétique individuel.

8. Procédé d'optimisation selon la revendication 7, qui est **caractérisé en ce qu'**outre les lectures mises en correspondance avec les allèles des loci génétiques sélectionnés ou du locus génétique individuel obtenues à l'étape (a), les lectures qui ne sont pas mises en correspondance avec le génome humain entier sont extraites, et il devient la cible de la nouvelle mise en correspondance à l'étape (b).

9. Procédé d'optimisation selon l'une quelconque des revendications 1 à 8, qui est **caractérisé en ce que** les loci génétiques sélectionnés ou le locus génétique individuel sont des loci génétiques de CMH ou un locus génétique individuel d'un CMH.

10. Procédé d'optimisation selon la revendication 9, qui est **caractérisé en ce que** le CMH est le HLA.

11. Procédé de détermination de génotype pour des loci génétiques sélectionnés ou un locus génétique individuel, dans lequel :

(I) la profondeur individuelle de couverture des lectures pour chaque allèle des loci génétiques ou du locus génétique individuel est calculée à partir de la fréquence d'allèles des loci génétiques sélectionnés ou du locus génétique individuel obtenue du procédé d'optimisation selon l'une quelconque des revendications 1 à 10, les deux ou moins de deux allèles supérieurs des loci génétiques ou du locus génétique individuel sont sélectionnés à partir d'une liste d'allèles qui sont triés sur la base de la profondeur individuelle de la couverture par ordre décroissant, et les allèles sont déterminés en tant que candidats pour le génotypage de chaque locus génétique des loci génétiques sélectionnés ou du locus génétique individuel ; ou

(II) la profondeur individuelle de couverture de lectures pour chaque allèle des loci génétiques ou du locus génétique individuel est calculée à partir de la fréquence d'allèles des loci génétiques ou du locus génétique individuel obtenue à partir du procédé d'optimisation selon l'une quelconque des revendications 1 à 10, les deux ou moins de deux allèles supérieurs des loci génétiques ou du locus génétique individuel sont sélectionnés à partir d'une liste d'allèles qui sont triés sur la base de la profondeur individuelle de la couverture par ordre décroissant, et les allèles sont déterminés en tant que candidats pour le génotypage de chaque locus génétique des loci génétiques sélectionnés ou du locus génétique individuel, et qui est **caractérisé par** la définition d'un seuil de profondeur de couverture d'allèles à 5 à 50% de la profondeur de couverture des lectures totales en tant que seuil de rejet, et l'élimination des allèles des loci génétiques ou du locus génétique dont la profondeur individuelle de couverture est inférieure au seuil de candidats pour le génotypage de chaque locus génétique des loci génétiques sélectionnés ou du locus génétique individuel.

12. Procédé de détermination selon la revendication 11, qui est **caractérisé en ce que**, dans la variante (II), après élimination des allèles de candidats pour le génotypage de chaque locus génétique des loci génétiques sélectionnés ou du locus génétique individuel, le (i) ou (ii) suivants est déterminé :

(i) pour un cas dans lequel il y a un allèle qui est sélectionné pour le génotypage du locus génétique des loci génétiques ou du locus génétique individuel, si la profondeur individuelle de couverture de l'allèle est deux fois ou plus le seuil de rejet, il est déterminé que le génotype est homozygote de l'allèle, et s'il est moins de deux fois le seuil de rejet, il est déterminé que le génotype est hétérozygote de l'allèle et pas d'autre allèle n'est déterminé, et

(ii) pour le cas dans lequel il y a deux allèles qui sont sélectionnés pour le génotypage du locus génétique des loci génétiques ou du locus génétique individuel, si la profondeur individuelle de couverture de celui dont la profondeur individuelle de couverture est plus grande est inférieure à deux fois la plus petite, il est déterminé que le génotype est hétérozygote des deux allèles, ou si la profondeur individuelle de couverture de celui dont la profondeur individuelle de couverture est plus grande est deux fois ou plus la plus petite, il est déterminé que le génotype est homozygote de l'allèle dont la profondeur individuelle de couverture est plus grande.

13. Système informatique :

(I) pour l'optimisation d'informations de lectures d'ADN issues d'allèles des loci génétiques ou d'un locus génétique individuel à optimiser, où il y a de multiples loci ayant des séquences de bases nucléotidiques similaires dans un génome ou un grand nombre de polymorphismes génétiques existe dans les loci, comprenant une unité d'enregistrement et une unité de traitement arithmétique, qui est **caractérisée en ce que** les processus (A) à (G) suivants sont exécutés :

(A) dans l'unité d'enregistrement, les informations de lectures d'ADN issues d'un sujet sont enregistrées en tant que données de séquences nucléotidiques de lecture et d'allèles des loci génétiques ou du locus génétique individuel avec lesquels la lecture est mise en correspondance ;

(B) dans l'unité de traitement arithmétique, sur la base des informations de la section d'enregistrement, pour chaque lecture, un nombre attendu de correspondances pour chaque allèle des loci génétiques ou du locus génétique individuel est calculé par exécution d'un traitement numérique ;

(C) pour chaque allèle, le nombre de correspondances attendues dans le processus (B) est additionné pour chaque allèle des loci génétiques ou du locus génétique individuel pour calculer un nombre total de correspondances attendues ;

(D) pour chaque allèle, le nombre total de correspondances attendues calculé dans le processus (C) est divisé par la somme du nombre total des correspondances attendues pour les allèles des loci génétiques ou du locus génétique individuel, et le processus de calcul d'une fraction du nombre de lectures attribuées à chaque allèle des loci génétiques ou du locus génétique individuel par rapport à la quantité totale de lectures mises en correspondance avec les allèles des loci génétiques ou du locus génétique individuel est exécuté ;

(E) la fraction de lectures calculée dans le processus (C) ci-dessus est assignée en tant que fréquence à chaque allèle des loci génétiques ou du locus génétique individuel, et étant donné la fréquence d'assignation, le processus (B) est exécuté de nouveau dans lequel pour chaque lecture, le nombre attendu de correspondances avec chaque allèle des loci génétiques ou du locus génétique individuel est calculé ;

(F) le processus (C) ou (D) est de nouveau exécuté pour le nouveau nombre anticipé de correspondances calculé par le processus (E), et le processus de calcul de la fraction de lectures attribuée à chaque allèle des loci génétiques ou du locus génétique individuel par rapport à la quantité totale de lectures mises en correspondance avec les allèles des loci génétiques ou du locus génétique individuel est exécuté ; et

(G) les processus (E) et (F) sont exécutés de manière répétée, jusqu'à ce que pour toutes les lectures, la différence entre le nombre de correspondances attendues avec chaque allèle des loci génétiques ou du locus génétique individuel calculé dans le processus (E) et celui calculé dans le processus (E) précédent ne soit pas reconnue, ou pour tous les allèles des loci génétiques ou du locus génétique individuel, la différence entre la fraction de lectures calculées dans le processus (F) et celle calculée dans le processus (F) précédent ne soit pas reconnue, et un nombre convergent de correspondances attendues pour chaque lecture avec chaque allèle des loci génétiques ou du locus génétique individuel, ou la valeur convergente de la fraction de lectures pour chaque allèle des loci génétiques ou du locus génétique individuel, soit reconnue en tant que les données optimisées,

dans lequel les données dans lesquelles les informations de lectures d'ADN issues d'allèles des loci génétiques sélectionnés ou de locus génétique individuel sont mélangées, sont des informations de lectures dans lesquelles la correspondance de chaque lecture avec chaque allèle des loci génétiques ou du locus génétique individuel est identifiée par mise en correspondance d'informations de lectures dans un génome entier d'un sujet avec des séquences nucléotidiques de référence d'allèles des loci génétiques ou du locus génétique individuel enregistrées dans une base de données, et la correspondance est **caractérisée par** exécution des processus (a) et (b) suivants :

(a) un processus dans lequel par rapport aux informations de séquences nucléotidiques de lectures dans le génome entier obtenu du sujet, les lectures sont mises en correspondance avec la séquence de référence du génome humain, puis celles mises en correspondance avec les loci génétiques ou le locus génétique individuel sont extraites ; et

(b) un processus dans lequel les informations de séquences de lectures mises en correspondance avec les loci génétiques ou le locus génétique individuel obtenues à l'étape (a) sont mises en correspondance avec les séquences nucléotidiques de référence d'allèles des loci génétiques ou du locus génétique individuel enregistrées dans une base de données, et les informations de lecture sont obtenues, dans lequel la correspondance de chaque lecture avec chaque allèle des loci génétiques ou du locus génétique individuel et des états de correspondance sont identifiés ;

(II) pour l'optimisation, pour chaque lecture, le nombre de correspondances attendues avec chaque allèle des loci génétiques sélectionnés ou du locus génétique individuel pour les données dans lesquelles les informations de lectures d'ADN issues d'allèles des loci génétiques ou de locus génétique individuel sont mélangées, où il y a de multiples loci ayant des séquences de bases nucléotidiques similaires dans un génome ou un grand nombre de polymorphismes génétiques existe dans les loci, comprenant une unité d'enregistrement et une unité de traitement arithmétique, qui est **caractérisée en ce que** les processus (A) à (E) suivants sont exécutés :

(A) dans l'unité d'enregistrement, les informations de lectures d'ADN issues d'un sujet sont enregistrées en tant que données observées de séquences nucléotidiques de lecture et d'allèles des loci génétiques ou du locus génétique individuel avec lequel la lecture est mise en correspondance ;

(B) dans l'unité de traitement arithmétique, sur la base des informations de la section d'enregistrement, l'un ou l'autre des processus d'initialisation (B)-1 et (B)-2 suivants est exécuté :

(B)-1 : le processus de calcul d'une valeur initiale de $\theta$, qui représente la fréquence d'allèles des loci génétiques ou du locus génétique individuel, et

(B)-2 : le processus de calcul de valeurs initiales de deux variables latentes, (i) et (ii) décrites ci-dessous, qui sont liées au $\theta$ décrit ci-dessus et les données des informations de lectures d'ADN du sujet, qui sont les données observées :

(i) est une variable $T_n$ qui est dépendante de $\theta$, qui représente la sélection d'allèles de la lecture n des loci génétiques ou du locus génétique individuel,

(ii) une probabilité postérieure d'une variable d'indicateur $Z_{nts}$ ($Z_{nts}$ est égal à un si $(T_n, S_n) = (t,$

s), et zéro autrement), qui résume $S_n$ et une position de début de lecture n qui est dépendante de $T_n$, ou $Z_{nt}$ ($Z_{nt}$ est égal à un si $T_n = t$, et zéro autrement), qui résume la variable latente $T_n$ ;

(C) dans l'unité de traitement arithmétique, sur la base du paramètre θ calculé dans le processus (B)-1, le processus de calcul de la probabilité postérieure de la variable d'indicateur $Z_{nts}$ ou $Z_{nt} = 1$ est exécuté ;

(D) dans l'unité de traitement arithmétique, une première valeur mise à jour de l'estimation de vraisemblance maximale du paramètre θ est calculée sur la base de la probabilité postérieure de la variable d'indicateur $Z_{nts}$ ou $Z_{nt} = 1$ calculée dans le processus (B)-2 ou (C) ; et

(E) dans l'unité de traitement arithmétique, les processus (C) et (D) sont exécutés de nouveau sur la base de la première valeur mise à jour de l'estimation de vraisemblance maximale du paramètre θ calculé dans le processus (D), et un processus en boucle itératif pour calculer une seconde valeur mise à jour du paramètre θ est exécuté de manière répétée, jusqu'à ce qu'une différence entre la nouvelle valeur mise à jour de θ et la valeur mise à jour précédente de θ ne soit pas reconnue, et le paramètre θ convergent est enregistré en tant que valeur optimisée dans l'unité d'enregistrement,

dans lequel les données dans lesquelles les informations de lectures d'ADN issues d'allèles des loci génétiques sélectionnés ou de locus génétique individuel sont mélangées, sont des informations de lecture dans lesquelles la correspondance de chaque lecture avec chaque allèle des loci génétiques ou du locus génétique individuel est identifiée par mise en correspondance d'informations de lecture dans un génome entier d'un sujet avec des séquences nucléotidiques de référence d'allèles des loci génétiques ou du locus génétique individuel enregistrées dans une base de données, et la correspondance est **caractérisée par** l'exécution des processus (a) et (b) suivants :

(a) un processus dans lequel par rapport aux informations de séquences nucléotidiques de lectures dans le génome entier obtenu du sujet, les lectures sont mises en correspondance avec une séquence de référence du génome humain, puis celles mises en correspondance avec les loci génétiques ou le locus génétique individuel sont extraites ; et

(b) un processus dans lequel les informations de séquences de lectures mises en correspondance avec les loci génétiques ou le locus génétique individuel obtenues à l'étape (a) sont mises en correspondance avec les séquences nucléotidiques de référence d'allèles des loci génétiques ou de locus génétique individuel enregistrées dans une base de données, et les informations de lectures sont obtenues, dans lequel la correspondance de chaque lecture avec chaque allèle des loci génétiques ou du locus génétique individuel et des états de correspondance sont identifiés ;

(III) pour l'optimisation, pour chaque lecture, du nombre de correspondances attendues avec chaque allèle des loci génétiques sélectionnés ou du locus génétique individuel pour les données où les informations de mise en correspondance de lectures des loci génétiques ou de locus génétique individuel sont mélangées, où il y a de multiples loci ayant des séquences de bases nucléotidiques similaires dans un génome ou un grand nombre de polymorphismes génétiques existe dans les loci, comprenant une unité d'enregistrement et une unité de traitement arithmétique, qui est **caractérisée en ce que** les processus (A) à (E) suivants sont exécutés :

(A) dans l'unité d'enregistrement, les informations de lectures d'ADN issues du sujet sont enregistrées en tant que données observées de séquences nucléotidiques de lecture et d'allèles des loci génétiques ou du locus génétique individuel avec lesquelles la lecture est mise en correspondance,

(B) dans l'unité de traitement arithmétique, sur la base des données observées retrouvées à partir de l'unité d'enregistrement, l'un ou l'autre des processus d'initialisation (B)-1 et (B)-2 suivants est exécuté :

(B)-1 : le processus de calcul d'une valeur initiale de distribution postérieure de θ, sur la base d'une valeur initiale d'hyperparamètre $\alpha_t$ qui représente des informations préalables de la fréquence d'allèles des loci génétiques ou d'un locus génétique individuel, et

(B)-2 : le processus de calcul de distribution initiale de deux variables latentes, (i) et (ii) décrites ci-dessous, qui sont liées à la distribution de θ décrite ci-dessus et aux données d'informations de lectures d'ADN du sujet, qui sont des données observées :

(i) est une variable $T_n$ qui est dépendante de θ, qui représente la sélection d'allèles de la lecture n dans les loci génétiques ou du locus génétique individuel, et

(ii) une distribution postérieure d'une variable d'indicateur $Z_{nts}$ ($Z_{nts}$ est égal à un si ($T_n$, $S_n$) = (t, s), et zéro autrement), qui résume $S_n$, la position de départ de la lecture n qui est dépendante de $T_n$, ou $Z_{nt}$ ($Z_{nt}$ est égal à un si $T_n = t$, et zéro autrement), qui résume la variable latente $T_n$ ;

(C) dans l'unité de traitement arithmétique, sur la base de la distribution du paramètre θ calculé dans le processus (B)-1, le processus de calcul de la distribution postérieure de la variable d'indicateur $Z_{nts}$ ou $Z_{nt}$ est exécuté ;

(D) dans l'unité de traitement arithmétique, la première distribution postérieure mise à jour du paramètre θ est calculée sur la base de la distribution postérieure de $Z_{nts}$ ou $Z_{nt}$ calculée dans le processus (B)-2 ou (C),

(E) dans l'unité de traitement arithmétique, les processus (C) et (D) sont exécutés de nouveau sur la base de la première valeur mise à jour de la distribution postérieure de θ, et un processus en boucle itératif pour calculer une seconde distribution mise à jour postérieure de θ est exécuté de manière répétée, jusqu'à ce qu'une différence entre la valeur anticipée de la nouvelle distribution postérieure mise à jour de θ et la valeur anticipée de la distribution postérieure mise à jour préalable de θ ne soit pas reconnue, et la valeur anticipée convergente de la distribution postérieure de θ est enregistrée en tant que la valeur optimisée dans l'unité d'enregistrement,

dans lequel les données dans lesquelles les informations de lectures d'ADN issues d'allèles des loci génétiques sélectionnés ou de locus génétique individuel sont mélangées, sont des informations de lectures dans lesquelles la correspondance de chaque lecture avec chaque allèle des loci génétiques ou du locus génétique individuel est identifiée par mise en correspondance d'informations de lectures dans un génome entier d'un sujet avec des séquences nucléotidiques de référence d'allèles des loci génétiques ou du locus génétique individuel enregistrées dans une base de données, et la mise en correspondance est **caractérisée par** l'exécution des processus (a) et (b) suivants :

(a) un processus dans lequel par rapport aux informations de séquences nucléotidiques de lectures dans le génome entier obtenu du sujet, les lectures sont mises en correspondance avec la séquence de référence du génome humain, puis celles mises en correspondance avec les loci génétiques ou le locus génétique individuel sont extraites ; et

(b) un processus dans lequel les informations de séquences de lectures mises en correspondance avec des allèles de loci génétiques ou de locus génétique individuel obtenus dans le processus (a) sont mises en correspondance avec des séquences nucléotidiques de référence d'allèles des loci génétiques ou de locus génétique individuel enregistrées dans une base de données, et les informations de lectures sont obtenues, dans lequel la correspondance de chaque lecture avec chaque allèle des loci génétiques ou du locus génétique individuel et des états de mise en correspondance sont identifiés ; ou

(IV) pour la détermination du génotype de chaque locus génétique de loci génétiques sélectionnés ou d'un locus génétique individuel d'un sujet, où il y a de multiples loci ayant des séquences de bases nucléotidiques similaires dans un génome ou un grand nombre de polymorphismes génétiques existe dans les loci, comprenant une unité d'enregistrement et une unité de traitement arithmétique, qui est **caractérisé en ce que** les processus (α) à (δ) suivants sont exécutés :

(α) dans l'unité d'enregistrement, au moins les fréquences d'allèles et la profondeur de couverture de lectures totales des loci génétiques ou d'un locus génétique individuel du sujet qui sont obtenues par le procédé d'optimisation selon l'une quelconque des revendications 1 à 10 sont enregistrées ;

(β) dans l'unité de traitement arithmétique, sur la base des fréquences d'allèles des loci génétiques ou du locus génétique individuel enregistrées dans l'unité d'enregistrement, le traitement de calcul de la profondeur individuelle de couverture de chaque allèle des loci génétiques ou du locus génétique individuel, et le traitement de l'attribution de la profondeur de couverture individuelle calculée aux allèles des loci génétiques ou du locus génétique individuel, sont exécutés ;

(γ) étant donné une valeur de seuil de rejet, qui est définie en tant que nombre de fréquence de 5 à 50 % de la profondeur de couverture moyenne de toutes les lectures, un processus dans lequel les allèles des loci génétiques ou du locus génétique individuel dont la profondeur individuelle de couverture est inférieure à la valeur seuil sont exclus des candidats pour le génotypage, est exécuté ;

(δ) :

(δ)-1 après le processus d'exclusion de (γ), pour un cas dans lequel il y a un allèle qui est sélectionné pour le génotypage du locus génétique des loci génétiques sélectionnés ou du locus génétique individuel, si la profondeur individuelle de couverture de l'allèle est deux fois ou plus la valeur seuil de rejet, il est déterminé que le génotype est homozygote de l'allèle, et s'il est moins de deux fois la valeur seuil de rejet, il est déterminé que le génotype est hétérozygote de l'allèle et pas d'autre allèle n'est déterminé, et

(δ)-2 après le processus d'exclusion de (γ), pour un cas dans lequel il y a deux allèles qui sont sélectionnés pour le génotypage du locus génétique des loci génétiques sélectionnés ou du locus génétique individuel, si la profondeur individuelle de couverture de celui dont la profondeur individuelle de couverture est plus grande est inférieure à deux fois la plus petite, il est déterminé que le génotype est hétérozygote des deux allèles, ou si la profondeur individuelle de couverture de celui dont la profondeur individuelle de couverture est plus grande est deux fois ou plus la plus petite, il est déterminé que le génotype est homozygote de l'allèle dont la profondeur individuelle de couverture est plus grande.

**14.** Système informatique selon la revendication 13, dans lequel, dans l'une des variantes (I) à (III), les informations de lectures sont des informations de lectures à extrémité unique ou des informations de lectures à extrémités appariées.

**15.** Système informatique selon la revendication 13 ou 14, qui est **caractérisé en ce que**, dans l'une quelconque des variantes (I) à (III), la mise en correspondance réalisée dans le processus (b) permet la mise en correspondance d'une lecture avec de multiples allèles des loci génétiques sélectionnés ou du locus génétique individuel.

**16.** Système informatique selon la revendication 15, qui est **caractérisé en ce qu'**outre les lectures mises en correspondance avec les allèles des loci génétiques sélectionnés ou du locus génétique individuel obtenues dans le processus (a), les lectures qui ne sont pas mises en correspondance avec le génome humain entier sont extraites, et il devient la cible de la nouvelle mise en correspondance au processus (b).

**17.** Système informatique selon l'une quelconque des revendications 13 à 16, qui est **caractérisé en ce que** les loci génétiques sélectionnés ou le locus génétique individuel sont des loci génétiques de CMH ou un locus génétique individuel d'un CMH.

**18.** Système informatique selon la revendication 17, qui est **caractérisé en ce que** le CMH est le HLA.

**19.** Programme informatique comprenant des instructions qui, lorsque le programme informatique est exécuté par un ordinateur, conduisent l'ordinateur à :

(I) optimiser des informations de lectures d'ADN issues d'allèles des loci génétiques sélectionnés ou d'un locus génétique individuel, où il y a de multiples loci ayant des séquences de bases nucléotidiques similaires dans un génome ou un grand nombre de polymorphismes génétiques existe dans les loci, en réalisant les fonctions (1) à (7) suivantes :

(A) la fonction (1) dans laquelle des informations de lectures d'ADN issues d'un sujet enregistrées dans une unité d'enregistrement en tant que données de séquence nucléotidique de lecture et d'allèles des loci génétiques ou du locus génétique individuel avec lesquels la lecture est mise en correspondance sont récupérées de l'unité d'enregistrement ;
(B) la fonction (2) dans laquelle sur la base des informations de lecture récupérées dans la fonction (1), pour chaque lecture, un nombre anticipé de correspondances pour chaque allèle des loci génétiques ou du locus génétique individuel est calculé par exécution d'un traitement numérique ;
(C) la fonction (3) dans laquelle pour chaque allèle, le nombre de correspondances quantifiées dans la fonction (2) est additionné pour chaque allèle des loci génétiques ou du locus génétique individuel pour calculer le nombre total de correspondances attendues ;
(D) la fonction (4) dans laquelle pour chaque allèle, le nombre total de correspondances attendues calculé dans la fonction (3) est divisé par la somme du nombre total des correspondances attendues pour les allèles des loci génétiques ou des loci génétiques individuels, et le processus de calcul de la fraction du nombre de lectures attribuées à chaque allèle des loci génétiques ou du locus génétique individuel par rapport à la quantité totale de lectures mises en correspondance avec les allèles des loci génétiques ou du locus génétique individuel est exécuté ;
(E) la fonction (5) dans laquelle la fraction de lectures calculées dans la fonction (4) est assignée en tant que fréquence à chaque allèle des loci génétiques ou du locus génétique individuel, et étant donné la fréquence d'assignation, la fonction (2) est exécutée de nouveau dans laquelle pour chaque lecture, le nombre attendu de correspondances avec chaque allèle des loci génétiques ou du locus génétique individuel est de nouveau calculé ;
(F) la fonction (6) dans laquelle la fonction (3) ou (4) est exécutée de nouveau pour le nombre attendu nouvellement obtenu de correspondances calculé par la fonction (5), et le processus de calcul de la fraction de lectures attribuées à chaque allèle des loci génétiques ou du locus génétique individuel par rapport à

**EP 3 239 875 B1**

la quantité totale de lectures mises en correspondance avec les allèles des loci génétiques ou du locus génétique individuel est exécuté ; et

(G) la fonction (7) dans laquelle les fonctions (5) et (6) sont exécutées de manière répétée, jusqu'à ce que pour toutes les lectures, la différence entre le nombre attendu de correspondances avec chaque allèle des loci génétiques ou du locus génétique individuel calculé dans la fonction (5) et celui calculé dans la fonction (5) précédente, ne soit pas reconnue, ou pour tous les allèles des loci génétiques ou du locus génétique individuel, la différence entre la valeur de la fraction de lectures calculée dans la fonction (6) et celle calculée dans la fonction (6) précédente, ne soit pas reconnue, et le nombre convergent de correspondances attendues pour chaque lecture avec chaque allèle des loci génétiques ou du locus génétique individuel, la valeur convergente de la fraction de lectures pour chaque allèle des loci génétiques ou du locus génétique individuel, soit reconnue en tant que données optimisées,

dans lequel les données dans lesquelles les informations de lectures d'ADN issues d'allèles des loci génétiques sélectionnés ou du locus génétique individuel sont mélangées, sont des informations de lectures dans lesquelles la correspondance de chaque lecture avec chaque allèle des loci génétiques ou du locus génétique individuel est identifiée par mise en correspondance d'informations de lectures dans un génome entier d'un sujet avec des séquences nucléotidiques de référence d'allèles des loci génétiques ou du locus génétique individuel enregistrées dans une base de données, et la mise en correspondance est **caractérisée par** exécution des processus (a) et (b) suivants :

(a) un processus dans lequel par rapport aux informations de séquences nucléotidiques de lectures dans le génome entier obtenu du sujet, les lectures sont mises en correspondance avec la séquence de référence du génome humain, puis celles mises en correspondance avec les loci génétiques ou le locus génétique individuel sont extraites ; et

(b) un processus dans lequel les informations de séquences de lectures mises en correspondance avec des allèles de loci génétiques ou de locus génétique individuel obtenus dans le processus (a) sont mises en correspondance avec des séquences nucléotidiques de référence d'allèles des loci génétiques ou de locus génétique individuel enregistrées dans une base de données, et les informations de lecture sont obtenues, dans lequel la correspondance de chaque lecture avec chaque allèle des loci génétiques ou du locus génétique individuel et des états de correspondance sont identifiés ;

(II) optimiser, pour chaque lecture, le nombre de correspondances attendues avec chaque allèle des loci génétiques sélectionnés ou d'un locus génétique individuel pour des données dans lesquelles les informations de lecture d'ADN issues d'allèles des loci génétiques ou de locus individuel sont mélangées, où il y a de multiples loci ayant des séquences de bases nucléotidiques similaires dans un génome ou un grand nombre de polymorphismes génétiques existe dans les loci, par réalisation des fonctions 1 à 5 suivantes :

(A) la fonction 1 dans laquelle les informations de lectures d'ADN issues d'un sujet enregistrées dans une unité d'enregistrement en tant que données observées de séquences nucléotidiques de lectures et d'allèles des loci génétiques ou du locus génétique individuel avec lesquelles la lecture est mise en correspondance, sont récupérées de l'unité d'enregistrement ;

(B) la fonction 2 dans laquelle sur la base des données observées récupérées par la fonction 1, l'un ou l'autre des processus d'initialisation (B)-1 et (B)-2 suivants est exécuté :

(B)-1 : le processus de calcul d'une valeur initiale de $\theta$, qui représente la fréquence d'allèles des loci génétiques ou du locus génétique individuel, et

(B)-2 : le processus de calcul de valeurs initiales de deux variables latentes, (i) et (ii) décrites ci-dessous, qui sont liées au $\theta$ décrit ci-dessus et les données des informations de lectures d'ADN du sujet, qui sont les données observées :

(i) une variable $T_n$ qui est dépendante de $\theta$, qui représente la sélection d'allèles de la lecture n des loci génétiques ou du locus génétique individuel, et

(ii) une probabilité postérieure d'une variable d'indicateur $Z_{nts}$ ($Z_{nts}$ est égal à un si $(T_n, S_n) = (t, s)$, et zéro autrement), qui résume $S_n$ qui est une position de début de lecture n qui est dépendante de $T_n$, ou $Z_{nt}$ ($Z_{nt}$ est égal à un si $T_n = t$, et zéro autrement), qui résume une variable latente $T_n$ ;

(C) la fonction 3 dans laquelle sur la base du paramètre $\theta$ calculé dans le processus (B)-1, le processus de calcul de la probabilité postérieure de la variable d'indicateur $Z_{nts}$ ou $Z_{nt} = 1$ est exécuté ;

(D) la fonction 4 dans laquelle une première valeur mise à jour de l'estimation de vraisemblance maximale

du paramètre $\theta$ est calculée sur la base de la probabilité postérieure de $Z_{nts}$ ou $Z_{nt} = 1$ calculée dans le processus (B)-2 ou la fonction 3 ; et

(E) la fonction 5 dans laquelle les fonctions 3 et 4 sont exécutées de nouveau sur la base de la première valeur mise à jour de l'estimation de vraisemblance maximale de $\theta$, et le processus en boucle itératif de calcul d'une seconde valeur mise à jour de $\theta$, est exécuté plusieurs fois, jusqu'à ce qu'une différence entre la nouvelle valeur mise à jour de $\theta$ et la valeur mise à jour précédente de $\theta$ ne soit pas reconnue, et le paramètre convergent $\theta$ est enregistré en tant que valeur optimisée dans l'unité d'enregistrement,

dans lequel les données dans lesquelles les informations de lectures d'ADN issues d'allèles des loci génétiques sélectionnés ou du locus génétique individuel sont mélangées, sont des informations de lectures dans lesquelles la correspondance de chaque lecture avec chaque allèle des loci génétiques ou du locus génétique individuel est identifiée par mise en correspondance d'informations de lecture dans un génome entier d'un sujet avec des séquences nucléotidiques de référence d'allèles des loci génétiques ou du locus génétique individuel enregistrées dans une base de données, et la correspondance est **caractérisée par** exécution des processus (a) et (b) suivants :

(a) un processus dans lequel par rapport aux informations de séquences nucléotidiques de lectures dans le génome entier obtenu du sujet, les lectures sont mises en correspondance avec la séquence de référence du génome humain, puis celles mises en correspondance avec les loci génétiques ou le locus génétique individuel sont extraites ; et

(b) un processus dans lequel les informations de séquences de lectures mises en correspondance avec des allèles de loci génétiques ou de locus génétique individuel obtenus dans le processus (a) sont mises en correspondance avec des séquences nucléotidiques de référence d'allèles des loci génétiques ou de locus génétique individuel enregistrées dans une base de données, et les informations de lecture sont obtenues, dans lequel la correspondance de chaque lecture avec chaque allèle des loci génétiques ou du locus génétique individuel et des états de correspondance sont identifiés ;

(III) optimiser, pour chaque lecture, le nombre de correspondances attendues avec chaque allèle des loci génétiques sélectionnés ou du locus génétique individuel pour les données dans lesquelles les informations de lectures d'ADN issues d'allèles des loci génétiques ou de locus individuel sont mélangées, où il y a de multiples loci ayant des séquences de bases nucléotidiques similaires dans un génome ou un grand nombre de polymorphismes génétiques existe dans les loci, en réalisant la totalité ou une partie des fonctions suivantes 1 à 5 :

(A) la fonction 1 dans laquelle les informations de lectures d'ADN issues d'un sujet enregistrées dans une unité d'enregistrement en tant que données observées de séquences nucléotidiques de lecture et d'allèles des loci génétiques ou du locus génétique individuel avec lesquels la lecture est mise en correspondance, sont récupérées de l'unité d'enregistrement ;

(B) la fonction 2 dans laquelle sur la base des données observées récupérées par la fonction 1, l'un ou l'autre des processus d'initialisation (B)-1 et (B)-2 suivants est exécuté :

(B)-1 : un processus de calcul d'une valeur mise à jour de distribution postérieure de $\theta$, sur la base d'une valeur initiale d'hyperparamètre $\alpha_t$ qui représente des informations préalables de la fréquence d'allèle des loci génétiques ou d'un locus génétique individuel, et

(B)-2 : un processus de calcul de distributions initiales de deux variables latentes, (i) et (ii) décrites ci-dessous, qui sont liées à la distribution de $\theta$ décrite ci-dessus et aux données d'informations de lectures d'ADN du sujet, qui sont des données observées :

(i) est une variable $T_n$ qui est dépendante de $\theta$, qui représente la sélection d'allèles de la lecture $n$ des loci génétiques ou du locus génétique individuel,

(ii) une distribution postérieure d'une variable d'indicateur $Z_{nts}$ ($Z_{nts}$ est égal à un si $(T_n, S_n) = (t, s)$, et zéro autrement), qui résume $S_n$ qui est une position de début de lecture $n$ qui est dépendante de $T_n$, ou $Z_{nt}$ ($Z_{nt}$ est égal à un si $T_n = t$, et zéro autrement), qui résume la variable latente $T_n$ ;

(C) la fonction 3 dans laquelle sur la base de la distribution $\theta$ calculée dans le processus (B)-1, le processus de calcul de la distribution postérieure de la variable d'indicateur $Z_{nts}$ ou $Z_{nt}$ est exécuté ;

(D) la fonction 4 qui est une première valeur mise à jour de la distribution postérieure de $\theta$ est calculée sur la base de la distribution postérieure de $Z_{nts}$ ou de $Z_{nt}$ calculée en fonction de la fonction (B)-2 ou de la fonction 3 ; et

(E) la fonction 5 dans laquelle les fonctions 3 et 4 sont exécutées de nouveau sur la base de la première valeur mise à jour de la distribution postérieure de $\theta$, et un processus en boucle itératif pour calculer une seconde valeur mise à jour de la distribution postérieure de $\theta$ est exécuté de manière répétée, jusqu'à ce qu'une différence entre une valeur attendue de la nouvelle distribution postérieure mise à jour de $\theta$ et une valeur attendue de la distribution postérieure mise à jour préalable de $\theta$ ne soit pas reconnue, et la valeur attendue convergente de la distribution postérieure de $\theta$ est enregistrée en tant que valeur optimisée dans l'unité d'enregistrement,

dans lequel les données dans lesquelles les informations de lectures d'ADN issues d'allèles des loci génétiques sélectionnés ou du locus génétique individuel sont mélangées, sont des informations de lecture dans lesquelles la correspondance de chaque lecture avec chaque allèle des loci génétiques ou du locus génétique individuel est identifiée par mise en correspondance d'informations de lectures dans un génome entier d'un sujet avec des séquences nucléotidiques de référence d'allèles des loci génétiques ou du locus génétique individuel enregistrées dans une base de données, et la correspondance est **caractérisée par** exécution des processus (a) et (b) suivants :

(a) un processus dans lequel par rapport aux informations de séquences nucléotidiques de lectures dans le génome entier obtenu du sujet, les lectures sont mises en correspondance avec la séquence de référence du génome humain, puis celles mises en correspondance avec les loci génétiques ou le locus génétique individuel sont extraites ; et

(b) un processus dans lequel les informations de séquences de lectures mises en correspondance avec des allèles de loci génétiques ou de locus génétique individuel obtenus dans le processus (a) sont mises en correspondance avec des séquences nucléotidiques de référence d'allèles des loci génétiques ou de locus génétique individuel enregistrées dans une base de données, et les informations de lectures sont obtenues, dans lequel la correspondance de chaque lecture avec chaque allèle des loci génétiques ou du locus génétique individuel et des états de mise en correspondance sont identifiés ;

(IV) déterminer le génotype d'un locus génétique de loci génétiques sélectionnés ou d'un locus génétique individuel d'un sujet, où il y a de multiples loci ayant des séquences de bases nucléotidiques similaires dans un génome ou un grand nombre de polymorphismes génétiques existe dans les loci, en réalisant les fonctions ($\alpha$) à ($\delta$) suivantes :

($\alpha$) une fonction $\alpha$, dans laquelle au moins des fréquences d'allèles et une profondeur de couverture de lectures totales des loci génétiques ou d'un locus génétique individuel du sujet qui sont obtenues par exécution du programme informatique tel que décrit dans l'une quelconque des variantes (I) à (III), sont récupérées ;

($\beta$) une fonction $\beta$, dans laquelle sur la base des fréquences d'allèles des loci génétiques ou du locus génétique individuel récupérées par l'exécution de la fonction $\alpha$, le calcul de la profondeur individuelle de couverture de chaque allèle des loci génétiques ou du locus génétique individuel, et l'attribution de la profondeur de couverture individuelle calculée aux allèles des loci génétiques ou du locus génétique individuel, sont exécutés ;

($\gamma$) une fonction $\gamma$, dans laquelle étant donné une valeur seuil de rejet, qui est définie en tant que nombre de fréquence de 5 à 50% de la profondeur de couverture moyenne de toutes les lectures, un processus dans lequel les allèles des loci génétiques ou de locus génétique individuel dont la profondeur de couverture individuelle spécifiée par la fonction $\beta$ est inférieure à la valeur seuil sont exclus des candidats pour le génotypage, est exécuté ; et

($\delta$) : une fonction $\delta$ spécifiée comme ($\delta$)-1 et ($\delta$)-2 ci-dessous :

($\delta$)-1 après le processus d'exclusion dans la fonction ($\gamma$), pour un cas dans lequel il y a un allèle qui est sélectionné pour le génotypage du locus génétique des loci génétiques sélectionnés ou du locus génétique individuel, si la profondeur individuelle de couverture de l'allèle est deux fois ou plus la valeur seuil de rejet, un processus dans lequel il est déterminé que le génotype est homozygote de l'allèle, et s'il est inférieur à deux fois la valeur seuil de rejet, il est déterminé que le génotype est hétérozygote de l'allèle et pas d'autre allèle n'est déterminé, et

($\delta$)-2 après le processus d'exclusion dans la fonction ($\gamma$), pour un cas dans lequel il y a deux allèles qui sont sélectionnés pour le génotypage du locus génétique des loci génétiques sélectionnés ou du locus génétique individuel, si la profondeur individuelle de couverture de celui dont la profondeur individuelle de couverture est plus grande est inférieure à deux fois la plus petite, un processus dans lequel il est déterminé que le génotype est hétérozygote des deux allèles, ou si la profondeur individuelle de couverture de celui dont la profondeur individuelle de couverture est plus grande est deux fois ou plus la plus petite, il est déterminé que le génotype est homozygote de l'allèle dont la profondeur individuelle de couverture est plus grande, est exécuté.

20. Programme informatique selon la revendication 19, dans lequel, dans l'une des variantes (I) à (III), les informations de lectures sont des informations de lectures à extrémité unique ou des informations de lectures à extrémités appariées.

21. Programme informatique selon la revendication 19 ou 20, qui est **caractérisé en ce que**, dans l'une quelconque des variantes (I) à (III), la génération des données dans lesquelles les informations de lectures d'ADN issues d'allèles des loci génétiques sélectionnés ou du locus génétique individuel sont mélangées, est réalisée par exécution des fonctions (a) et (b) suivantes :

(a) une fonction dans laquelle par rapport aux informations de séquences nucléotidiques de lectures obtenues du sujet, les lectures sont mises en correspondance avec la séquence nucléotidique du génome humain, puis celles mises en correspondance avec allèles des loci génétiques ou le locus génétique individuel sont extraites ; et
(b) une fonction dans laquelle par rapport aux informations de séquences de lectures mises en correspondance avec les allèles des loci génétiques ou du locus génétique individuel obtenus dans la fonction (a), les lectures sont mises en correspondance avec des séquences nucléotidiques de référence d'allèles de loci génétiques ou du locus génétique individuel qui sont enregistrées dans une base de données, et les lectures mises en correspondance avec chaque allèle des loci ou du locus individuel sont extraites, et les informations de lecture sont obtenues, dans lequel la correspondance de chaque lecture avec chaque allèle des loci génétiques ou du locus génétique individuel est identifiée.

22. Système informatique selon l'une quelconque des revendications 19 à 21, qui est **caractérisé en ce que**, dans l'une quelconque des variantes (I) à (III), la mise en correspondance réalisée dans la fonction (b) permet la mise en correspondance d'une lecture avec de multiples allèles des loci génétiques sélectionnés ou du locus génétique individuel.

23. Programme informatique selon la revendication 22, qui est **caractérisé en ce que**, outre les lectures mises en correspondance avec les allèles des loci génétiques sélectionnés ou du locus génétique individuel obtenus dans la fonction (a), les lectures qui ne sont pas mises en correspondance avec le génome humain entier sont extraites, et il devient la cible de la nouvelle mise en correspondance au processus (b).

24. Système informatique selon l'une quelconque des revendications 19 à 23, qui est **caractérisé en ce que** les loci génétiques sélectionnés ou le locus génétique individuel sont des loci génétiques de CMH ou un locus génétique d'un CMH individuel.

25. Programme informatique selon la revendication 24, qui est **caractérisé en ce que** le CMH est le HLA.

26. Support d'enregistrement pouvant être lu par ordinateur, dans lequel le programme informatique selon l'une quelconque des revendications 19 à 25 est enregistré.

[Fig. 1]

EP 3 239 875 B1

[Fig. 2-1]

$N$: Total number of reads

$T$: Total number of HLA alleles

$Z_{nt}$: An indicator variable that equals to one if read n is generated from HLA allele t, and zero otherwise

$E[Z_{nt}]$: The expected number of mappings of read n to HLA allele t

$\theta_t$: The fraction of the total expected number of reads allocated to HLA allele t relative to the total read amount

$E[\theta_t]$: The expected value of the fraction of the total expected number of reads allocated to HLA allele t relative to the total read amount

$\alpha_0$: A hyperparameter

Start

S4-11
HLA allele reference sequences are retrieved
A list of reads and HLA alleles to which reads are mapped is retrieved

S4-12
Initialization of $E[Z_{nt}]$:
for $n = 1$ to $N$
  for $t = 1$ to $T$
    $E[Z_{nt}]=1/T$;
Initialization of $E[\theta_t]$:
for $t = 1$ to $T$
  $E[\theta_t]=1/T$;

S4-13
for $n = 1$ to $N$
  for $t = 1$ to $T$
    [ Recalculation of $E[Z_{nt}]$ ];
for $t = 1$ to $T$
  $r_t = \sum_{n=1}^{N} E[Z_{nt}]$

S4-14
for $t = 1$ to $T$
  $E[\theta_t] = \dfrac{\alpha_0 + r_t}{\sum_{t'}(\alpha_0 + r_{t'})}$

S4-15
Convergence determination:
All $E[\theta_t]$ are not changed

NO

YES

End

68

[Fig. 2-2]

N: Total number of reads

T: Total number of HLA alleles

$Z_{nts}$: An indicator variable that equals to one if read n is generated from HLA allele t at start position s, and zero otherwise

$E[Z_{nts}]$: The expected number of mappings of read n to HLA allele t at position s

$l_t$: The length of HLA allele t

$L$: The length of reads

$M_n$: The total number of locations to which read n mapped

$\theta_t$: The fraction of the total expected number of reads allocated to HLA allele t relative to the total read amount

**Start**

S4-21

HLA allele reference sequences are retrieved from the IMGT/HLA or other databases
Reads and HLA alleles to which reads are mapped is retrieved from a BAM file

S4-22

Initialization of $E[Z_{nts}]$: for $n = 1$ to $N$
for $t = 1$ to $T$
for $s = 1$ to $l_{th} - L + 1$
$E_Z[Z_{nts}] = 1/M_n$

Initialization of $\theta_t$: for $t = 1$ to $T$
$\theta_t = 1/T$;

S4-23

E step: for $n = 1$ to $N$
for $t = 1$ to $T$
for $s = 1$ to $l_t - L + 1$
Recalculation of $E_Z[Z_{nts}]$;

S4-24

M step: for $t = 1$ to $T$
$r_t = \sum_{n,t:t=t,s} E_Z[Z_{nts}]$
$\theta_t = \dfrac{r_t}{\sum_{t'} r_{t'}}$

S4-25

Convergence determination:
All $\theta_t$ are not changed

NO

YES

**End**

69

[Fig. 2-3]

EP 3 239 875 B1

**Start**

S4−31

HLA allele reference sequences are retrieved from the IMGT/HLA or other databases
Reads and HLA alleles to which reads are mapped is retrieved from a BAM file

S4−32

Initialization of E[$Z_{nts}$]:  for $n$ = 1 to $N$
for $t$ = 1 to $T$
for $s$ = 1 to $l_{th}$ - $L$ + 1
$E_Z[Z_{nts}]$ = 1 / $M_n$

Initialization of $E_\theta[\theta_t]$:  for $t$ = 1 to $T$
$\alpha_t^* = \alpha_0 + 1/T$
$E_\theta[\theta_t] = \dfrac{\alpha_t^*}{\sum_{t'} \alpha_{t'}^*}$

S4−33

VBE step:  for $n$ = 1 to $N$
for $t$ = 1 to $T$
for $s$ = 1 to $l_t$ - $L$ + 1
Recalculation of $E_Z[Z_{nts}]$;

S4−34

VBM step:  for $t$ = 1 to $T$
$\alpha_t^* = \alpha_0 + \sum_{n',t'=t,s'} E_Z[Z_{n't's'}]$
$E_\theta[\theta_t] = \dfrac{\alpha_t^*}{\sum_{t'} \alpha_{t'}^*}$

S4−35

Convergence determination:
All $E_\theta[\theta_t]$ are not changed

NO

YES

**End**

$N$:  Total number of reads

$T$:  Total number of HLA alleles

$Z_{nts}$ :  An indicator variable that equals to one if read n is generated from HLA allele t at start position s, and zero otherwise

$E[Z_{nts}]$ :  The expected number of mappings of read n to HLA allele t at position s

$l_t$:  The length of the reference sequence of HLA allele t

$L$:  The length of reads

$M_n$:  The total number of locations to which read n mapped

$\theta_t$:  The fraction of the total expected number of reads allocated to HLA allele t relative to the total read amount

$E_\theta[\theta_t]$ :  The expected value of the fraction of the total expected number of reads allocated to HLA allele t relative to the total read amount

$\alpha_0$:  A hyperparameter

[Fig. 31]

EP 3 239 875 B1

**Start**

The individual depth of coverage $d_t$
for each HLA allele is calculated :
for $t$ = 1 to $T$
    for $n$ = 1 to $N$

$$d_t = \left( \sum_{n=1}^{N} E[Z_{nt}] c_n \right) / l_t$$

— S5−1

For each HLA locus, HLA alleles with the two largest individual depth
of coverage are selected. The allele with the largest individual depth
of coverage $t$ = *first*, and the allele with the second largest individual
depth of coverage $t$ = *second*.

— S5−2

$N$ :  Total number of reads

$c_n$ :  The number of nucleotide bases in read n

$T$ :  Total number of HLA alleles

$Z_{nt}$ :  An indicator variable that equals to one if read n is
generated from HLA allele t, and zero otherwise

$E[Z_{nt}]$ : The expected number of mappings of read n to HLA allele t

$d_t$ :  The individual depth of coverage of HLA allele t

$l_t$ :  The length of the reference sequence of HLA allele t
(the number of nucleotide base)

$D$ :  Rejection depth

$d_{first} < D$ ?    YES → HLA type is not determined — D5−1

S5−3

↓ NO

$d_{second} < D$ ?    YES → $d_{first} > 2 * D$ ?    YES → HLA type is determined as homozygous of $t$ = *first* — D5−2

S5−4

S5−5

↓ NO                                    NO → HLA type is determined as heterozygous of $t$ = *first*, and another allele is not determined — D5−3

$d_{first} > 2 * d_{second}$ ?    YES → HLA type is determined as homozygous of $t$ = *first* — D5−4

S5−6

NO → HLA type is determined as heterozygous of $t$ = *first*, and $t$ = *second* — D5−5

[Fig. 4]

[Fig. 5-1]

[Fig. 5-2]

[Fig. 5-3]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014065410 A1
- JP 2014265704 A

**Non-patent literature cited in the description**

- **ITOH Y et al.** *Immunogenetics,* 2005
- **NARIAI et al.** *Bioinformatics,* 2013, vol. 15; 29 (18
- **NARIAI et al.** *Bioinformatics,* vol. 15; 29 (18
- **BISHOP CM.** Pattern Recognition and Machine Learning. Springer Science:Business Media, LLC, 2006
- **BAI et al.** *BMC genomics,* 2014, vol. 15 (325
- **WARREN et al.** *Genome medicine,* 2013, vol. 4 (12), 102
- **MAJOR et al.** *PloS one,* 2013, vol. 8 (11), e78410
- **ITOH, Y. et al.** *Immunogenetics,* 2005, vol. 57, 717-729